(19) Europäisches Patentamt
European Patent Office
Office européen des brevets

(11) EP 3 049 082 B1

(12) **EUROPEAN PATENT SPECIFICATION**

(45) Date of publication and mention of the grant of the patent:
**22.05.2019 Bulletin 2019/21**

(21) Application number: **14799198.8**

(22) Date of filing: **23.09.2014**

(51) Int Cl.:
***A61K 31/444*** *(2006.01)* ***A61K 31/4545*** *(2006.01)*
***A61K 31/497*** *(2006.01)* ***A61K 31/501*** *(2006.01)*
***A61K 31/506*** *(2006.01)* ***A61P 17/02*** *(2006.01)*

(86) International application number:
**PCT/IB2014/001932**

(87) International publication number:
**WO 2015/044759 (02.04.2015 Gazette 2015/13)**

(54) **TREATMENT OF BURN PAIN BY TRPV1 MODULATORS**

BEHANDLUNG VON VERBRENNUNGSSCHMERZEN MIT TRPV1-MODULATOREN

TRAITEMENT DE LA DOULEUR OCCASIONNÉE PAR DES BRÛLURES PAR DES MODULATEURS DE TRPV1

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.09.2013 US 201361881723 P**

(43) Date of publication of application:
**03.08.2016 Bulletin 2016/31**

(73) Proprietor: **Purdue Pharma L.P.**
**Stamford, CT 06901-3431 (US)**

(72) Inventor: **SYLVESTRE, George**
**Moosup, CT 06354 (US)**

(74) Representative: **Maiwald Patent- und Rechtsanwaltsgesellschaft mbH**
**Elisenhof**
**Elisenstraße 3**
**80335 München (DE)**

(56) References cited:
**EP-A1- 2 210 878** **WO-A1-2008/133973**
**WO-A2-2005/030753** **US-A1- 2009 170 868**

- **LAYCOCK HELEN ET AL: "Peripheral mechanisms of burn injury-associated pain", EUROPEAN JOURNAL OF PHARMACOLOGY, vol. 716, no. 1, 13 March 2013 (2013-03-13), pages 169-178, XP028739252, ISSN: 0014-2999, DOI: 10.1016/J.EJPHAR.2013.01.071**
- **KYLE DONALD J ET AL: "TRPV1 antagonists: a survey of the patent literature", EXPERT OPINION ON THERAPEUTIC PATENTS, INFORMA HEALTHCARE, GB, vol. 16, no. 7, 1 July 2006 (2006-07-01), pages 977-996, XP002464449, ISSN: 1354-3776, DOI: 10.1517/13543776.16.7.977**
- **TAFESSE L ET AL: "Structure-activity relationship studies and discovery of a potent transient receptor potential vanilloid (TRPV1) antagonist 4-[3-chloro-5-[(1 S)-1,2-dihydroxyethyl]-2-pyridyl]-N-[5-(tr ifluoromethyl)-2-pyridyl]-3, 6-dihydro-2 H-pyridine-1-carboxamide (V116517) as a clinical candidate for pain managem", JOURNAL OF MEDICINAL CHEMISTRY 20140814 AMERICAN CHEMICAL SOCIETY USA, vol. 57, no. 15, 14 August 2014 (2014-08-14), pages 6781-6794, XP002734328, ISSN: 0022-2623**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 049 082 B1

Printed by Jouve, 75001 PARIS (FR)

## Description

### 1. FIELD

The disclosure relates to TRPV1 modulators and a method to treat burn pain comprising administering to an animal in need thereof an effective amount of a TRPV1 modulator or a composition comprising an effective amount of a TRPV1 modulator.

### 2. BACKGROUND

Burn wounds are a widespread clinical problem. The American Burn Association estimates that almost a half a million individuals receive medical treatment each year in the United States alone for burn-related injuries (American Burn Association: Burn Incidence and Treatment in the United States: 2012 Fact Sheet @ www.ameriburn.org/resources_factsheet.php). Although burn care has improved considerably over the past several decades, there is still extensive morbidity and mortality associated with burn-related injuries, particularly in children (Meena et al., "Effect of topical phenytoin on burn wound healing in rats," Indian J. Exper. Biology 49:56-59 (2011); Stoddard et al., "Acute stress symptoms in young children with burns," J. Amer. Acad. Child Adolesc. Psychiatry 45:87-93 (2006); Wang et al., "Nociceptive behavior following hindpaw burn injury in young rats: response to systemic morphine," Pain Med. 12:87-98 (2011)).

Severe burn injury causes major tissue damage and the resulting attempts of the body to defend against infection and to effect repair of the injured tissue generates a massive inflammatory response, thereby initiating and maintaining the sensation of pain associated with the burn injury (White et al., "Severe burn injury induces a characteristic activation of extracellular signal-regulated kinase 1/2 in spinal dorsal horn neurons," Eur. J. Pain 15:683-690 (2011)). Overall, wound repair and burn pain processing are extremely complex and multifaceted. The clinical management of burn injury has been challenging due to the limited knowledge of the basic mechanisms causing altered pain sensitivity, the molecular, cellular and/or physiological mechanisms governing burn wound progression and healing, and the factors that regulate skin re-epithelialization and long-term sequelae, such as scarring. Thus, impairments in wound healing constitute a great medical burden. Aberrations in the normal biological response to cutaneous injury following a burn wound can often lead to significant complications, such as fibroproliferatine diseases, skin fibrosis, functional impairment and/or aesthetic issues (Wong et al., "Surgical approaches to create murine models of human wound healing," J Biomedicine Biotech. 2011:1-8 (2011)).

A burn injury can originate from a thermal exposure, such as a burn caused by exposure to a flame, a hot surface, a hot liquid, such as scalding-type burn injury, or exposure to extreme cold. A painful burn injury can also result from radiation exposure, for example, UVB exposure causing sunburn (*see*, *e.g.*, U.S. Pat. Nos. 6,984,647 and 7,678,812), from contact with chemical agents, for example, exposure to alkaline material (*see*, *e.g.*, Okada et al., "TRPV1 involvement in inflammatory tissue fibrosis in mice," Amer. J. Pathology 178:2654-2664 (2011)), or from friction (*see*, *e.g.*, Pereira et al., "Development of animal model for studying deep second-degree thermal burns," J. Biomedicine Biotech. 2012:1-7 (2012)).

Clinically, opioids are first line drugs used to treat severe pain associated with burn injury. Three major classes of opioid receptors in the central nervous system (CNS) have long been known, with each class having subtype receptors. These receptor classes are known as $\mu$, $\kappa$ and $\delta$. However, the analgesic effects of opioid compounds are often unpredictable in burn injuries. Furthermore, repeated opioid exposure alters the immune response compounding the issues of infection and tolerance. Given these shortcomings, alternate forms of therapy for burn-induced pain are both needed and desirable.

There is a body of evidence relating activity at vanilloid receptors (TRPVR1) to pain processing (Di Marzo et al., "Endovanilloid signaling in pain," Current Opinion in Neurobiology 12:372-379 (2002)). The discovery of the Transient Receptor Potential Vanilloid 1 ("TRPV1," formerly known as Vanilloid Receptor 1 or VR1) receptor presents an opportunity in drug discovery for compounds that can be administered for burn pain management or other syndromes modulated by this receptor.

U.S. Pat. App. No. 2010/0261911 discloses TRPV1 mediation, by a particular genus of agonist compounds, of thermal burn pain caused by exposure to heat, chemical burn pain caused by exposure to an acid, and sunburn. U.S. Pat. App. No. 2009/0209633 discloses TRPV1-modulated mediation of burn pain by TRPV1 agonists, such as resiniferatoxin, tinyatoxin and capsaicin.

U.S. Pat. No. 8,394,828 discloses TRPV1-modulated treatment of burn pain by a particular genus of compounds, where the burn pain is induced thermally or by the sun.

U.S. Pat. No. 7,767,705 and U.S. Pat. App. Pub. No. 2010/0249203 disclose TRPV1-modulated treatment of burn pain by a particular genus of TRPV1 antagonist compounds.

Laycock et al. disclose in Eur. J. Pharmacol., vol. 716 (2013), no. 1, pp 169 to 178 a review on the "Peripheral

mechanisms of burn injury-associated pain. It was previously assumed that TRPV1 and TRPV2 were implicated in the detection of heat. It is also stated that the most common analgesia used in burn injury pain in burn units are opioids, which , however, in the majority of cases do not provide sufficient pain controly, and may even induce severe side effects.

Citation of any reference in Section 2 of this application is not to be construed as an admission that such reference is prior art to the present application.

## 3. SUMMARY

The present invention and the scope thereof is defined by the appended claims. The more generic description of the invention is provided for illustrative purposes only. Embodiments not falling under these claims are for reference purposes only.

In one aspect of the disclosure, compounds of the disclosure can be used to treat an animal suffering from burn pain.

In another aspect of the disclosure, compounds for use in treating burn pain in an animal by administering one or more compounds of formula (I) to an animal in need of such treatment are described. In certain embodiments, such compounds of formula (I) effectively treat burn pain in the animal, while producing fewer or reduced side effects compared to previously available compounds.

In some embodiments, such compounds exhibit antagonist activity at the TRPV1 receptor.

Herein disclosed to treat an animal suffering from burn pain is a therapeutically effective amount of a compound of formula (I):

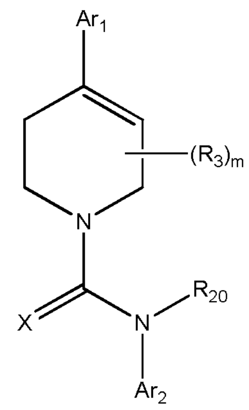

(I)

wherein

X is selected from the group consisting of O and S;
$Ar_1$ is selected from the group consisting of:

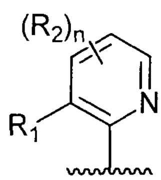 , 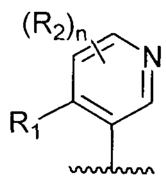 , 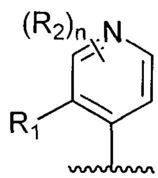 , 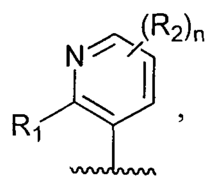 ,

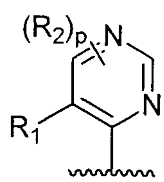 , 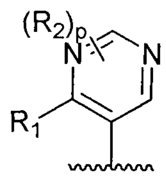 , 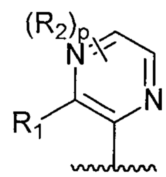 ,

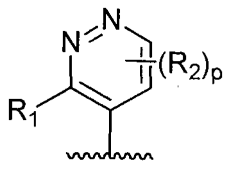 , 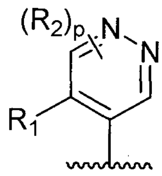 and 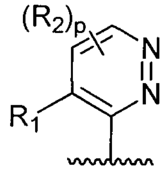 ;

$Ar_2$ is selected from the group consisting of:

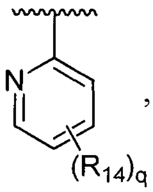 , 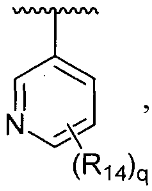 , 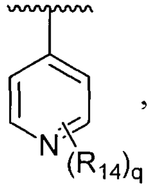 , 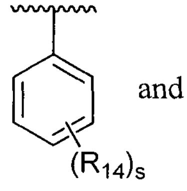 and 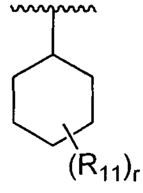 ;

$R_1$ is selected from the group consisting of -H, -halo, -($C_1$-$C_4$)alkyl, -OH, -$OCH_3$, -$NH_2$, -C(halo)$_3$, -CH(halo)$_2$, -$CH_2$(halo), -OC(halo)$_3$, -OCH(halo)$_2$, and -$OCH_2$(halo);
each $R_2$ is independently selected from the group consisting of:

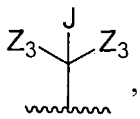 , 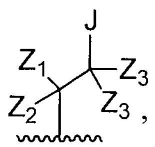 , 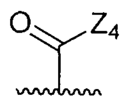 , and 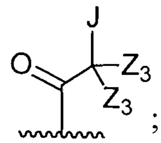 ;

$Z_1$ is -H, -$OR_7$, -$CH_2$-$OR_7$, or -halo;
$Z_2$ is -H, -($C_1$-$C_6$)alkyl, -($C_2$-$C_6$)alkenyl, -$CH_2$-$OR_7$, or -halo;
each $Z_3$ is independently -H, -($C_1$-$C_6$)alkyl, or -($C_2$-$C_6$)alkenyl,;
$Z_4$ is -H, -OH, -$OR_{20}$, or -($C_1$-$C_6$)alkyl;
J is -$OR_{20}$;
provided that when $Z_1$ is -$OR_7$, then $Z_2$ is not -halo;
each $R_3$ is independently selected from the group consisting of -H, ($C_1$-$C_6$)alkyl, and $CH_2OR_7$;
each $R_7$ is independently -H, -($C_1$-$C_6$)alkyl, -($C_2$-$C_6$)alkenyl, -($C_1$-$C_6$)haloalkyl, -($C_1$-$C_6$)hydroxyalkyl, or -($C_1$-$C_6$)alkoxy($C_1$-$C_6$)alkyl;
each $R_{11}$ is independently -CN, -OH, -($C_1$-$C_6$)alkyl, -($C_2$-$C_6$)alkenyl, -halo, -N($R_7$)$_2$, -$NR_7$OH, -$OR_7$, -C(O)$R_7$, or -C(O)O$R_7$;
each $R_{14}$ is independently -H, -($C_1$-$C_6$)alkyl, -($C_2$-$C_6$)alkenyl, -($C_1$-$C_6$)alkoxy-($C_1$-$C_6$)alkyl, -C(halo)$_3$, -CH(halo)$_2$, -$CH_2$(halo), -($C_1$-$C_6$)haloalkyl, -($C_2$-$C_6$)haloalkenyl, -($C_2$-$C_6$)hydroxyalkenyl, -($C_1$-$C_6$)alkoxy($C_2$-$C_6$)alkyl, -($C_1$-$C_6$)alkoxy($C_2$-$C_6$)alkenyl, -CN, -OH, -halo, -OC(halo)$_3$, -N($R_7$)$_2$, -$OR_7$, -C(O)$R_7$, -C(O)O$R_7$, -S(O)$R_7$, -S(O)$_2R_7$, -S(O)$_2$N($R_7$)$_2$, or -$SO_2$C(halo)$_3$;
each $R_{20}$ is independently -H, or -($C_1$-$C_6$)alkyl;
each halo is independently -F, -Cl, -Br, or -I;
n is the integer 1, 2, or 3;
p is the integer 1 or 2;
q is the integer 0, 1, 2, 3 or 4;
r is the integer 0, 1, 2, 3, 4, 5, or 6;
s is the integer 0, 1, 2, 3, 4, or 5;
m is the integer 0, 1, or 2;

or a pharmaceutically acceptable salt or solvate thereof.

In certain embodiments, an animal suffering from burn pain is administered a therapeutically effective amount of a compound of formula (I), or a pharmaceutically salt or solvate thereof, wherein:

X = O;
$R_1$ is halo;
$Ar_1$ is:

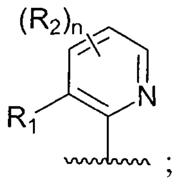
;

each $Z_3$ is independently selected from H or $(C_1\text{-}C_6)$alkyl;
$Z_1$ is H or $-CH_2OR_7$;
$Z_2$ is selected from H, $-(C_1\text{-}C_6)$alkyl, or $-CH_2OR_7$;
$Ar_2$ is:

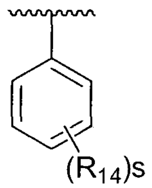
or
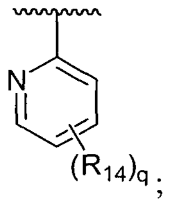
;

each $R_{14}$ is independently selected from halo, $C(halo)_3$, $-(C_1\text{-}C_6)$alkyl, $OR_7$, $OC(halo)_3$, or $SO_2C(halo)_3$, and preferably is halo, $C(halo)_3$, or $OC(halo)_3$; and
s and q are each 1 or 2; and
$R_2$, $R_7$, $R_{20}$, $R_3$, m, and n are defined as above.

In other embodiments, an animal suffering from burn pain is administered a therapeutically effective amount of a compound of formula (I), or a pharmaceutically acceptable salt or solvate thereof, wherein $Ar_1$ is:

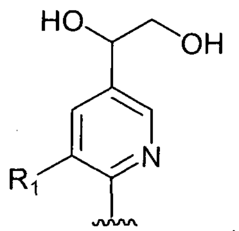
.

In other embodiments, an animal suffering from burn pain is administered a therapeutically effective amount of a compound of formula (I), or a pharmaceutically acceptable salt or solvate thereof, wherein $Ar_2$ is selected from:

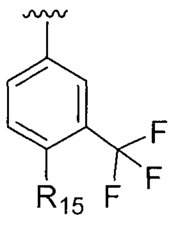
,
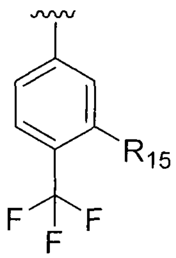
,
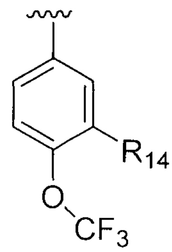
,
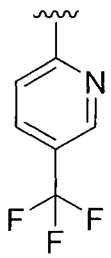
,
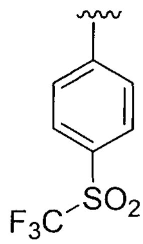
or
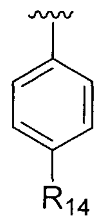
;

wherein $R_{14}$ is selected from H, halo, $C(halo)_3$, $-(C_1\text{-}C_6)$alkyl, $OR_7$, $OC(halo)_3$, or $SO_2C(halo)_3$, and preferably is halo, $C(halo)_3$, or $OC(halo)_3$; and
$R_{15}$ is -H, -Cl, -F, -Br, $-CH_3$, $-CH_2CH_3$, $-OCH_3$, $-OCH(CH_3)_2$ or $-OCH_2CH_3$.

In certain embodiments, an animal suffering from burn pain is administered a therapeutically effective amount of a compound of formula (I),selected from the group consisting of:

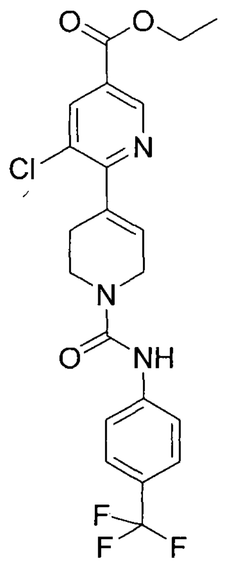,

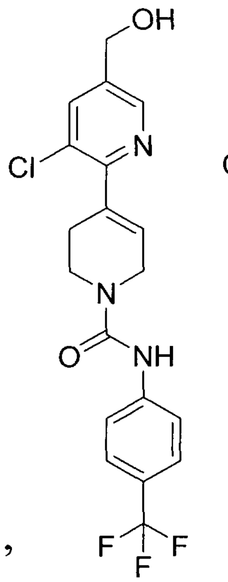,

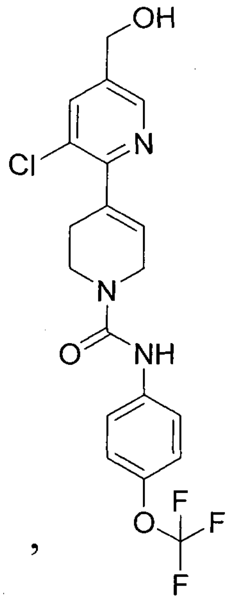,

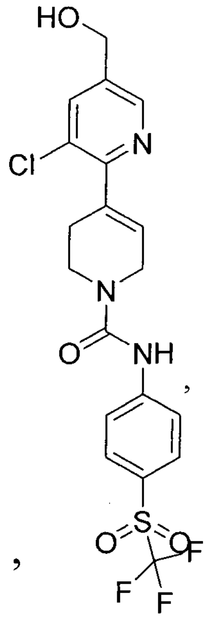,

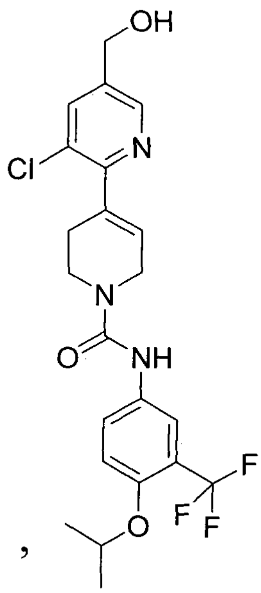,

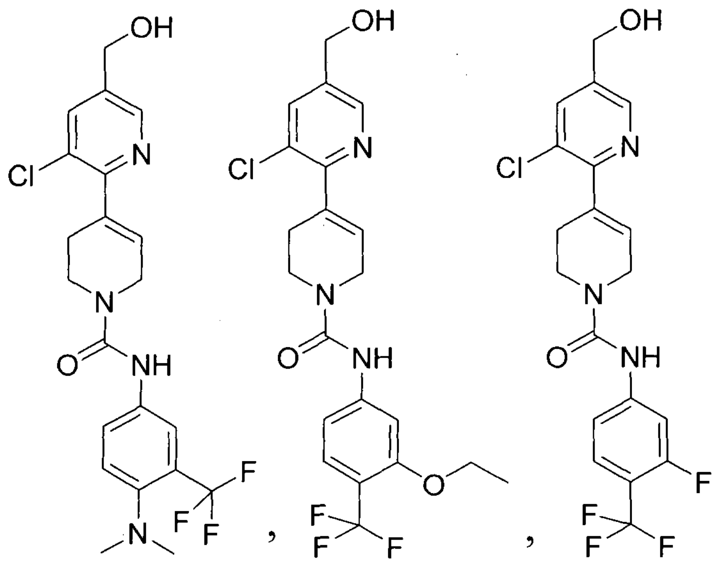,

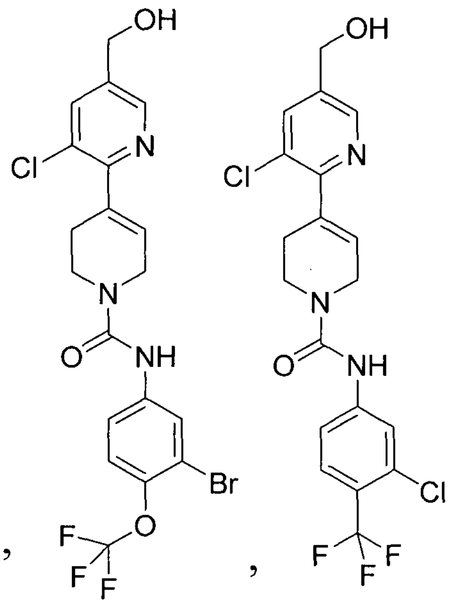,

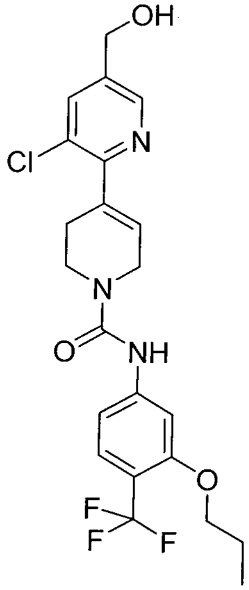 , 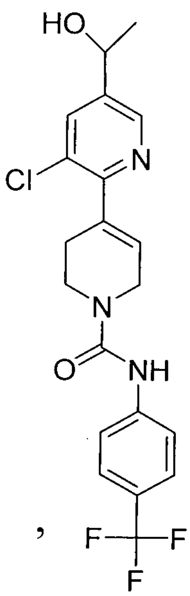 , 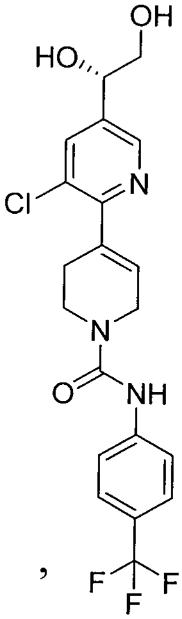 , 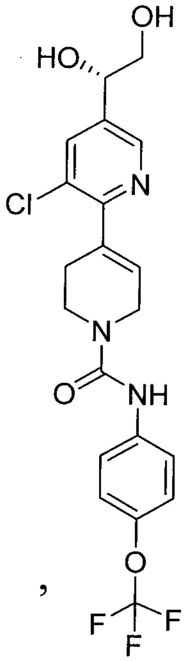 , 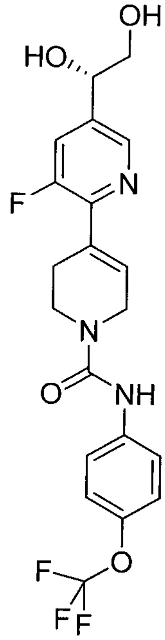 ,

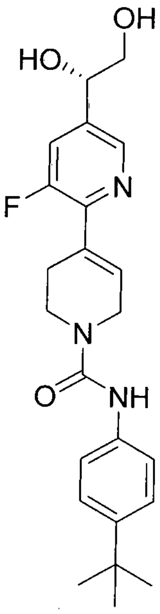 , 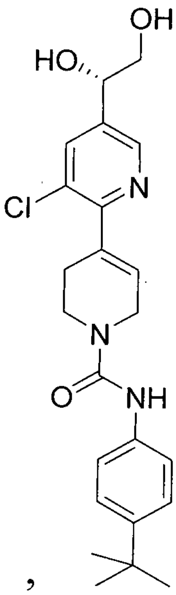 , 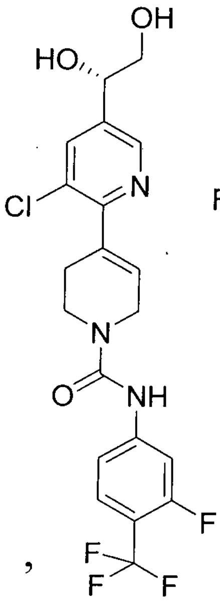 , 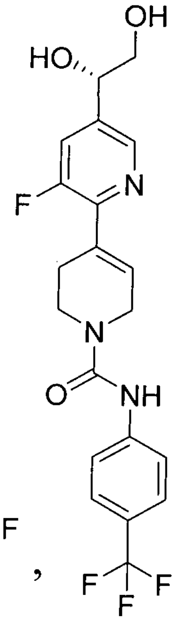 , 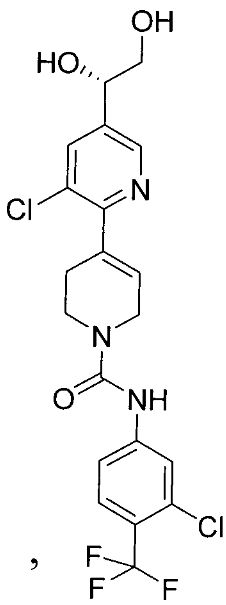 ,

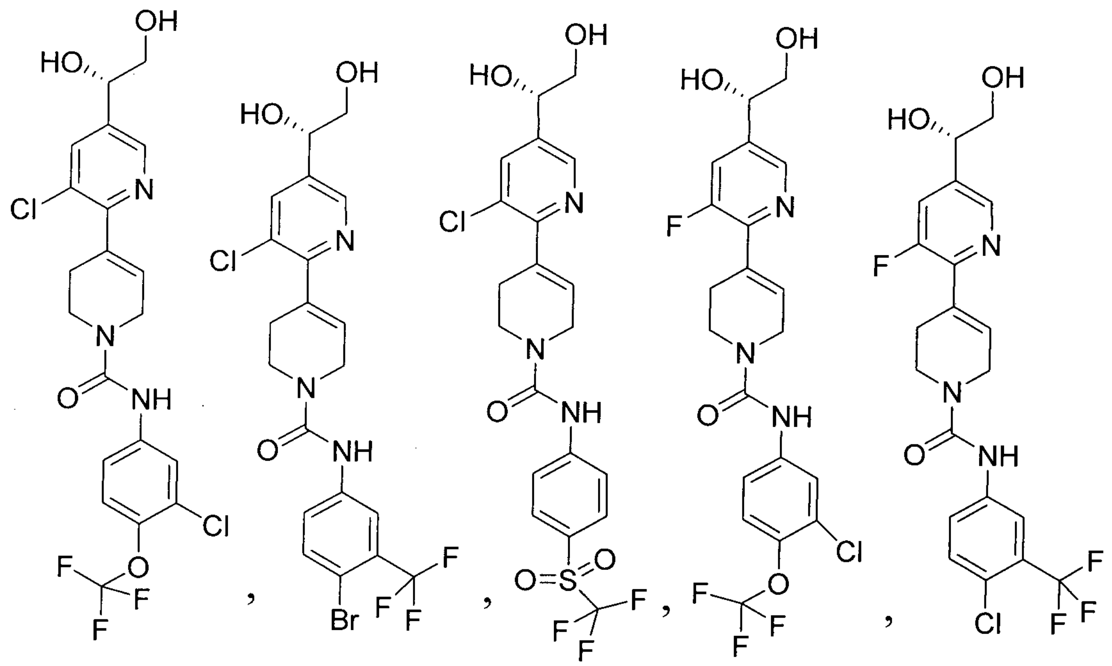

, , , , ,

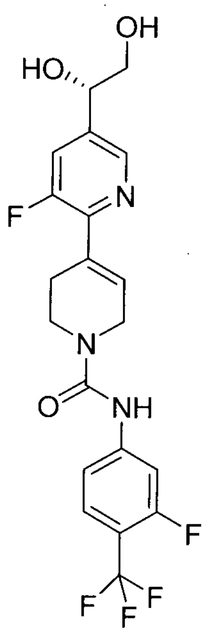

,

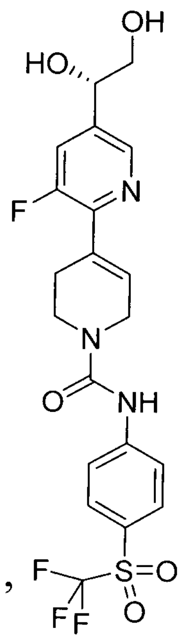

,

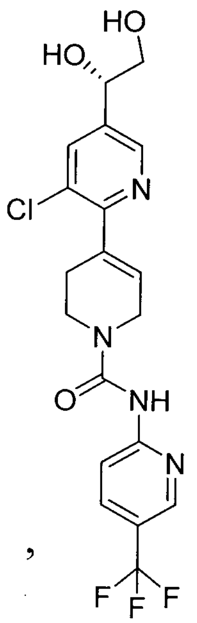

,

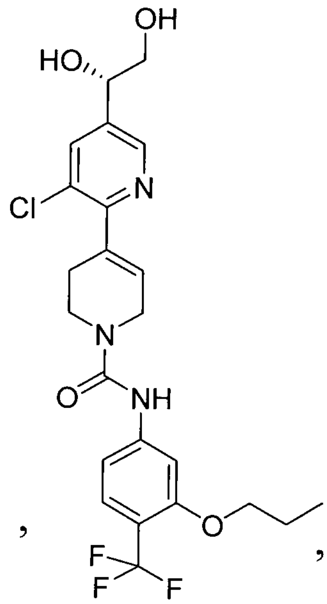

,

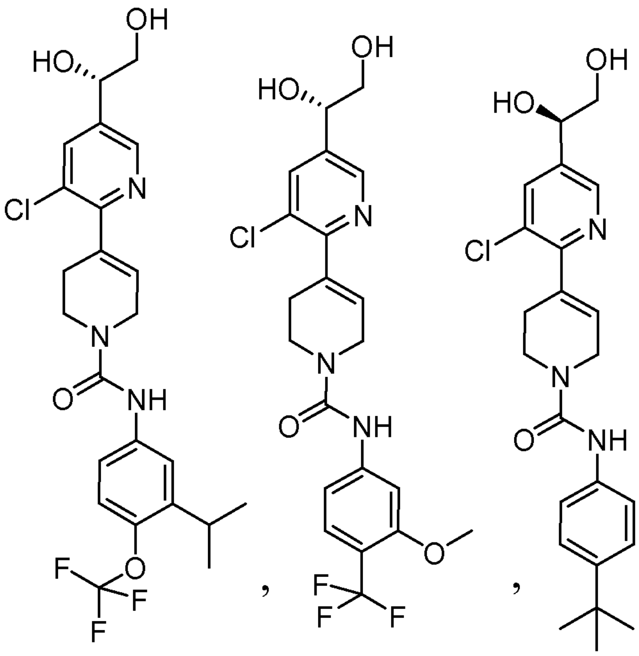

,

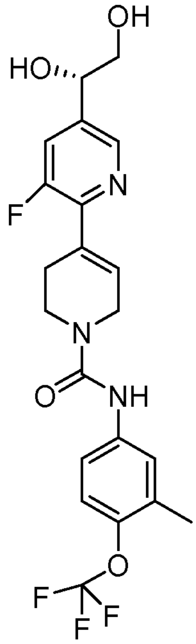

,

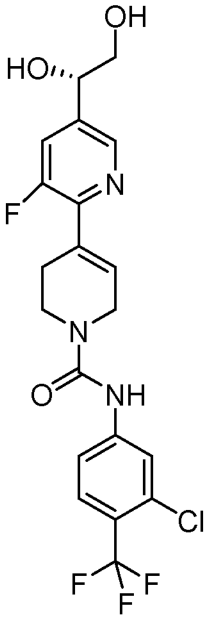

,

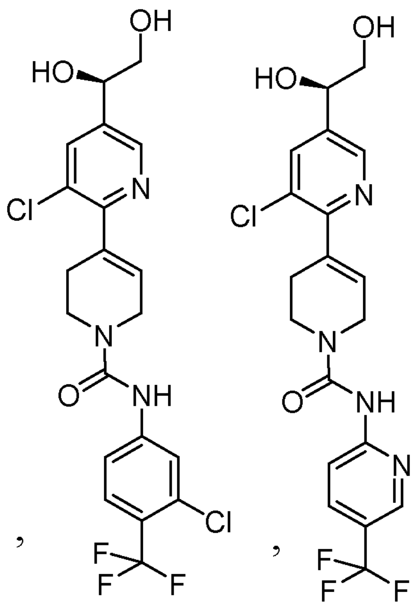

,

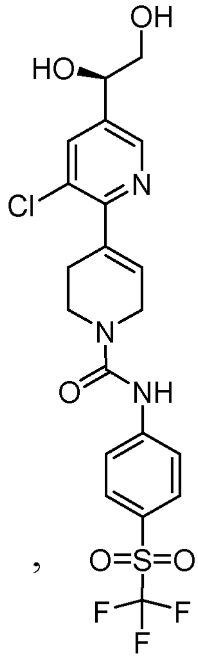

,

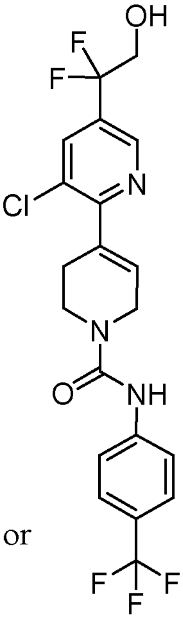

, or

and the pharmaceutically acceptable salts and solvates thereof.

A compound of formula (I), or a pharmaceutically acceptable salt or solvate thereof, is useful for treating pain associated with a burn injury originating from a thermal exposure, from a radiation exposure, from contact with a chemical agent, and/or from friction (each being a "Condition") in an animal.

The disclosure further relates to a pharmaceutical composition for use in treating a Condition in an animal in need of said treatment, the composition comprising a therapeutically effective amount of a compound of formula (I), or a pharmaceutically acceptable salt or solvate thereof, and a pharmaceutically acceptable carrier or excipient.

The disclosure further relates to use of a compound of formula (I), or a pharmaceutically acceptable salt or solvate thereof, in the manufacture of a medicament for treating a Condition.

The disclosure further relates to a compound of formula (I), or a pharmaceutically acceptable salt or solvate thereof, for use in the treatment of a Condition.

The disclosure still further relates to a method for preparing a pharmaceutical composition according to the

invention, comprising the step of admixing a compound of formula (I), or a pharmaceutically acceptable salt or solvate thereof, and a pharmaceutically acceptable carrier or excipient.

The disclosure still further relates to a kit for treating a Condition, comprising a sterile container comprising a therapeutically effective amount of a compound of formula (I), or a pharmaceutically acceptable salt or solvate thereof.

In one embodiment, preferred compounds of formula (I) are compounds of formula (II):

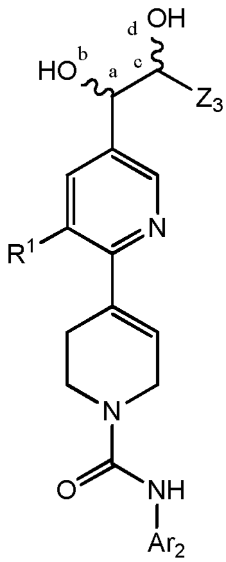

(II)

or a pharmaceutically acceptable salt or solvate thereof, where:

$R_1$ is -H, -halo, -($C_1$-$C_4$)alkyl, -C(halo)$_3$, -CH(halo)$_2$, or -$CH_2$(halo);

$Z_3$ is -H or -$CH_3$;

$Ar_2$ is as defined for compounds of formula (I); and

each halo is independently -F, -Cl, -Br, or -I.

The disclosure can be understood more fully by reference to the following detailed description and illustrative examples, which are intended to exemplify non-limiting embodiments of the disclosure.

4. DETAILED DESCRIPTION

4.1 Compounds of Formula (I)

The disclosure encompasses compounds of formula (I):

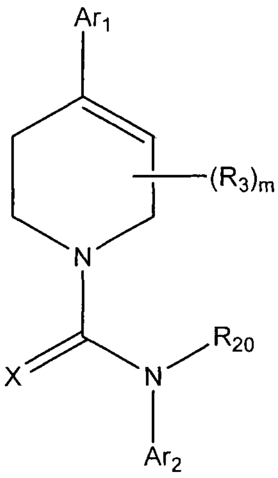

(I)

wherein

X is selected from the group consisting of O and S;
$Ar_1$ is selected from the group consisting of:

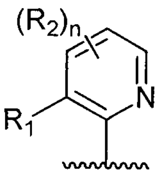 , 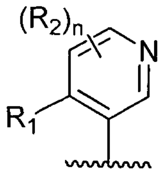 , 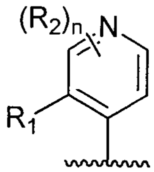 , 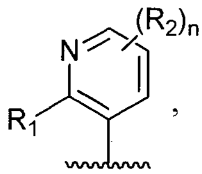 ,

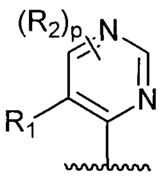 , 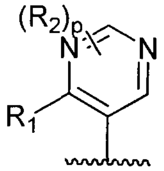 , 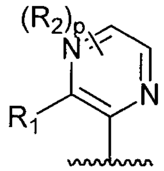 ,

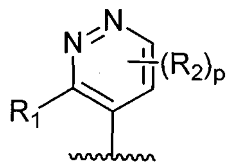 , 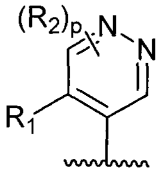 and 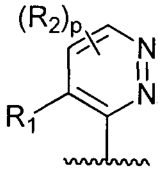 ;

$Ar_2$ is selected from the group consisting of:

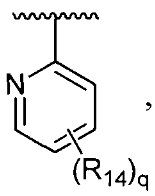 , 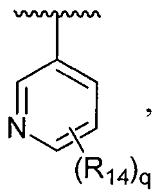 , 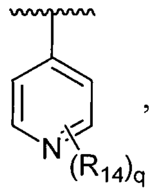 , 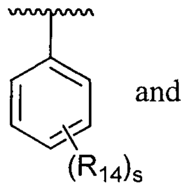 and 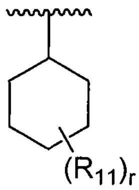 ;

$R_1$ is selected from the group consisting of -H, -halo, -($C_1$-$C_4$)alkyl, -OH, $-OCH_3$, $-NH_2$, $-C(halo)_3$, $-CH(halo)_2$, $-CH_2(halo)$, $-OC(halo)_3$, $-OCH(halo)_2$, and $-OCH_2(halo)$;
each $R_2$ is independently selected from the group consisting of:

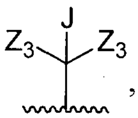 , 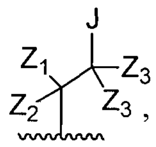 , 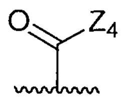 , and 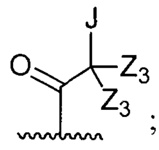 ;

$Z_1$ is -H, $-OR_7$, $-CH_2-OR_7$, or -halo;
$Z_2$ is -H, $-(C_1-C_6)$alkyl, $-(C_2-C_6)$alkenyl, $-CH_2-OR_7$, or -halo;
each $Z_3$ is independently -H, $-(C_1-C_6)$alkyl, or $-(C_2-C_6)$alkenyl,;
$Z_4$ is -H, -OH, $-OR_{20}$, or $-(C_1-C_6)$alkyl;
J is $-OR_{20}$;
provided that when $Z_1$ is $-OR_7$, then $Z_2$ is not -halo;
each $R_3$ is independently selected from the group consisting of-H, $(C_1-C_6)$alkyl, and $CH_2OR_7$;
each $R_7$ is independently -H, $-(C_1-C_6)$alkyl, $-(C_2-C_6)$alkenyl, $-(C_1-C_6)$haloalkyl,$-(C_1-C_6)$hydroxyalkyl, or $-(C_1-C_6)$alkoxy$(C_1-C_6)$alkyl;
each $R_{11}$ is independently -CN, -OH, $-(C_1-C_6)$alkyl, $-(C_2-C_6)$alkenyl, -halo, $-N(R_7)_2$, $-NR_7OH$, $-OR_7$, $-C(O)R_7$, or $-C(O)OR_7$;
each $R_{14}$ is independently -H, $-(C_1-C_6)$alkyl, $-(C_2-C_6)$alkenyl, $-(C_1-C_6)$alkoxy-$(C_1-C_6)$alkyl, $-C(halo)_3$, $-CH(halo)_2$, $-CH_2(halo)$, $-(C_1-C_6)$haloalkyl, $-(C_2-C_6)$haloalkenyl, $-(C_2-C_6)$hydroxyalkenyl, $-(C_1-C_6)$alkoxy$(C_2-C_6)$alkyl, $-(C_1-C_6)$alkoxy$(C_2-C_6)$alkenyl, -CN, -OH, -halo, $-OC(halo)_3$, $-N(R_7)_2$, $-OR_7$, $-C(O)R_7$, $-C(O)OR_7$, $-S(O)R_7$, $-S(O)_2R_7$, $-S(O)_2N(R_7)_2$, or $-SO_2C(halo)_3$;
each $R_{20}$ is independently -H, or $-(C_1-C_6)$alkyl;
each halo is independently -F, -Cl, -Br, or -I;
n is the integer 1, 2, or 3;
p is the integer 1 or 2;
q is the integer 0, 1, 2, 3 or 4;
r is the integer 0, 1, 2, 3, 4, 5, or 6;
s is the integer 0, 1, 2, 3, 4, or 5;
m is the integer 0, 1, or 2;
and the pharmaceutically acceptable salts and solvates as defined herein.

Certain embodiments of formula (I) are presented below.
In one embodiment, a compound of formula (I) is a free base.
In another embodiment, a compound of formula (I) is a pharmaceutically acceptable salt or solvate of a compound of formula (I).
In another embodiment, a compound of formula (I) is a pharmaceutically acceptable salt.
In another embodiment, $R_1$ is -F, -Cl, -Br, or $-CF_3$.
In another embodiment, $R_1$ is -F, -Cl, or $-CF_3$.
In another embodiment, $R_1$ is -F or $-CF_3$.
In another embodiment, $R_1$ is -Cl or $-CF_3$.
In another embodiment, $R_1$ is -F or -Cl.
In another embodiment, $R_1$ is -F.
In another embodiment, $R_1$ is -Cl.
In another embodiment, $R_1$ is $-CF_3$.
In another embodiment, $R_1$ is -Br.
In certain embodiments, an animal suffering from a Condition is administered a therapeutically effective amount of a compound of formula (I), or a pharmaceutically salt or solvate thereof, wherein:

X=O;
$R_1$ is halo;
$Ar_1$ is:

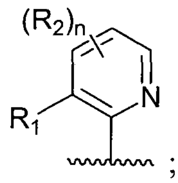 ;

each $Z_3$ is independently selected from H or ($C_1$-$C_6$)alkyl;
$Z_1$ is H or -$CH_2OR_7$;
$Z_2$ is selected from H, -($C_1$-$C_6$)alkyl, or -$CH_2OR_7$;
$Ar_2$ is:

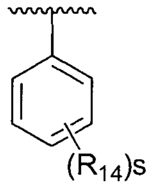

or

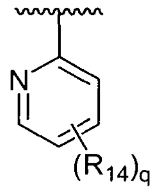

;

each $R_{14}$ is independently selected from halo, C(halo)$_3$, -($C_1$-$C_6$)alkyl, $OR_7$, OC(halo)$_3$, or $SO_2$C(halo)$_3$, and preferably is halo, C(halo)$_3$, or OC(halo)$_3$; and
s and q are each 1 or 2.

In other embodiments, an animal suffering from a Condition is administered a therapeutically effective amount of a compound of formula (I), or a pharmaceutically acceptable salt or solvate thereof, wherein $Ar_1$ is:

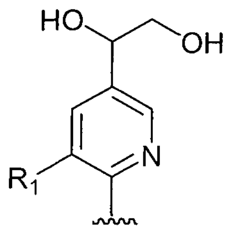

.

In other embodiments, an animal suffering from a Condition is administered a therapeutically effective amount of a compound of formula (I), or a pharmaceutically acceptable salt or solvate thereof, wherein $Ar_2$ is selected from:

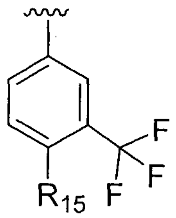

,

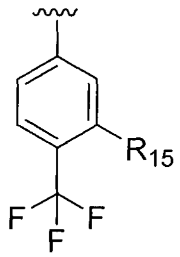

,

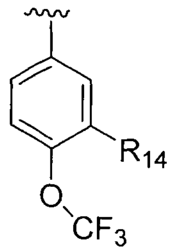

,

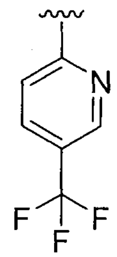

,

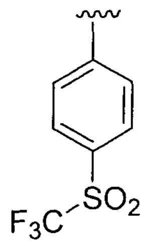

or

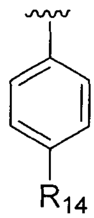

;

wherein $R_{14}$ is selected from H, halo, C(halo)$_3$, -($C_1$-$C_6$)alkyl, $OR_7$, OC(halo)$_3$, or $SO_2$C(halo)$_3$, and preferably is halo, C(halo)$_3$, or OC(halo)$_3$; and
$R_{15}$ is -H, -Cl, -F, -Br, -$CH_3$, -$CH_2CH_3$, -$OCH_3$, -$OCH(CH_3)_2$ or -$OCH_2CH_3$.

In certain embodiments, an animal suffering from a Condition is administered a therapeutically effective amount of a compound of formula (I) selected from the group consisting of:

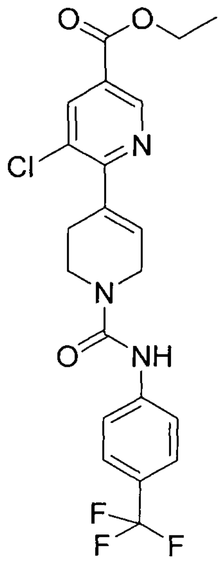 , 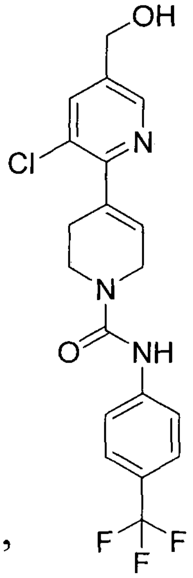 , 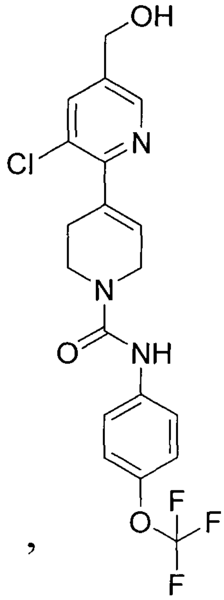 , 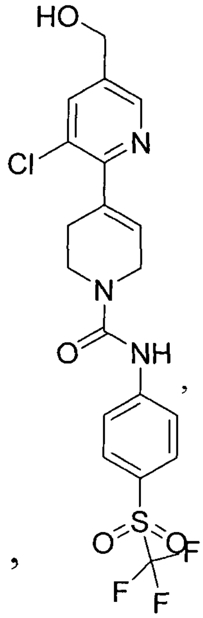 , 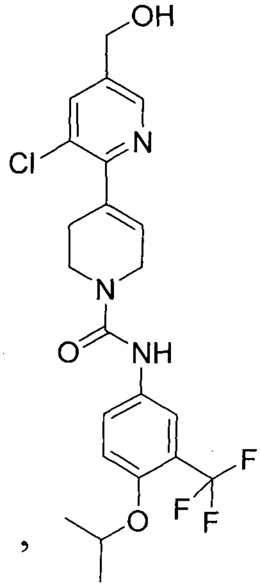 ,

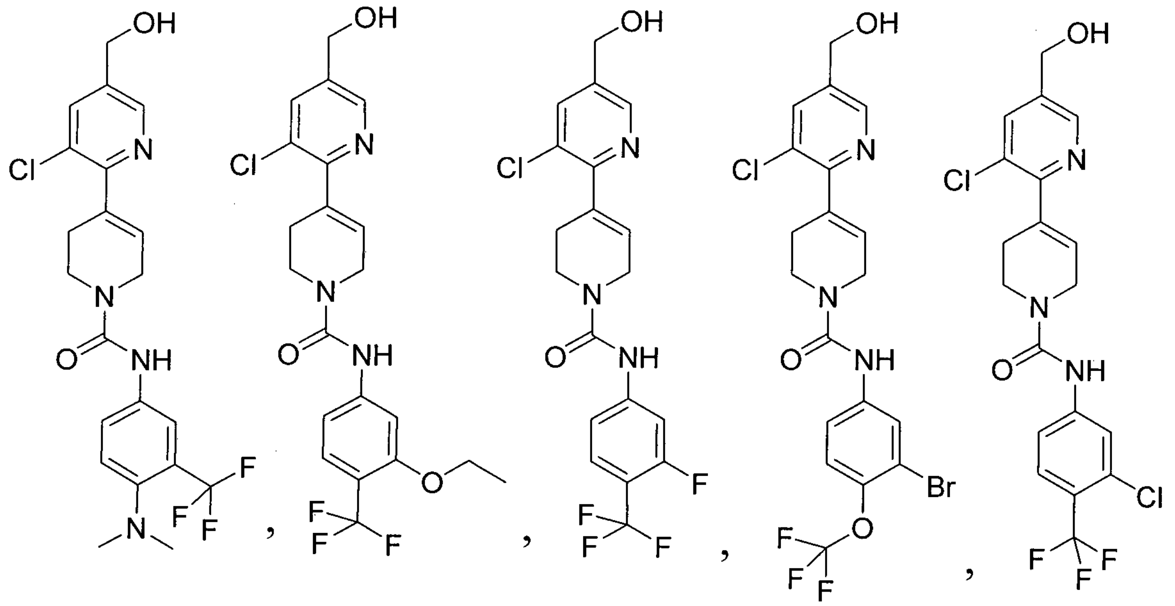 ,

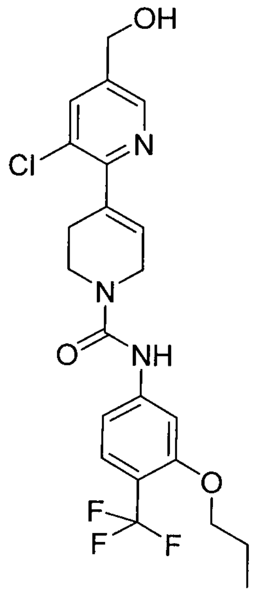,
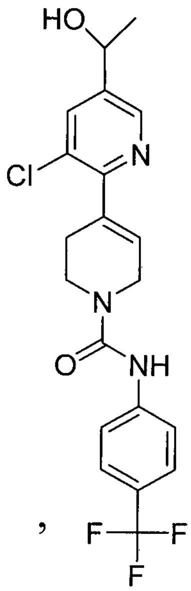,
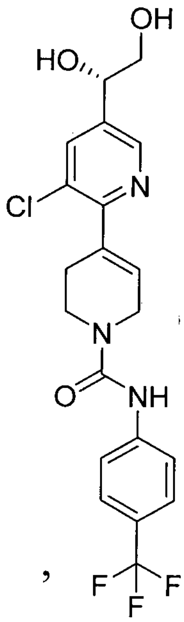,
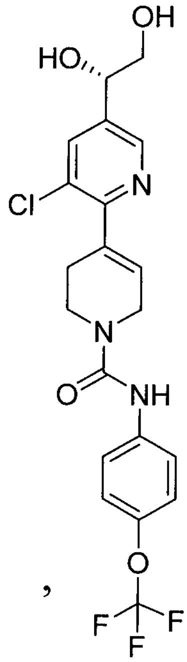,
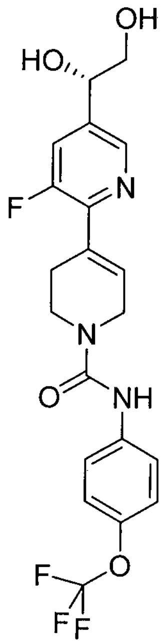,

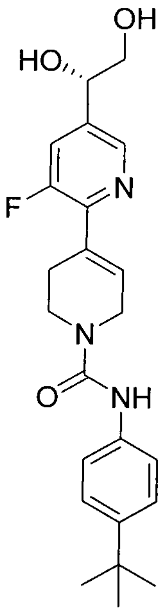,
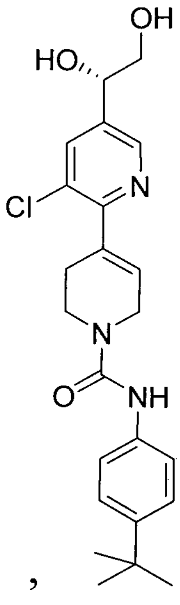,
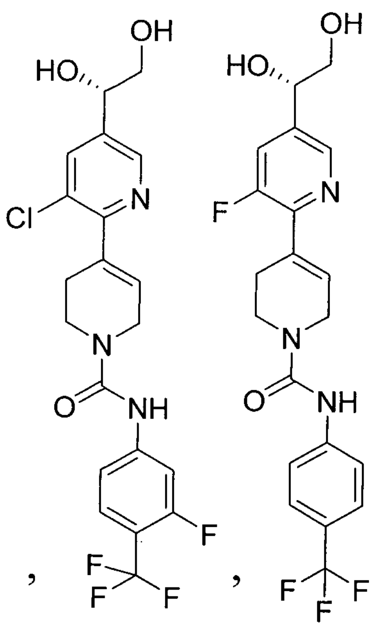,
,
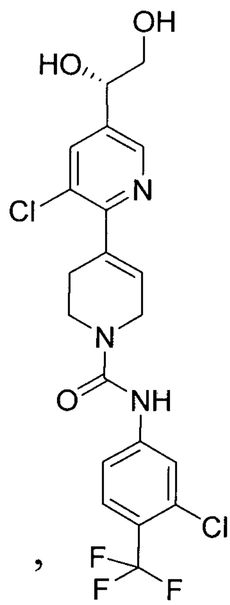,

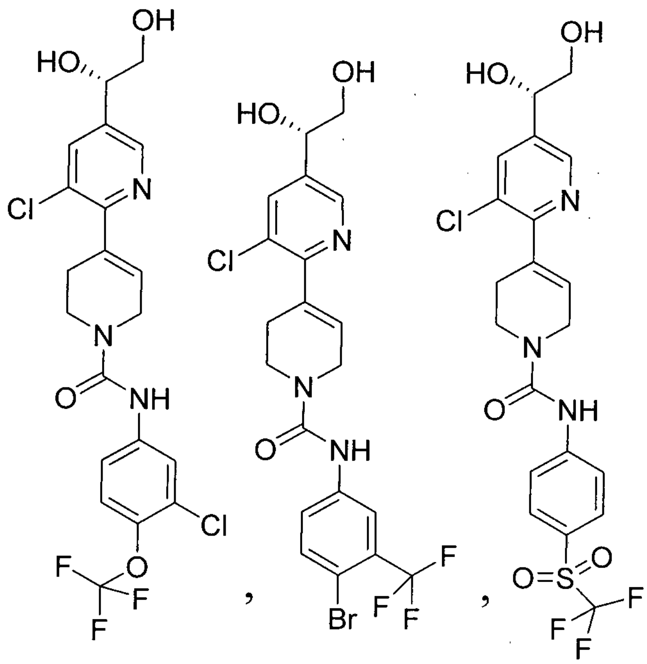

, , ,

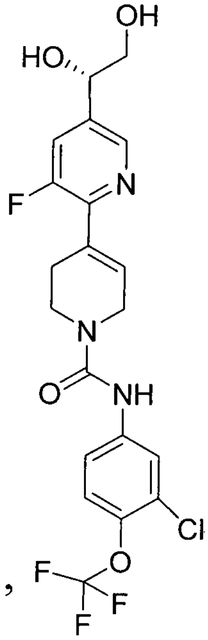

,

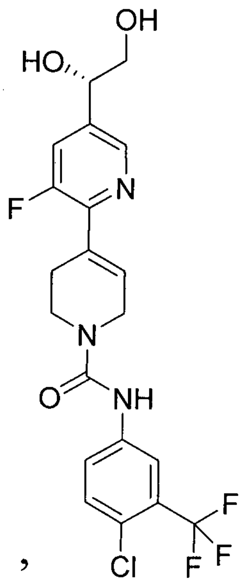

,

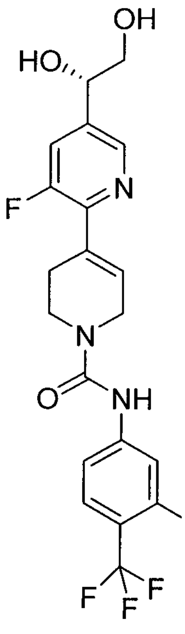

,

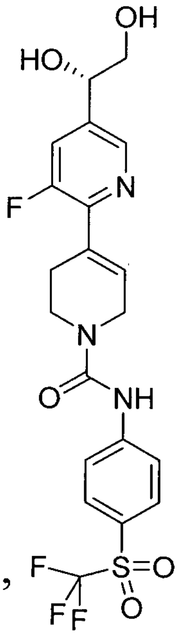

,

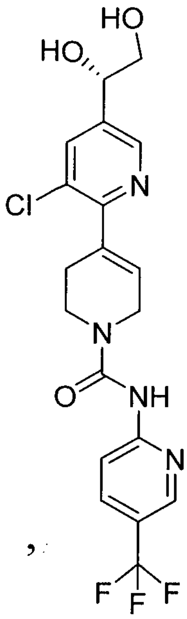

,

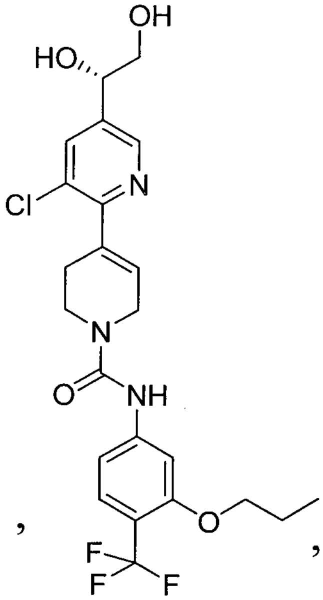

,

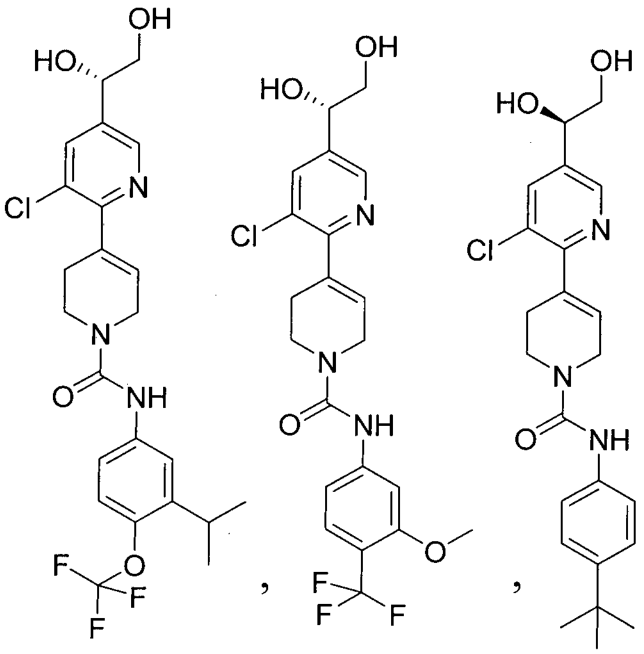

,

,

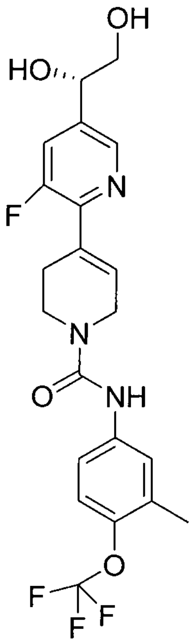

,

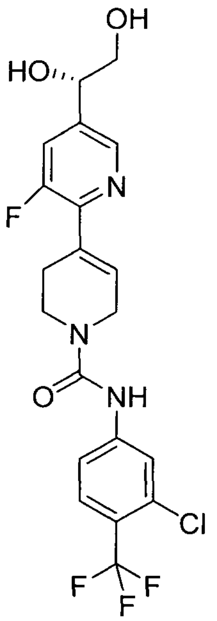

,

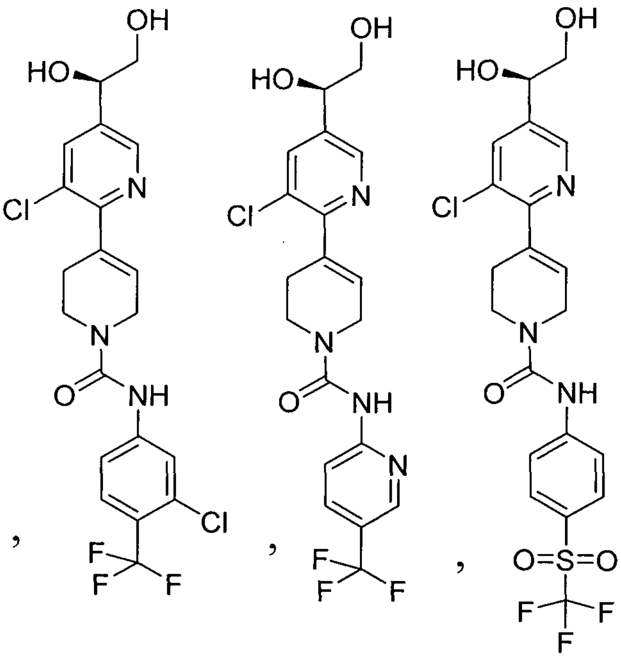

,

,

,

or

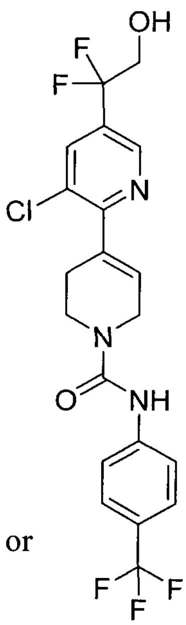

and the pharmaceutically acceptable salts and solvates thereof.

4.2 Compounds of Formula (II)

Preferred compounds of formula (I) are compounds of formula (II):

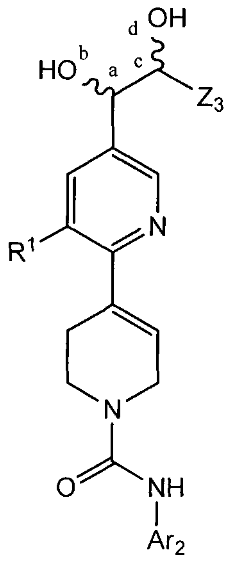

(II)

and the pharmaceutically acceptable salts and solvates thereof, where:

$R_1$ is -H, -halo, -($C_1$-$C_4$)alkyl, -C(halo)$_3$, -CH(halo)$_2$, or -$CH_2$(halo);
$Z_3$ is -H or -$CH_3$;
$Ar_2$ is as defined for compounds of formula (I); and
each halo is independently -F, -Cl, -Br, or -I.

Certain embodiments of formula (II) are presented below.
In one embodiment, a compound of formula (II) is a free base.
In another embodiment, a compound of formula (II) is a pharmaceutically acceptable salt or solvate of a compound of formula (II).
In another embodiment, a compound of formula (II) is a pharmaceutically acceptable salt.
In another embodiment, the compound of formula (II) is a free base of:

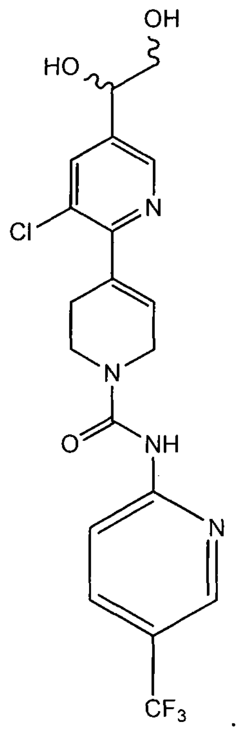

In another embodiment, the compound of formula (II) is a pharmaceutically acceptable salt of:

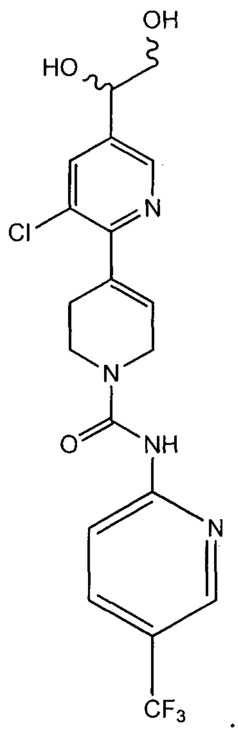

In another embodiment, the compound of formula (II) is a free base of:

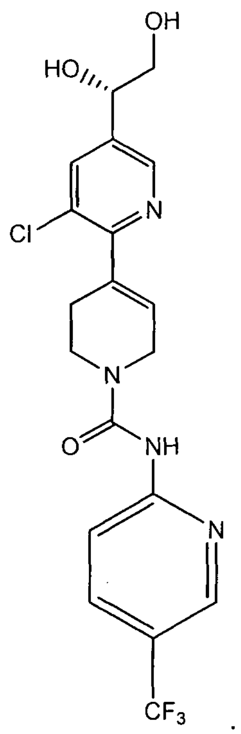

In another embodiment, the compound of formula (II) is a pharmaceutically acceptable salt of:

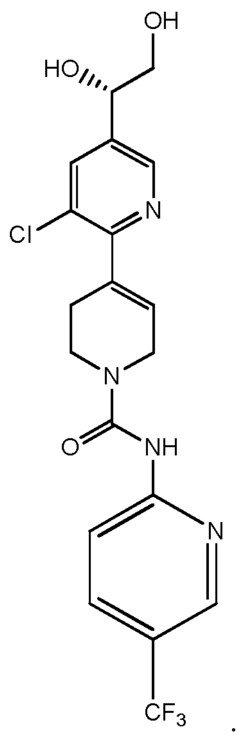

In another embodiment, the % *ee* of the compound is at least about 90 %, or at least 93%.

4.3 Definitions

As used herein, the terms used above having following meaning:
As used herein, the term "-($C_1$-$C_6$)alkyl" refers to straight-chain non-cyclic saturated hydrocarbons having from 1 to 6

carbon atoms, and branched non-cyclic saturated hydrocarbons having from 3 to 6 carbon atoms. Representative straight chain -($C_1$-$C_6$)alkyl groups include methyl, -ethyl, -n-propyl, -n-butyl, -n-pentyl, and -n-hexyl. Representative branched-chain-($C_1$-$C_6$)alkyl groups, having from 3 to 6 carbon atoms, include isopropyl, sec-butyl, isobutyl, tert-butyl, isopentyl, neopentyl, 1-methylbutyl, 2-methylbutyl, 3-methylbutyl, 1,1-dimethylpropyl, and 1,2-dimethylpropyl, methylpentyl, 2-methylpentyl, 3-methylpentyl, 4-methylpentyl, 1-ethylbutyl, 2-ethylbutyl, 3-ethylbutyl, 1,1-dimethylbutyl, 1,2-dimethylbutyl, 1,3-dimethylbutyl, 2,2-dimethylbutyl, 2,3-dimethylbutyl, 3,3-dimethylbutyl, and the like.

As used herein, "-($C_1$-$C_4$)alkyl" means a straight chain or branched non-cyclic hydrocarbon having 1, 2, 3, or 4 carbon atoms. Representative straight chain -($C_1$-$C_4$)alkyls include -methyl, -ethyl, -n-propyl, and -n-butyl. Representative branched -($C_1$-$C_4$)alkyls include -*iso*-propyl, -sec-butyl, -*iso*-butyl, and -tert-butyl.

As used herein, the term "-($C_2$-$C_6$)alkenyl" refers to straight chain non-cyclic hydrocarbons having from 2 to 6 carbon atoms and including at least one carbon-carbon double bond, and branched non-cyclic hydrocarbons having from 3 to 6 carbon atoms and including at least one carbon-carbon double bond. Representative straight chain and branched -($C_2$-$C_6$)alkenyl groups include -vinyl, -allyl, -1-butenyl, -2-butenyl, -isobutylenyl, -1-pentenyl, -2-pentenyl, -3-methyl-1-butenyl, -2-methyl-2-butenyl, and the like.

As used herein, the terms "halo" and "halogen" refer to fluoro, chloro, bromo or iodo.

As used herein, "-$CH_2$(halo)" means a methyl group where one of the hydrogens of the methyl group has been replaced with a halogen. Representative -$CH_2$(halo) groups include -$CH_2F$, -$CH_2Cl$, -$CH_2Br$, and -$CH_2I$.

As used herein, "-CH(halo)$_2$" means a methyl group where two of the hydrogens of the methyl group have been replaced with independently selected halogen atoms. Representative -CH(halo)$_2$ groups include -$CHF_2$, -$CHCl_2$, -$CHBr_2$, -CHBrCl, -CHClI, and -$CHI_2$.

As used herein, "-C(halo)$_3$" means a methyl group where each of the hydrogens of the methyl group has been replaced with independently selected halogen atoms. Representative -C(halo)$_3$ groups include -$CF_3$, -$CCl_3$, -$CBr_3$, and -$CI_3$.

As used herein, "-($C_1$-$C_6$)alkoxy" means a straight chain or branched non-cyclic hydrocarbon having one or more ether groups and from 1 to 6 carbon atoms. Representative straight chain and branched ($C_1$-$C_6$)alkoxys include methoxy, ethoxy, propoxy, butyloxy, pentyloxy, hexyloxy, methoxymethyl, 2-methoxyethyl, 5-methoxypentyl, 3-ethoxybutyl and the like. In a preferred embodiment, "-($C_1$-$C_6$)alkoxy" refers to a group -O-($C_1$-$C_6$)alkyl, wherein ($C_1$-$C_6$)alkyl is defined as above. Representative substituents preferably include methoxy, ethoxy, propoxy, butyloxy, pentyloxy, and hexyloxy.

As used herein, "-($C_1$-$C_6$)alkoxy($C_1$-$C_6$)alkyl" means a -($C_1$-$C_6$)alkyl group as defined above that is substituted with a -($C_1$-$C_6$)alkoxy.

As used herein, "-($C_1$-$C_6$)alkoxy($C_2$-$C_6$)alkenyl" means a -($C_2$-$C_6$)alkenyl group as defined above that is substituted with a -($C_1$-$C_6$)alkoxy group.

As used herein a "-($C_1$-$C_6$) haloalkyl" means any of the above-mentioned $C_{1-6}$ alkyl groups substituted by one or more halo groups. Representative ($C_1$-$C_6$) hydroxyalkyl groups include halomethyl, haloethyl, halopropyl and halobutyl groups, and especially halomethyl, 1-haloethyl, 2-haloethyl, 1,2-dihaloethyl, 2-halopropyl, 3-halopropyl, 3-halobutyl, 4-halobutyl, 2-halo-1-methylpropyl, and 1,3-dihaloprop-2-yl.

As used herein a "-($C_1$-$C_6$) hydroxyalkyl" means any of the above-mentioned $C_{1-6}$ alkyl groups substituted by one or more hydroxy groups. Representative ($C_1$-$C_6$) hydroxyalkyl groups include hydroxymethyl, hydroxyethyl, hydroxypropyl and hydroxybutyl groups, and especially hydroxymethyl, 1-hydroxyethyl, 2-hydroxyethyl, 1,2-dihydroxyethyl, 2-hydroxypropyl, 3-hydroxypropyl, 3-hydroxybutyl, 4-hydroxybutyl, 2-hydroxy-1-methylpropyl, and 1,3-dihydroxyprop-2-yl.

As used herein a "-($C_2$-$C_6$) haloalkenyl" means any of the above-mentioned $C_{2-6}$ alkenyl groups substituted by one or more halo groups. Representative ($C_2$-$C_6$) haloalkenyl groups include -halovinyl, -haloallyl, halobut-1-enyl, halobut-2-enyl, -haloisobutylenyl, and the like.

As used herein a "-($C_2$-$C_6$) hydroxyalkenyl" means any of the above-mentioned $C_{2-6}$ alkenyl groups substituted by one or more hydroxy groups. Representative ($C_2$-$C_6$) haloalkenyl groups include - hydroxyvinyl, - hydroxyallyl, hydroxybut-1-enyl, hydroxybut-2-enyl, -hydroxyisobutylenyl, and the like.

When a first group is "substituted with one or more" second groups, one or more hydrogen atoms of the first group is replaced with a corresponding number of second groups. When the number of second groups is two or greater, each second group can be the same or different. In one embodiment, the number of second groups is one or two. In another embodiment, the number of second groups is one.

In connection with the substituent of the pyridine ring containing $Z_3$, the phrase "wherein $Z_3$ is -$CH_3$ and the carbon atoms at the a and c positions of the a-b bond and the c-d bond are each in the (*S*) configuration" and the like means

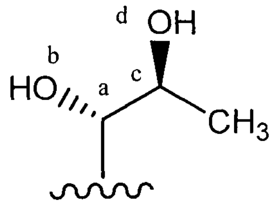

where the lower-case letters are used to designate a particular C-O bond in that substituent.

In connection with the substituent of the pyridine ring containing $Z_3$, the phrase "wherein $Z_3$ is $-CH_3$ and the carbon atoms at the a and c positions of the a-b bond and the c-d bond are each in the (*R*) configuration" and the like means

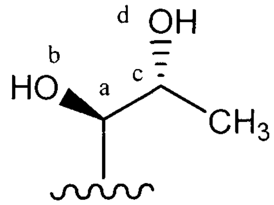

where the lower-case letters are used to designate a particular C-O bond in that substituent.

In connection with the substituent of the pyridine ring containing $Z_3$, the phrase "wherein $Z_3$ is $-CH_3$, the carbon atom at the a position of the a-b bond is in the (*R*) configuration, and the carbon atom at the c position of the c-d bond is in the (*S*) configuration" and the like means

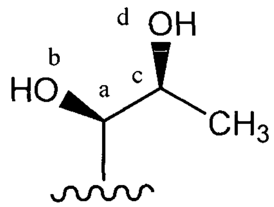

where the lower-case letters are used to designate a particular C-O bond in that substituent.

In connection with the substituent of the pyridine ring containing $Z_3$, the phrase "wherein $Z_3$ is $-CH_3$, the carbon atom at the a position of the a-b bond is in the (*S*) configuration, and the carbon atom at the c position of the c-d bond is in the (*R*) configuration" and the like means

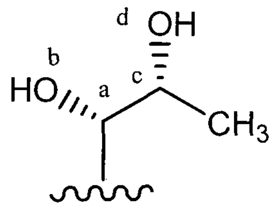

where the lower-case letters are used to designate a particular C-O bond in that substituent.

In connection with the substituent of the pyridine ring containing $Z_3$, the phrase "wherein $Z_3$ is -H and the carbon atom at the a position of the a-b bond is in the (*S*) configuration" and the like means

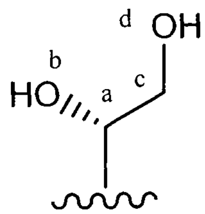

where the lower-case letters are used to designate a particular C-O bond in that substituent.

In connection with the substituent of the pyridine ring containing $Z_3$, the phrase "wherein $Z_3$ is -H and the carbon atom at the a position of the a-b bond is in the (*R*) configuration" and the like means

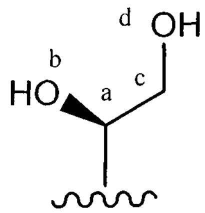

where the lower-case letters are used to designate a particular C-O bond in that substituent.

The term "animal," includes, but is not limited to, a cow, monkey, baboon, chimpanzee, horse, sheep, pig, chicken, turkey, quail, cat, dog, mouse, rat, rabbit, guinea pig, and human, and preferably refers to a human.

The phrase "pharmaceutically acceptable salt", as used herein, is any pharmaceutically acceptable salt that can be prepared from a compound of formula (I) including a salt formed from an acid and a basic functional group, such as a nitrogen group, of a compound of formula (I). Illustrative salts include, but are not limited, to sulfate, citrate, acetate, trifluoroacetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucoronate, saccharate, formate, benzoate, glutamate, methanesulfonate, ethanesulfonate, benzenesulfonate, *p*-toluenesulfonate, and pamoate (*i.e.*, 1,1'-methylene-bis-(2-hydroxy-3-naphthoate)) salts. The term "pharmaceutically acceptable salt" also includes a salt prepared from a compound of formula (I) having an acidic functional group, such as a carboxylic acid functional group, and a pharmaceutically acceptable inorganic or organic base. Suitable bases include, but are not limited to, hydroxides of alkali metals such as sodium, potassium, cesium, and lithium; hydroxides of alkaline earth metal such as calcium and magnesium; hydroxides of other metals, such as aluminum and zinc; ammonia and organic amines, such as unsubstituted or hydroxy-substituted mono-, di-, or trialkylamines; dicyclohexylamine; tributyl amine; pyridine; picoline; *N*-methyl-*N*-ethylamine; diethylamine; triethylamine; mono-, bis-, or tris-(2-hydroxy-($C_1$-$C_3$)alkyl amines), such as mono-, bis-, or tris-(2-hydroxyethyl)amine, 2-hydroxy-*tert*-butylamine, or tris-(hydroxymethyl)methylamine, *N*,*N*-di-[($C_1$-$C_3$)alkyl]-*N*-(hydroxy-($C_1$-$C_3$)alkyl)-amines, such as *N*,*N*-dimethyl-*N*-(2-hydroxyethyl)amine, or tri-(2-hydroxyethyl)amine; *N*-methyl-D-glucamine; and amino acids such as arginine, lysine, and the like. In one embodiment, the pharmaceutically acceptable salt is a hydrochloride-salt, a sulfate-salt, a sodium-salt, a potassium-salt, a benzene sulfonic acid-salt, a *para*-toluenesulfonic acid-salt, or a fumaric acid-salt. In another embodiment, the pharmaceutically acceptable salt is a hydrochloride-salt or a sulfate-salt. In another embodiment, the pharmaceutically acceptable salt is a hydrochloride-salt. In another embodiment, the pharmaceutically acceptable salt is a sulfate-salt. In another embodiment, the pharmaceutically acceptable salt is a sodium-salt. In another embodiment, the pharmaceutically acceptable salt is a potassium-salt. In another embodiment, the pharmaceutically acceptable salt is a *para*-toluenesulfonic acid-salt. One skilled in the art will recognize that, *e.g.*, acid addition salts, of a compound of formula (I) can be prepared by reaction of the compounds with the appropriate acid by a variety of known methods.

The compounds of the disclosure provided herein also encompass all solvates of the compounds of formula (I). "Solvates" are known in the art and are considered to be a combination, physical association and/or solvation of a compound of formula (I) with a solvent molecule. This physical association can involve varying degrees of ionic and covalent bonding, including hydrogen bonding. When the solvate is of the stoichiometric type, there is a fixed ratio of the solvent molecule to compound of formula (I), *e.g.*, a disolvate, monosolvate or hemisolvate when the solvent molecule:compound of formula (I) molecule molar ratio is 2:1, 1:1 or 1:2, respectively. In other embodiments, the solvate is of the nonstoichiometric type. For example, the compound of formula (I) crystal can contain solvent molecules in the structural voids, *e.g.*, channels, of the crystal lattice. In certain instances, the solvate can be isolated, for example when one or more solvent molecules are incorporated into the crystal lattice of a crystalline solid. Thus, "solvate", as used herein, encompasses both solution-phase and isolatable solvates. A compound of formula (I) of the disclosure can be present as a solvated form with a pharmaceutically acceptable solvent, such as water, methanol, ethanol, and the like, and it is intended that the disclosure include both solvated and unsolvated compound of formula (I) forms. As "hydrate" relates to a particular subgroup of solvates, *i.e.*, where the solvent molecule is water, hydrates are included within the solvates of the disclosure. In one embodiment, the compound of formula (I) is present as a monohydrate, *i.e.*, as a free base where the water:compound of formula (I) molar ratio is about 1:1, *e.g.*, from 0.91:1 to 1.09:1 in one embodiment, from 0.94:1 to 1.06:1 in another embodiment, from 0.97:1 to 1.03:1 in another embodiment, and from 0.985:1 to 1.015:1 in another embodiment, each said embodiment taking no account of surface water that might be present, if any.

Preparation of solvates is known in the art. For example, Caira et al., "Preparation and Crystal Characterization of a Polymorph, a Monohydrate, and an Ethyl Acetate Solvate of the Antifungal Fluconazole," J. Pharmaceut. Sci.,

93(3):601-611 (2004), describes the preparation of solvates of fluconazole with ethyl acetate and with water. Similar preparations of solvates, hemisolvate, hydrates, and the like are described by Van Tonder et al., "Preparation and Physicochemical Characterization of 5 Niclosamide Solvates and 1 Hemisolvate," AAPS Pharm. Sci. Tech., 5(1):Article 12 (2004), and Bingham et al., "Over one hundred solvates of sulfathiazole," Chem. Comm., pp. 603-604 (2001). In one embodiment, a non-limiting, process involves dissolving the compound of formula (I) in a desired amount of the desired solvent (organic, water or mixtures thereof) at temperatures above about 20°C to about 25°C, cooling the solution at a rate sufficient to form crystals, and isolating the crystals by known methods, *e.g.*, filtration. Analytical techniques, for example, infrared spectroscopy, can be used to show the presence of the solvent in a crystal of the solvate.

In addition, one or more hydrogen, carbon or other atoms of a compound of formula (I) can be replaced by a radioactive isotope of the hydrogen, carbon or other atoms. Such a "radiolabeled", "radiolabeled form", and the like of a compound of formula (I), each of which is encompassed by the disclosure, is useful as a research and/or diagnostic tool in metabolism pharmacokinetic studies and in binding assays. "Radioactive", as used herein with respect to an atom, means an atom that comprises a radioactive atom and therefore the specific radioactivity thereof is above the background level of radioactivity. Examples of radioactive isotopes that can be incorporated into a compound of formula (I) of the disclosure include isotopes of hydrogen, carbon, nitrogen, oxygen, phosphorous, sulfur, fluorine, chlorine, bromine, and iodine, such as $^{2}H$, $^{3}H$, $^{11}C$, $^{13}C$, $^{14}C$, $^{15}N$, $^{17}O$, $^{18}O$, $^{31}P$, $^{32}P$, $^{35}S$ , $^{18}F$, $^{19}F$, $^{36}Cl$, $^{37}Cl$, $^{76}Br$, $^{77}Br$, $^{81}Br$, $^{123}I$, $^{124}I$, $^{125}I$, and $^{131}I$, respectively. In one embodiment, a radiolabeled compound of formula (I) contains 1, 2, 3, 4, or more radioactive isotopes, each of which is independently selected from hydrogen, carbon, nitrogen, oxygen, phosphorous, sulfur, fluorine, chlorine, bromine, and iodine. In another embodiment, a radiolabeled compound of formula (I) contains 1 or 2 radioactive isotopes, each of which is independently selected from hydrogen, carbon, nitrogen, oxygen, phosphorous, sulfur, fluorine, chlorine, bromine, and iodine. In another embodiment, a radiolabeled compound of formula (I) contains 1 radioactive isotope which is selected from hydrogen, carbon, nitrogen, oxygen, phosphorous, sulfur, fluorine, chlorine, bromine, and iodine. In another embodiment, a radiolabeled compound of formula (I) contains 1, 2, 3, 4, or more radioactive isotopes, each of which is independently selected from $^{2}H$, $^{3}H$, $^{11}C$, $^{13}C$, $^{14}C$, $^{15}N$, $^{17}O$, $^{18}O$, $^{31}P$, $^{32}P$, $^{35}S$, $^{18}F$, $^{19}F$, $^{36}Cl$, $^{37}Cl$, $^{76}Br$, $^{77}Br$, $^{81}Br$, $^{123}I$, $^{124}I$, $^{125}I$, and $^{131}I$. In another embodiment, a radiolabeled compound of formula (I) contains 1 or 2 radioactive isotopes, each of which is independently selected from $^{2}H$, $^{3}H$, $^{11}C$, $^{13}C$, $^{14}C$, $^{15}N$, $^{17}O$, $^{18}O$, $^{31}P$, $^{32}P$, $^{35}S$, $^{18}F$, $^{19}F$, $^{36}Cl$, $^{37}Cl$, $^{76}Br$, $^{77}Br$, $^{81}Br$, $^{123}I$, $^{124}I$, $^{125}I$, and $^{131}I$. In another embodiment, a radiolabeled compound of formula (I) contains 1 radioactive isotope which is selected from $^{2}H$, $^{3}H$, $^{11}C$, $^{13}C$, $^{14}C$, $^{15}N$, $^{17}O$, $^{18}O$, $^{31}P$, $^{32}P$, $^{35}S$, $^{18}F$, $^{19}F$, $^{36}Cl$, $^{37}Cl$, $^{76}Br$, $^{77}Br$, $^{81}Br$, $^{123}I$, $^{124}I$, $^{125}I$, and $^{131}I$. In another embodiment, a radiolabeled compound of formula (I) contains 1, 2, 3, 4, or more radioactive isotopes, each of which is independently selected from $^{2}H$, $^{3}H$, $^{13}C$, $^{14}C$, $^{15}N$, $^{18}O$, $^{32}P$, and $^{125}I$. In another embodiment, a radiolabeled compound of formula (I) contains 1 or 2 radioactive isotopes, each of which is independently selected from $^{3}H$, $^{14}C$, $^{15}N$, $^{18}O$, $^{32}P$, and $^{125}I$. In another embodiment, a radiolabeled compound of formula (I) contains 1 radioactive isotope which is selected from $^{3}H$, $^{14}C$, $^{15}N$, $^{18}O$, $^{32}P$, and $^{125}I$.

Radiolabeled compounds of the disclosure can be prepared by methods known in the art. For example, tritiated compounds of formula (I) can be prepared by introducing tritium into the particular compound of formula (I), for example, by catalytic dehalogenation with tritium. This method can include reacting a suitably halogen-substituted precursor of a compound of formula (I) with tritium gas in the presence of a suitable catalyst, for example, Pd/C, in the presence or absence of a base. Other suitable methods for preparing tritiated compounds can be found in Filer, "The Preparation and Characterization of Tritiated Neurochemicals," Chapter 6, pp. 155-192 in Isotopes in the Physical and Biomedical Sciences, Vol. 1, Labeled Compounds (Part A) (1987). $^{14}C$-labeled compounds can be prepared by employing starting materials having a $^{14}C$ carbon. Compounds containing piperazine isotopcially enriched with $^{13}C$ and/or $^{15}N$ can be prepared as described in, *e.g.*, Figure 5A and the associated description, of U.S. Patent No. 7,355,045 B2.

A compound of formula (I) can contain one or more asymmetric centers and can thus give rise to enantiomers, diastereomers, and other stereoisomeric forms. Unless specifically otherwise indicated, the disclosure encompasses compounds with all such possible forms as well as their racemic and resolved forms or any mixture thereof. When a compound of formula (I) contains an olefinic double bond or other center of geometric asymmetry, and unless specifically otherwise indicated, it is intended to include all "geometric isomers", *e.g.*, both E and Z geometric isomers. Unless specifically otherwise indicated, all "tautomers", *e.g.*, ketone-enol, amide-imidic acid, lactam-lactim, enamine-imine, amine-imine, and enamine-enimine tautomers, are intended to be encompassed by the disclosure as well.

As used herein, the terms "stereoisomer", "stereoisomeric form", and the like are general terms for all isomers of individual molecules that differ only in the orientation of their atoms in space. It includes enantiomers and isomers of compounds with more than one chiral center that are not mirror images of one another ("diastereomers").

The term "chiral center" refers to a carbon atom to which four different groups are attached.

The term "enantiomer" or "enantiomeric" refers to a molecule that is nonsuperimposeable on its mirror image and hence optically active where the enantiomer rotates the plane of polarized light in one direction and its mirror image rotates the plane of polarized light in the opposite direction.

The term "racemic" refers to a mixture of equal parts of enantiomers which is optically inactive.

The term "resolution" refers to the separation or concentration or depletion of one of the two enantiomeric forms of a molecule. Optical isomers of a compound of formula (I) can be obtained by known techniques such as chiral chromatography or formation of diastereomeric salts from an optically active acid or base.

Optical purity can be stated in terms of enantiomeric excess (% *ee*), which is determined by the formula:

$$\% \, ee = \left[ \frac{\text{major enantiomer(mol)} - \text{minor enantiomer(mol)}}{\text{major enantiomer(mol)} + \text{minor enantiomer(mol)}} \right] \times 100\% .$$

The term "MeOH" means methanol, *i.e.*, methyl alcohol.

The term "EtOH" means ethanol, *i.e.*, ethyl alcohol.

The term "*t*-BuOH" means *tert*-butyl alcohol, *i.e.*, 2-methylpropan-2-ol.

The term "THF" means tetrahydrofuran.

The term "DMF" means *N,N*-dimethylformamide.

The term "DCM" means methylene chloride, *i.e.*, dichloromethane.

The term "DCE" means dichloroethane.

The term "DME" means 1,2-dimethoxyethane, *i.e.*, ethylene glycol dimethyl ether.

The term "EtOAc" means ethyl acetate.

The term "$NH_4OH$" means ammonium hydroxide.

The term "TEA" means triethylamine.

The term "MeCN" means acetonitrile.

The term "NaH" means sodium hydride.

The term "AcOH" means acetic acid.

The term "DMSO" means dimethylsulfoxide, *i.e.*, methylsulfinylmethane.

The term "DIEA" means diisopropylethylamine, *i.e.*, *N*-ethyl-*N*-isopropylpropan-2-amine.

The term "BuLi" means butyl lithium.

The term "BOC" means *tert*-butyloxycarbonyl:

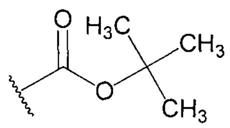

The term "HOBT" means 1-hydroxybenzotriazole hydrate.

The term "EDCI" means 1-ethyl-3-[3-(dimethylamino)propyl]carbodiimide.

The term "IBD" means inflammatory-bowel disease.

The term "IBS" means irritable-bowel syndrome.

The term "ALS" means amyotrophic lateral sclerosis.

The phrase "effective amount," when used in connection with a compound of formula (I) means an amount effective for: (a) treating a Condition or symptom thereof; (b) detectably inhibiting TRPV1 receptor function in a cell, or (c) detectably activating TRPV1 receptor function in a cell.

The phrase "effective amount," when used in connection with another therapeutic agent or a second therapeutic agent means an amount for providing the therapeutic effect of the second therapeutic agent.

The phrase "therapeutic index," describes the gap between the dose that is effective, and the dose that induces adverse effects.

The terms "modulate", "modulating", and the like as used herein with respect to the TRPV1 receptor mean the mediation of a pharmacodynamic response (*e.g.*, analgesia) in an animal from (i) inhibiting or activating the receptor, or (ii) directly or indirectly affecting the normal regulation of the receptor activity. Compounds that modulate the receptor activity include agonists, partial agonists, antagonists, mixed agonists/antagonists, mixed partial agonists/antagonists and compounds which directly or indirectly affect regulation of the receptor activity.

As used herein, a compound that binds to a receptor and mimics the regulatory effect(s) of an endogenous ligand is defined as an "agonist". As used herein, a compound that binds to a receptor and is only partly effective as an agonist is defined as a "partial agonist". As used herein, a compound that binds to a receptor but produces no regulatory effect, but rather blocks binding of another agent to the receptor is defined as an "antagonist". (See Ross and Kenakin, Pharmacodynamics: Mechanisms of Drug Action and the Relationship Between Drug Concentration and Effect, Chapter 2 in Goodman & Gilman's The Pharmacological Basis of Therapeutics 31-32 (Hardman et al., eds., 10th ed. 2001).

The phrases "treatment of," "treating" and the like include the amelioration or cessation of a Condition or a symptom thereof. In one embodiment, treating includes inhibiting, for example, decreasing the overall frequency of episodes of a Condition or a symptom thereof.

The phrases "prevention of," "preventing" and the like include the avoidance of the onset of a Condition or a symptom thereof.

A "disorder" includes, but is not limited to, the Conditions defined above.

In the event of doubt as to the agreement of a depicted chemical structure and a chemical name, the depicted chemical structure governs.

It is appreciated that various features of the disclosure which are, for clarity, described in the context of separate embodiments, can also be provided in combination in a single embodiment unless otherwise specifically herein excluded. Conversely, various features of the disclosure which are, for brevity, described in the context of a single embodiment, can also be provided separately and/or in any suitable subcombination unless otherwise specifically herein excluded.

4.4 Methods for Making Compounds of Formula (I)

The compounds of formula (I) can be made using conventional organic synthesis or by the illustrative methods provided in U.S. Pat. No 8,476,277 or U.S. Pat. No 7,776,861.

4.5 Therapeutic Uses of Compounds of Formula (I)

In accordance with the disclosure, the compounds of formula (I) are administered to an animal in need of treatment of a Condition.

In one embodiment, an effective amount of a compound of formula (I) can be used to treat any condition treatable by inhibiting TRPV1. Examples of Conditions that are treatable by inhibiting TRPV1 include, but are not limited to, pain, *e.g.*, pain associated with a burn injury originating from a thermal exposure, from a radiation exposure, from contact with a chemical agent, and/or from friction.

Applicants believe that the compounds of formula (I) and their pharmaceutically acceptable salts and solvates thereof are antagonists of TRPV1. The disclosure also relates to *in-vitro* methods for inhibiting TRPV1 function in a cell comprising contacting a cell capable of expressing TRPV1 with an effective amount of a compound of formula (I) or a pharmaceutically acceptable salt or solvate thereof. This method can be used *in vitro,* for example, as an assay to select cells that express TRPV1 and, accordingly, are useful as part of an assay to select compounds useful for treating pain, *e.g.*, pain associated with a burn injury originating from a thermal exposure, from a radiation exposure, from contact with a chemical agent, and/or from friction.

Examples of tissue comprising cells capable of expressing TRPV1 include, but are not limited to, neuronal, brain, kidney, urothelium, and bladder tissue. Methods for assaying cells that express TRPV1 are known in the art.

4.6 Therapeutic/Prophylactic Administration and Compositions of the Disclosure

Due to their activity, compounds of formula (I) are advantageously useful in veterinary and human medicine. As described above, compounds of formula (I) are useful for treating a Condition.

When administered to an animal, a compound of formula (I) or a pharmaceutically acceptable salt or solvate thereof is, in one embodiment, administered as a component of a composition that comprises a pharmaceutically acceptable carrier or excipient. The compositions, which comprise a compound of formula (I) or a pharmaceutically acceptable salt or solvate thereof, can be administered orally. Compounds of formula (I) and pharmaceutically acceptable salts and solvates thereof can also be administered by any other convenient route, for example, by infusion or bolus injection, by absorption through epithelial or mucocutaneous linings (*e.g.*, oral, rectal, and intestinal mucosa, *etc.*) and can be administered together with another therapeutically active agent. Administration can be systemic or local. Various delivery systems are known, *e.g.*, encapsulation in liposomes, microparticles, microcapsules, capsules, *etc.*, and can be used to administer the compound of formula (I).

Methods of administration include, but are not limited to, intradermal, intramuscular, intraperitoneal, intravenous, subcutaneous, intranasal, epidural, oral, sublingual, intracerebral, intravaginal, transdermal, rectal, by inhalation, or topical, particularly to the ears, nose, eyes, or skin. The mode of administration is left to the discretion of the practitioner. In most instances, administration will result in the release of compounds of formula (I) into the bloodstream.

In specific embodiments, it can be desirable to administer the compounds of formula (I) locally. This can be achieved, for example, and not by way of limitation, by local infusion during surgery, topical application, *e.g.*, in conjunction with a wound dressing after surgery, by injection, by means of a catheter, by means of a suppository or enema, or by means of an implant, said implant being of a porous, non-porous, or gelatinous material, including membranes, such as sialastic membranes, or fibers.

In certain embodiments, it can be desirable to introduce a compound of formula (I) or a pharmaceutically acceptable salt or solvate thereof into the central nervous system or gastrointestinal tract by any suitable route, including intraventricular, intrathecal, and epidural injection, and enema. Intraventricular injection can be facilitated by an intraventricular catheter, for example, attached to a reservoir, such as an Ommaya reservoir.

Pulmonary administration can also be employed, *e.g.*, by use of an inhaler or nebulizer, and formulation with an aerosolizing agent, or via perfusion in a fluorocarbon or synthetic pulmonary surfactant. In certain embodiments, a compound of formula (I) or a pharmaceutically acceptable salt or solvate thereof can be formulated as a suppository, with traditional binders and excipients such as triglycerides.

In another embodiment, a compound of formula (I) or a pharmaceutically acceptable salt or solvate thereof can be delivered in a vesicle, in particular a liposome (*see* Langer, "New Methods of Drug Delivery," Science 249:1527-1533 (1990); Lopez-Berestein, "Treatment of Systemic Fungal Infections with Liposomal-Amphotericin B," Liposomes in the Therapy of Infectious Disease and Cancer, pp. 317-327 (1989); and Treat et al., "Liposome encapsulated doxorubicin - preliminary results of phase I and phase II trials" Liposomes in the Therapy of Infectious Disease and Cancer, pp. 353-365 (1989).

In yet another embodiment, a compound of formula (I) or a pharmaceutically acceptable salt or solvate thereof can be delivered in a controlled-release system or sustained-release system (see, *e.g.,* Goodson, "Dental Applications," pp. 115-138 in Medical Applications of Controlled Release, Vol. 2, Applications and Evaluation, Langer and Wise, eds., CRC Press (1984), hereafter "Goodson"). Other controlled- or sustained-release systems discussed in the review by Langer, Science 249:1527-1533 (1990) can be used. In one embodiment, a pump can be used (Langer, Science 249:1527-1533 (1990); Sefton, "Implantable Pumps," in CRC Crit. Rev. Biomed. Eng. 14(3):201-240 (1987); Buchwald et al., "Long-term, Continuous Intravenous Heparin Administration by an Implantable Infusion Pump in Ambulatory Patients with Recurrent Venous Thrombosis," Surgery 88:507-516 (1980); and Saudek et al., "A Preliminary Trial of the Programmable Implantable Medication System for Insulin Delivery," New Engl. J. Med. 321:574-579 (1989)). In another embodiment, polymeric materials can be used (see Goodson; Smolen et al., "Drug Product Design and Performance," Controlled Drug Bioavailability Vol. 1, John Wiley & Sons, New York (1984); Langer et al., "Chemical and Physical Structure of Polymers as Carriers for Controlled Release of Bioactive Agents: A Review," J. Macromol. Sci. Rev. Macromol. Chem. C23(1):61-126 (1983); Levy et al., "Inhibition of Calcification of Bioprosthetic Heart Valves by Local Controlled-Release Diphosphonate," Science 228:190-192 (1985); During et al., "Controlled Release of Dopamine from a Polymeric Brain Implant: In Vivo Characterization," Ann. Neurol. 25:351-356 (1989); and Howard et al., "Intracerebral drug delivery in rats with lesion-induced memory deficits," J. Neurosurg. 71:105 (1989)). In yet another embodiment, a controlled- or sustained-release system can be placed in proximity of a target of the compounds of formula (I), *e.g.*, the spinal column, brain, or gastrointestinal tract, thus requiring only a fraction of the systemic dose.

The compositions can optionally comprise a suitable amount of a pharmaceutically acceptable excipient so as to provide the form for proper administration to the animal. Such a pharmaceutical excipient can be a diluent, suspending agent, solubilizer, binder, disintegrant, preservative, coloring agent, lubricant, and the like. The pharmaceutical excipient can be a liquid, such as water or an oil, including those of petroleum, animal, vegetable, or synthetic origin, such as peanut oil, soybean oil, mineral oil, sesame oil, and the like. The pharmaceutical excipient can be saline, gum acacia, gelatin, starch paste, talc, keratin, colloidal silica, urea, and the like. In addition, auxiliary, stabilizing, thickening, lubricating, and coloring agents can be used. In one embodiment, the pharmaceutically acceptable excipient is sterile when administered to an animal. Water is a particularly useful excipient when a compound of formula (I) is administered intravenously. Saline solutions and aqueous dextrose and glycerol solutions can also be employed as liquid excipients, particularly for injectable solutions. Suitable pharmaceutical excipients also include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol, and the like. The compositions, if desired, can also contain wetting or emulsifying agents, or pH buffering agents. Specific examples of pharmaceutically acceptable carriers and excipients that can be used to formulate oral dosage forms are described in the Handbook of Pharmaceutical Excipients, (Amer. Pharmaceutical Ass'n, Washington, DC, 1986).

The compositions can take the form of solutions, suspensions, emulsions, tablets, pills, pellets, multiparticulates, capsules, capsules containing liquids, powders, sustained-release formulations, suppositories, aerosols, sprays, ointments, gels, salves, plasters, transdermal patches, suspensions, or any other form suitable for use. In one embodiment, the composition is in the form of a capsule (see *e.g.*, U.S. Patent No. 5,698,155). Other examples of suitable pharmaceutical excipients are described by Radebough et al., "Preformulation," pp. 1447-1676 in Remington's Pharmaceutical Sciences Vol. 2 (Gennaro, ed., 19th ed., Mack Publishing, Easton, PA, 1995).

In one embodiment, a compound of formula (I) or a pharmaceutically acceptable salt or solvate thereof is formulated in accordance with routine procedures as a composition adapted for oral administration to human beings. A compound of formula (I), or a pharmaceutically acceptable salt or solvate thereof, to be orally delivered can be in the form of tablets, capsules, gelcaps, caplets, lozenges, aqueous or oily solutions, suspensions, granules, powders, emulsions, syrups, or elixirs, for example. When a compound of formula (I) or a pharmaceutically acceptable salt or solvate

thereof is incorporated into oral tablets, such tablets can be compressed, tablet triturates, enteric-coated, sugar-coated, film-coated, multiply compressed or multiply layered. Techniques and compositions for making solid oral dosage forms are described in Pharmaceutical Dosage Forms: Tablets (Lieberman et al., eds., 2nd ed., Marcel Dekker, Inc., 1989 & 1990). Techniques and compositions for making tablets (compressed and molded), capsules (hard and soft gelatin) and pills are also described by King, "Tablets, Capsules, and Pills," pp. 1553-1593 in Remington's Pharmaceutical Sciences (Osol, ed., 16th ed., Mack Publishing, Easton, PA, 1980).

Liquid oral dosage forms include aqueous and nonaqueous solutions, emulsions, suspensions, and solutions and/or suspensions reconstituted from non-effervescent granules, optionally containing one or more suitable solvents, preservatives, emulsifying agents, suspending agents, diluents, sweeteners, coloring agents, flavoring agents, and the like. Techniques and composition for making liquid oral dosage forms are described in Pharmaceutical Dosage Forms: Disperse Systems (Lieberman et al., eds., 2nd ed., Marcel Dekker, Inc., 1996 & 1998).

When a compound of formula (I), or a pharmaceutically acceptable salt or solvate thereof, is to be administered topically to the skin, it can formulated into creams, ointments, gels, salves, plasters, poultices, pastes, or transdermal patches, The preparation of topical pharmaceutical dosage forms is described generally in Remington: The Science and Practice of Pharmacy (Allen, Loyd V., Jr ed., 22nd edition, Pharmaceutical Press, 2012).

When a compound of formula (I) is to be injected parenterally, it can be, *e.g.*, in the form of an isotonic sterile solution. Alternatively, when a compound of formula (I) is to be inhaled, it can be formulated into a dry aerosol or can be formulated into an aqueous or partially aqueous solution.

An orally administered compound of formula (I) or a pharmaceutically acceptable salt or solvate thereof can contain one or more agents, for example, sweetening agents such as fructose, aspartame or saccharin; flavoring agents such as peppermint, oil of wintergreen, or cherry; coloring agents; and preserving agents, to provide a pharmaceutically palatable preparation. Moreover, where in tablet or pill form, the compositions can be coated to delay disintegration and absorption in the gastrointestinal tract thereby providing a sustained action over an extended period of time. Selectively permeable membranes surrounding an osmotically active driving compound are also suitable for orally administered compositions. In these latter platforms, fluid from the environment surrounding the capsule is imbibed by the driving compound, which swells to displace the agent or agent composition through an aperture. These delivery platforms can provide an essentially zero order delivery profile as opposed to the spiked profiles of immediate release formulations. A time-delay material such as glycerol monostearate or glycerol stearate can also be used. Oral compositions can include standard excipients such as mannitol, lactose, starch, magnesium stearate, sodium saccharin, cellulose, and magnesium carbonate. In one embodiment, the excipients are of pharmaceutical grade.

In another embodiment, a compound of formula (I) or a pharmaceutically acceptable salt or solvate thereof can be formulated for intravenous administration. In one embodiment, compositions for intravenous administration comprise sterile isotonic aqueous buffer. Where necessary, the compositions can also include a solubilizing agent. A compound of formula (I) or a pharmaceutically acceptable salt or solvate thereof for intravenous administration can optionally include a local anesthetic such as benzocaine or prilocaine to lessen pain at the site of the injection. Generally, the ingredients are supplied either separately or mixed together in unit dosage form, for example, as a dry lyophilized powder or water free concentrate in a hermetically sealed container such as an ampule or sachette indicating the quantity of active agent. Where a compound of formula (I) or a pharmaceutically acceptable salt or solvate thereof is to be administered by infusion, it can be dispensed, for example, with an infusion bottle containing sterile pharmaceutical grade water or saline. Where a compound of formula (I) or a pharmaceutically acceptable salt or solvate thereof is administered by injection, an ampule of sterile water for injection or saline can be provided so that the ingredients can be mixed prior to administration.

Compounds of formula (I) and the pharmaceutically acceptable salts and solvates thereof can be administered by controlled-release or sustained-release means or by delivery devices that are known to those of ordinary skill in the art. Examples include, but are not limited to, those described in U.S. Patent Nos.: 3,845,770; 3,916,899; 3,536,809; 3,598,123; 4,008,719; 5,674,533; 5,059,595; 5,591,767; 5,120,548; 5,073,543; 5,639,476; 5,354,556; and 5,733,566. Such dosage forms can be used to provide controlled- or sustained-release of one or more active ingredients using, for example, hydropropylmethyl cellulose, ethylcellulose, other polymer matrices, gels, permeable membranes, osmotic systems, multilayer coatings, microparticles, liposomes, microspheres, or a combination thereof to provide the desired release profile in varying proportions. Suitable controlled- or sustained-release formulations known to those of ordinary skill in the art, including those described herein, can be readily selected for use with the active ingredients of the disclosure. The disclosure thus encompasses single unit dosage forms suitable for oral administration such as, but not limited to, tablets, capsules, gelcaps, and caplets that are adapted for controlled- or sustained-release.

Controlled- or sustained-release pharmaceutical compositions can have a common goal of improving drug therapy over that achieved by their non-controlled or non-sustained release counterparts. In one embodiment, a controlled- or sustained-release composition comprises a minimal amount of a compound of formula (I) or a pharmaceutically acceptable salt or solvate thereof to cure or control the condition in a minimum amount of time. Advantages of controlled- or sustained-release compositions include extended activity of the drug, reduced dosage frequency, and increased patient compliance. In addition, controlled- or sustained-release compositions can favorably affect the time of onset of

action or other characteristics, such as blood levels of the compound of formula (I), and can thus reduce the occurrence of adverse side effects.

Controlled- or sustained-release compositions can be designed to immediately release an amount of a compound of formula (I) or a pharmaceutically acceptable salt or solvate thereof that promptly produces the desired therapeutic or prophylactic effect, and gradually and continually release other amounts of the compound of formula (I) or a pharmaceutically acceptable salt or solvate thereof to maintain this level of therapeutic or prophylactic effect over an extended period of time. To maintain a constant level of the compound of formula (I) in the body, the compound of formula (I) or a pharmaceutically acceptable salt or solvate thereof can be released from the dosage form at a rate that will replace the amount of compound of formula (I) being metabolized and excreted from the body. Controlled- or sustained-release of an active ingredient can be stimulated by various conditions, including but not limited to, changes in pH, changes in temperature, concentration or availability of enzymes, concentration or availability of water, or other physiological conditions or compounds.

The amount of the compound of formula (I) or a pharmaceutically acceptable salt or solvate thereof that is effective in the treatment of a Condition can be determined by standard clinical techniques. In addition, *in vitro* or *in vivo* assays can optionally be employed to help identify optimal dosage ranges. The precise dose to be employed will also depend on the route of administration, and the seriousness of the Condition and can be decided according to the judgment of a practitioner and/or each animal's circumstances. Suitable effective dosage amounts, however, will, in one embodiment, range from about 0.01 mg/kg of body weight to about 2500 mg/kg of body weight. In another embodiment, effective dosage amounts will be about 100 mg/kg of body weight or less. In one embodiment, the effective dosage amount ranges from about 0.01 mg/kg of body weight to about 100 mg/kg of body weight of a compound of formula (I) or a pharmaceutically acceptable salt or solvate thereof; in another embodiment, about 0.02 mg/kg of body weight to about 50 mg/kg of body weight; and in another embodiment, about 0.025 mg/kg of body weight to about 20 mg/kg of body weight.

In one embodiment, an effective dosage amount is administered about every 24 h until the Condition is abated. In another embodiment, an effective dosage amount is administered about every 12 h until the Condition is abated. In another embodiment, an effective dosage amount is administered about every 8 h until the Condition is abated. In another embodiment, an effective dosage amount is administered about every 6 h until the Condition is abated. In another embodiment, an effective dosage amount is administered about every 4h until the Condition is abated.

The effective dosage amounts described herein refer to total amounts administered; that is, if more than one compound of formula (I) is administered, the effective dosage amounts correspond to the total amount administered.

Where a cell capable of expressing TRPV1 is contacted with a compound of formula (I) *in vitro,* the amount effective for inhibiting the TRPV1 receptor function in a cell will range from about 0.01 μg/L to about 5 mg/L; in one embodiment, from about 0.01 μg/L to about 2.5 mg/L; in another embodiment, from about 0.01 μg/L to about 0.5 mg/L; and in another embodiment, from about 0.01 μg/L to about 0.25 mg/L, of a solution or suspension of a pharmaceutically acceptable carrier or excipient. In one embodiment, the volume of solution or suspension comprising the compound of formula (I) or a pharmaceutically acceptable salt or solvate thereof, is from about 0.01 μL to about 1mL. In another embodiment, the volume of solution or suspension is about 200 μL.

The compounds of formula (I), and the pharmaceutically acceptable salts and solvates thereof, can be assayed *in vitro* or *in vivo* for the desired therapeutic or prophylactic activity prior to use in humans. Animal model systems can be used to demonstrate safety and efficacy.

The methods for treating a Condition in an animal in need thereof can further comprise administering a second therapeutic agent to the animal being administered a compound of formula (I) or a pharmaceutically acceptable salt or solvate thereof (*i.e.*, a first therapeutic agent). In one embodiment, the first therapeutic agent is administered in an effective amount. In one embodiment, the second therapeutic agent is administered in an effective amount.

The methods for inhibiting TRPV1 function in a cell capable of expressing TRPV1 can further comprise contacting the cell with an effective amount of a second therapeutic agent.

An effective amount of the second therapeutic agent(s) will be known to those skilled in the art depending on the agent. However, it is well within the skilled artisan's purview to determine the second therapeutic agent's optimal effective-amount range. A compound of formula (I) or a pharmaceutically acceptable salt or solvate thereof, and the second therapeutic agent combined can act either additively or synergistically to treat the same Condition, or they can act independently of each other such that the compound of formula (I) or pharmaceutically acceptable salt or solvate thereof treats a first Condition and the second therapeutic agent treats or prevents a second disorder, which can be the same as the first Condition or another disorder. In one embodiment of the disclosure, where a second therapeutic agent is administered to an animal for treatment of a Condition (*e.g.*, pain), the minimal effective amount of the compound of formula (I) or pharmaceutically acceptable salt or solvate thereof will be less than its minimal effective amount would be where the second therapeutic agent is not administered. In this embodiment, the compound of formula (I) or pharmaceutically acceptable salt or solvate thereof and the second therapeutic agent can act synergistically to treat a Condition. In one embodiment, a compound of formula (I) or pharmaceutically acceptable salt or solvate thereof is administered concurrently with a second therapeutic agent as a single composition comprising an effective amount of a compound

of formula (I) or pharmaceutically acceptable salt or solvate thereof and an effective amount of the second therapeutic agent. Alternatively, a composition comprising an effective amount of a compound of formula (I) or pharmaceutically acceptable salt or solvate thereof and a second composition comprising an effective amount of the second therapeutic agent are concurrently administered. In another embodiment, an effective amount of a compound of formula (I) or pharmaceutically acceptable salt or solvate thereof is administered prior or subsequent to administration of an effective amount of the second therapeutic agent. In this embodiment, the compound of formula (I) or pharmaceutically acceptable salt or solvate thereof is administered while the second therapeutic agent exerts its therapeutic effect, or the second therapeutic agent is administered while the compound of formula (I) exerts its therapeutic effect for treating a Condition.

The second therapeutic agent can be, but is not limited to, an opioid agonist, a non-opioid analgesic, a non-steroidal anti-inflammatory agent, an antimigraine agent, a Cox-II inhibitor, an antiemetic, a β-adrenergic blocker, an anticonvulsant, an antidepressant, a $Ca^{2+}$-channel blocker, an anticancer agent, an agent for treating or preventing UI, an agent for treating or preventing an ulcer, an agent for treating or preventing IBD, an agent for treating or preventing IBS, an agent for treating addictive disorder, an agent for treating Parkinson's disease and parkinsonism, an agent for treating anxiety, an agent for treating epilepsy, an agent for treating a stroke, an agent for treating a seizure, an agent for treating a pruritic condition, an agent for treating psychosis, an agent for treating Huntington's chorea, an agent for treating ALS, an agent for treating a cognitive disorder, an agent for treating a migraine, an agent for treating vomiting, an agent for treating dyskinesia, an agent for treating depression, or any mixture thereof.

Examples of useful opioid agonists include, but are not limited to, alfentanil, allylprodine, alphaprodine, anileridine, benzylmorphine, bezitramide, buprenorphine, butorphanol, clonitazene, codeine, desomorphine, dextromoramide, dezocine, diampromide, diamorphone, dihydrocodeine, dihydromorphine, dimenoxadol, dimepheptanol, dimethylthiambutene, dioxaphetyl butyrate, dipipanone, eptazocine, ethoheptazine, ethylmethylthiambutene, ethylmorphine, etonitazene, fentanyl, heroin, hydrocodone, hydromorphone, hydroxypethidine, isomethadone, ketobemidone, levorphanol, levophenacylmorphan, lofentanil, meperidine, meptazinol, metazocine, methadone, metopon, morphine, myrophine, nalbuphine, narceine, nicomorphine, norlevorphanol, normethadone, nalorphine, normorphine, norpipanone, opium, oxycodone, oxymorphone, papaveretum, pentazocine, phenadoxone, phenomorphan, phenazocine, phenoperidine, piminodine, piritramide, proheptazine, promedol, properidine, propiram, propoxyphene, sufentanil, tilidine, tramadol, or any mixture thereof.

In certain embodiments, the opioid agonist is codeine, hydromorphone, hydrocodone, oxycodone, dihydrocodeine, dihydromorphine, morphine, tramadol, oxymorphone, or any mixture thereof.

Examples of useful non-opioid analgesics include, but are not limited to, non-steroidal anti-inflammatory agents, such as aspirin, ibuprofen, diclofenac, naproxen, benoxaprofen, flurbiprofen, fenoprofen, flubufen, ketoprofen, indoprofen, piroprofen, carprofen, oxaprozin, pramoprofen, muroprofen, trioxaprofen, suprofen, aminoprofen, tiaprofenic acid, fluprofen, bucloxic acid, indomethacin, sulindac, tolmetin, zomepirac, tiopinac, zidometacin, acemetacin, fentiazac, clidanac, oxpinac, mefenamic acid, meclofenamic acid, flufenamic acid, niflumic acid, tolfenamic acid, diflurisal, flufenisal, piroxicam, sudoxicam, isoxicam, or any mixture thereof. Other suitable non-opioid analgesics include the following, non-limiting, chemical classes of analgesic, antipyretic, nonsteroidal anti-inflammatory drugs: salicylic acid derivatives, including aspirin, sodium salicylate, choline magnesium trisalicylate, salsalate, diflunisal, salicylsalicylic acid, sulfasalazine, and olsalazin; para-aminophenol derivatives including acetaminophen and phenacetin; indole and indene acetic acids, including indomethacin, sulindac, and etodolac; heteroaryl acetic acids, including tolmetin, diclofenac, and ketorolac; anthranilic acids (fenamates), including mefenamic acid and meclofenamic acid; enolic acids, including oxicams (piroxicam, tenoxicam), and pyrazolidinediones (phenylbutazone, oxyphenthartazone); alkanones, including nabumetone; or any mixture thereof. For a more detailed description of the NSAIDs, see Insel, "Analgesic-Antipyretic and Anti-inflammatory Agents and Drugs Employed in the Treatment of Gout," pp. 617-657 in Goodman & Gilman's The Pharmacological Basis of Therapeutics (Goodman et al., Eds., 9th Ed., McGraw-Hill, New York 1996), and Hanson, "Analgesic, Antipyretic and Anti-Inflammatory Drugs," pp. 1196-1221 in Remington: The Science and Practice of Pharmacy Vol 2 (Gennaro, ed., 19th ed., Mack Publishing, Easton, PA, 1995).

Examples of useful antimigraine agents include, but are not limited to, alpiropride, bromocriptine, dihydroergotamine, dolasetron, ergocornine, ergocorninine, ergocryptine, ergonovine, ergot, ergotamine, flumedroxone acetate, fonazine, ketanserin, lisuride, lomerizine, methylergonovine, methysergide, metoprolol, naratriptan, oxetorone, pizotyline, propranolol, risperidone, rizatriptan, sumatriptan, timolol, trazodone, zolmitriptan, or any mixture thereof.

Examples of useful Cox-II inhibitors and 5-lipoxygenase inhibitors, as well as combinations thereof, are described in U.S. Patent No. 6,136,839. Examples of useful Cox-II inhibitors include, but are not limited to, celecoxib, DUP-697, flosulide, meloxicam, 6-MNA, L-745337, rofecoxib, nabumetone, nimesulide, NS-398, SC-5766, T-614, L-768277, GR-253035, JTE-522, RS-57067-000, SC-58125, SC-078, PD-138387, NS-398, flosulide, D-1367, SC-5766, PD-164387, etoricoxib, valdecoxib, parecoxib, or any mixture thereof.

The second therapeutic agent can also be an agent useful for reducing any potential side effects of a compound of formula (I). For example, the second therapeutic agent can be an antiemetic agent. Examples of useful antiemetic agents include, but are not limited to, metoclopromide, domperidone, prochlorperazine, promethazine, chlorpromazine,

trimethobenzamide, ondansetron, granisetron, hydroxyzine, acetylleucine monoethanolamine, alizapride, azasetron, benzquinamide, bietanautine, bromopride, buclizine, clebopride, cyclizine, dimenhydrinate, diphenidol, dolasetron, meclizine, methallatal, metopimazine, nabilone, oxyperndyl, pipamazine, scopolamine, sulpiride, tetrahydrocannabinol, thiethylperazine, thioproperazine, tropisetron, or any mixture thereof.

Examples of useful β-adrenergic blockers include, but are not limited to, acebutolol, alprenolol, amosulabol, arotinolol, atenolol, befunolol, betaxolol, bevantolol, bisoprolol, bopindolol, bucumolol, bufetolol, bufuralol, bunitrolol, bupranolol, butidrine hydrochloride, butofilolol, carazolol, carteolol, carvedilol, celiprolol, cetamolol, cloranolol, dilevalol, epanolol, esmolol, indenolol, labetalol, levobunolol, mepindolol, metipranolol, metoprolol, moprolol, nadolol, nadoxolol, nebivalol, nifenalol, nipradilol, oxprenolol, penbutolol, pindolol, practolol, pronethalol, propranolol, sotalol, sulfinalol, talinolol, tertatolol, tilisolol, timolol, toliprolol, xibenolol, or any mixture thereof.

Examples of useful anticonvulsants include, but are not limited to, acetylpheneturide, albutoin, aloxidone, aminoglutethimide, 4-amino-3-hydroxybutyric acid, atrolactamide, beclamide, buramate, calcium bromide, carbamazepine, cinromide, clomethiazole, clonazepam, decimemide, diethadione, dimethadione, doxenitroin, eterobarb, ethadione, ethosuximide, ethotoin, felbamate, fluoresone, gabapentin, 5-hydroxytryptophan, lamotrigine, magnesium bromide, magnesium sulfate, mephenytoin, mephobarbital, metharbital, methetoin, methsuximide, 5 -methyl-5 -(3 -phenanthryl)-hydantoin, 3 -methyl-5 -phenylhydantoin, narcobarbital, nimetazepam, nitrazepam, oxcarbazepine, paramethadione, phenacemide, phenetharbital, pheneturide, phenobarbital, phensuximide, phenylmethylbarbituric acid, phenytoin, phethenylate sodium, potassium bromide, pregabaline, primidone, progabide, sodium bromide, solanum, strontium bromide, suclofenide, sulthiame, tetrantoin, tiagabine, topiramate, trimethadione, valproic acid, valpromide, vigabatrin, zonisamide, or any mixture thereof.

Examples of useful antidepressants include, but are not limited to, binedaline, caroxazone, citalopram, (S)-citalopram, dimethazan, fencamine, indalpine, indeloxazine hydrocholoride, nefopam, nomifensine, oxitriptan, oxypertine, paroxetine, sertraline, thiazesim, trazodone, benmoxine, iproclozide, iproniazid, isocarboxazid, nialamide, octamoxin, phenelzine, cotinine, rolicyprine, rolipram, maprotiline, metralindole, mianserin, mirtazepine, adinazolam, amitriptyline, amitriptylinoxide, amoxapine, butriptyline, clomipramine, demexiptiline, desipramine, dibenzepin, dimetacrine, dothiepin, doxepin, fluacizine, imipramine, imipramine N-oxide, iprindole, lofepramine, melitracen, metapramine, nortriptyline, noxiptilin, opipramol, pizotyline, propizepine, protriptyline, quinupramine, tianeptine, trimipramine, adrafinil, benactyzine, bupropion, butacetin, dioxadrol, duloxetine, etoperidone, febarbamate, femoxetine, fenpentadiol, fluoxetine, fluvoxamine, hematoporphyrin, hypericin, levophacetoperane, medifoxamine, milnacipran, minaprine, moclobemide, nefazodone, oxaflozane, piberaline, prolintane, pyrisuccideanol, ritanserin, roxindole, rubidium chloride, sulpiride, tandospirone, thozalinone, tofenacin, toloxatone, tranylcypromine, L-tryptophan, venlafaxine, viloxazine, zimeldine, or any mixture thereof.

Examples of useful $Ca^{2+}$-channel blockers include, but are not limited to, bepridil, clentiazem, diltiazem, fendiline, gallopamil, mibefradil, prenylamine, semotiadil, terodiline, verapamil, amlodipine, aranidipine, barnidipine, benidipine, cilnidipine, efonidipine, elgodipine, felodipine, isradipine, lacidipine, lercanidipine, manidipine, nicardipine, nifedipine, nilvadipine, nimodipine, nisoldipine, nitrendipine, cinnarizine, flunarizine, lidoflazine, lomerizine, bencyclane, etafenone, fantofarone, perhexiline, or any mixture thereof.

Examples of useful therapeutic agents for treating or preventing anxiety include, but are not limited to, benzodiazepines, such as alprazolam, brotizolam, chlordiazepoxide, clobazam, clonazepam, clorazepate, demoxepam, diazepam, estazolam, flumazenil, flurazepam, halazepam, lorazepam, midazolam, nitrazepam, nordazepam, oxazepam, prazepam, quazepam, temazepam, and triazolam; non-benzodiazepine agents, such as buspirone, gepirone, ipsapirone, tiospirone, zolpicone, zolpidem, and zaleplon; tranquilizers, such as barbituates, *e.g.*, amobarbital, aprobarbital, butabarbital, butalbital, mephobarbital, methohexital, pentobarbital, phenobarbital, secobarbital, and thiopental; propanediol carbamates, such as meprobamate and tybamate; or any mixture thereof.

Examples of useful therapeutic agents for treating or preventing a pruritic condition include, but are not limited to, naltrexone; nalmefene; danazol; tricyclics such as amitriptyline, imipramine, and doxepin; antidepressants such as those given below, menthol; camphor; phenol; pramoxine; capsaicin; tar; steroids; antihistamines; or any mixture thereof.

Examples of useful therapeutic agents for treating or preventing vomiting include, but are not limited to, 5-$HT_3$ receptor antagonists such as ondansetron, dolasetron, granisetron, and tropisetron; dopamine receptor antagonists such as prochlorperazine, thiethylperazine, chlorpromazin, metoclopramide, and domperidone; glucocorticoids such as dexamethasone; benzodiazepines such as lorazepam and alprazolam; or any mixture thereof.

Examples of useful therapeutic agents for treating or preventing depression include, but are not limited to, tricyclic antidepressants such as amitryptyline, amoxapine, bupropion, clomipramine, desipramine, doxepin, imipramine, maprotilinr, nefazadone, nortriptyline, protriptyline, trazodone, trimipramine, and venlaflaxine; selective serotonin reuptake inhibitors such as citalopram, (S)-citalopram, fluoxetine, fluvoxamine, paroxetine, and setraline; monoamine oxidase inhibitors such as isocarboxazid, pargyline, phenelzine, and tranylcypromine; psychostimulants such as dextroamphetamine and methylphenidate; or any mixture thereof.

A composition of the disclosure is prepared by a method comprising admixing a compound of formula (I) or a pharmaceutically acceptable salt or solvate thereof with a pharmaceutically acceptable carrier or excipient. Admixing

can be accomplished using methods known for admixing a compound and a pharmaceutically acceptable carrier or excipient. In one embodiment, the compound of formula (I) or pharmaceutically acceptable salt or solvate thereof is present in the composition in an effective amount.

4.7 Kits

The disclosure further provides kits that can simplify the handling and administration of a compound of formula (I) to an animal.

In one embodiment, a kit of the disclosure comprises a unit dosage form of a compound of formula (I). In one embodiment, the unit dosage form comprises a first container, which can be sterile, containing an effective amount of a compound of formula (I) and a pharmaceutically acceptable carrier or excipient. The kit can further comprise a label or printed instructions instructing the use of the compound of formula (I) to treat a Condition. The kit can further comprise a unit dosage form of a second therapeutic agent, for example, a second container containing an effective amount of the second therapeutic agent and a pharmaceutically acceptable carrier or excipient. In another embodiment, the kit comprises a container containing an effective amount of a compound of formula (I), an effective amount of a second therapeutic agent and a pharmaceutically acceptable vehicle, carrier, or excipient. Examples of second therapeutic agents include, but are not limited to, those listed above.

Kits of the disclosure can further comprise a device that is useful for administering the unit dosage forms. Examples of such a device include, but are not limited to, a syringe, a drip bag, a patch, an inhaler, and an enema bag.

The following examples are set forth to assist in understanding the invention and should not be construed as specifically limiting the invention described and claimed herein.

5. EXAMPLES

***Example 1: In Vivo Assays to Demonstrate Prevention or Treatment of Burn Pain***

**Test Animals:** Each experiment used male Sprague-Dawley rats weighing between 190-260 g at the time of thermal burn injury. The rats were housed two per cage, and were allowed to acclimate to their environment for at least six days before testing began. The rats had free access to food and water at all times, except during testing and prior to oral (PO) administration of a test compound, when food was removed about 16 hours before dosing. The rats were divided into treatment groups, and one of the following compounds was administered to each group at the dose and by the route of administration indicated in Tables 5.2A to 5.2C, 5.3A and 5.3B, and 5.4A and 5.4B: ketorolac (Sigma-Aldrich, St. Louis, MO), AMG 517 (selective TRPV1 antagonist described in Em. Doherty et al., "Novel vanilloid receptor-1 antagonists:2. Structure-activity relationships of 4-oxopyrimidines leading to the selection of a clinical candidate," J Med Chem 50:3515-3527 (2007).) (Purdue Pharma L.P., Cranbury, NJ), indomethacin (Alexis Biochemicals, Farmingdale, NY), gabapentin (Bosche Scientific, New Brunswick, NJ), or (*S*)-3-chloro-5-(1,2-dihydroxyethyl)-N-(5-(trifluoromethyl)pyridin-2-yl)-5',6'-dihydro-[2,4'-bipyridine]-1'(2'*H*)-carboxamide (Compound 1). The control groups were administered the vehicle for the compound of formula (I). The volume of administration for PO treatment was 5 ml/kg, and was the same for all treatment groups. Volume of administration for intraperitoneal (i.p.) drug administration was 2 ml/kg.

**Burn Pain**: To assess the actions of a compound of formula (I) for the treatment or prevention of burn pain, a model of thermal burn pain was used. A thermal burn injury was induced under isofluorane/$O_2$ inhalation anesthesia. To assess mechanical hyperalgesia and weight bearing, a thermal injury was produced by immersing the left hind paw into water maintained at 55°C for 24 seconds. The left hind paw of non-injured control animals was immersed in 22 °C water for 24 seconds. To assess thermal hyperalgesia, a thermal injury was produced by immersing the left hindpaw into water maintained at 50 °C for 48 seconds; the left hind-paw of non-injured control animals was immersed in 22 °C water for 48 seconds. The animals were assessed for response to noxious mechanical stimuli by determining PWT for mechanical hyperalgesia, weight bearing difference between the injured or non-injured side, or response to noxious thermal stimuli by determining PWL for thermal hyperalgesia, all as described below, prior to drug administration (baseline), then again at 1, 3 and 5 hours after induction of burn injury.

**Response to Mechanical Stimuli as an Assessment of Mechanical Hyperalgesia:** The paw pressure assay was used to assess mechanical hyperalgesia. For this assay, hind paw withdrawal thresholds (PWT) to a noxious mechanical stimulus were determined using an analgesimeter (Model 7200, commercially available from Ugo Basile of Italy) as described in C. Stein, "Unilateral Inflammation of the Hindpaw in Rats as a Model of Prolonged Noxious Stimulation: Alterations in Behavior and Nociceptive Thresholds," Pharmacol. Biochem. and Behavior 31:451-455 (1988). The rat's paw was placed on a small platform, and weight was applied in a graded manner up to a maximum of 250 grams. The endpoint was taken as the weight at which the paw was completely withdrawn. PWT was determined once for each rat at each time point, wherein the affected (ipsilateral; same side as the injury) rear paw was tested. Burn injury induced mechanical hyperalgesia was demonstrated by a decrease in PWT from baseline (expressed as a % decrease

from baseline), and a smaller % decrease from baseline indicated less hyperalgesia. Three sets of tests were performed, and the results are summarized in Tables 5.2A, 5.2B and 5.2C. All drug treatment groups were compared to the vehicle control group (*i.e.* injured, administered only the vehicle).

Table 5.2A: Series 1A - Mechanical Hyperalgesia Results after Dosing with Several Medicaments (n=10 rats/ treatment group)

| | Paw Withdrawal Threshold (g) (mean ± S.E.M.) | | | | | |
|---|---|---|---|---|---|---|
| Time after Administration (hours) | Gabapentin | AMG 517 | Ketorolac | Indomethacin | Control | Vehicle |
| | 100 mg/kg IP in 0.9% NaCl | 30 mg/kg PO | 10 mg/kg PO | 30 mg/kg PO | -- PO | -- PO |
| Baseline | 239 ± 7.7 | 242 ± 6.0 | 246 ± 4.5 | 240 ± 7.5 | 241 ± 9.0 | 237 ± 9.2 |
| 1 | 152 ± 14.1†† | 94 ± 5.3 | 84 ± 6.1 | 118* ± 7.5 | 241 ± 6.4 | 76 ± 7.0 |
| 3 | 178 ± 18.7†† | 115 ± 10.8 | 97 ± 7.5 | 133‡ ± 15.8 | 241 ± 7.0 | 75 ± 7.4 |
| 5 | 112 ± 124 | 86 ± 7.7 | 91 ± 7.1 | 103 ± 10.2 | 241 ± 8.0 | 75 ± 7.9 |
| | | | | | | |
| | % Decrease from Baseline (mean ± S.E.M.) | | | | | |
| Time after Administration (hours) | Gabapentin | AMG 517 | Ketorolac | Indomethacin | Control | Vehicle |
| | 100 mg/kg IP in 0.9% NaCl | 30 mg/kg PO | 10 mg/kg PO | 30 mg/kg PO | -- PO | -- PO |
| 1 | 37 ± 5.3†† | 61 ± 2.3 | 66 ± 2.2 | 51 ± 2.6* | -0.7 ± 2.3 | 68 ± 2.5 |
| 3 | 25 ± 8.2†† | 52 ± 4.3 | 60 ± 3.8 | 45 ± 5.8† | -0.9 ± 1.4 | 68 ± 3.2 |
| 5 | 53 ± 6.0 | 65 ± 2.9 | 63 ± 34 | 58 ± 3.6 | -0.2 ± 0.8 | 68 ± 3.0 |

Table 5.2B: Series 1B - Mechanical Hyperalgesia Results after Dosing with Several Medicaments (n=10 rats/ treatment group)

| Time after Administration (hours) | Paw Withdrawal Threshold (g) (mean ± S.E.M.) | | | | | |
|---|---|---|---|---|---|---|
| | Gabapentin | | | | Control | Vehicle |
| | 3 mg/kg IP | 10 mg/kg IP | 30 mg/kg IP | 100 mg/kg IP in 0.9% NaCl | -- PO | -- PO |
| Baseline | 232 ± 10.0 | 231 ± 9.7 | 237 ± 7.9 | 237 ± 7.8 | 235 ± 10.1 | 232 ± 11.0 |
| 1 | 90 ± 4.1 | 122 ± 9.1 | 144 ± 7.2† | 180 ± 13.7†† | 220 ± 9.9 | 87 ± 5.1 |
| 3 | 102 ± 7.4 | 130 ± 8.2 | 174 ± 14.8†† | 229 ± 9.4†† | 230 ± 7.7 | 96 ± 5.0 |
| 5 | 81 ± 6.5 | 100 ± 8.7 | 105 ± 11.2 | 105 ± 9.5 | 234 ± 7.4 | 95 ± 9.3 |
| | | | | | | |
| | % Decrease from Baseline (mean ± S.E.M.) | | | | | |
| Time after Administration (hours) | Gabapentin | | | | Control | Vehicle |
| | 3 mg/kg IP | 10 mg/kg IP | 30 mg/kg IP | 100 mg/kg IP in 0.9% NaCl | -- PO | -- PO |
| 1 | 61 ± 2.1 | 47 ± 3.9 | 40 ± 2.0† | 23 ± 6.6†† | 6 ± 3.0 | 62 ± 3.1 |

(continued)

| Time after Administration (hours) | % Decrease from Baseline (mean ± S.E.M.) | | | | | |
|---|---|---|---|---|---|---|
| | Gabapentin | | | | Control | Vehicle |
| | 3 mg/kg IP | 10 mg/kg IP | 30 mg/kg IP | 100 mg/kg IP in 0.9% NaCl | -- PO | -- PO |
| 3 | 55 ± 3.7 | 43 ± 3.7 | 27 ± 5.1†† | 2 ± 6.7†† | 1 ± 3.0 | 58 ± 2.4 |
| 5 | 65 ± 3.0 | 57 ± 3.2 | 56 ± 4.0 | 55 ± 4.6 | -0.5 ± 3.1 | 59 ± 3.7 |

Table 5.2C: Series 2 - Mechanical Hyperalgesia Results after Dosing with Compound 1 (n=10 rats/ treatment group)

| Time after Administration (hours) | Paw Withdrawal Threshold (g) (mean ± S.E.M.) | | | | | |
|---|---|---|---|---|---|---|
| | Compound 1 | | | Gabapentin | Control | Vehicle |
| | 3 mg/kg PO | 10 mg/kg PO | 30 mg/kg PO | 100 mg/kg IP in 0.9% NaCl | -- PO | -- PO |
| Baseline | 240 ± 8.4 | 243 ± 6.5 | 244 ± 6.0 | 246 ± 4.5 | 240 ± 10.0 | 239 ± 9.0 |
| 1 | 97 ± 4.5 | 108 ± 73 | 153 ± 4.4†† | 145 ± 9.8†† | 216 ± 12.0 | 78 ± 5.0 |
| 3 | 88 ± 5.6 | 97 ± 8.5 | 141 ± 9.0†† | 192 ± 9.8†† | 236 ± 6.4 | 81 ± 7.4 |
| 5 | 90 ± 4.0 | 83 ± 5.4 | 95 ± 4.5 | 108 ± 5.8 | 234 ± 5.2 | 78 ± 7.7 |
| Time after Administration (hours) | % Decrease from Baseline (mean ± S.E.M.) | | | | | |
| | Compound 1 | | | Gabapentin | Control | Vehicle |
| | 3 mg/kg PO | 10 mg/kg PO | 30 mg/kg PO | 100 mg/kg IP in NaCl | -- PO | -- PO |
| 1 | 59 ± 1.8 | 55 ± 3.2 | 37 ± 2.5†† | 41 ± 3.6†† | 8.9 ± 5.0 | 67 ± 1.9 |
| 3 | 63.2 ± 2.1 | 60 ± 3.2 | 42 ± 4.5†† | 22 ± 4.1†† | 0.3 ± 4.2 | 66 ± 2.8 |
| 5 | 63 ± 1.4 | 66 ± 2.4 | 61 ± 2.0 | 56 ± 2.2 | 1.1 ± 4.1 | 67 ± 3.0 |
| * indicates P < 0.05<br>† indicates P < 0.001<br>†† indicates P < 0.0001 | | | | | | |

At 30 mg/kg, Compound 1 significantly reduced mechanical hyperalgesia associated with the burn injury.

**Response to Thermal Stimuli as an Assessment of Thermal Hyperalgesia:** The plantar test was used to assess thermal hyperalgesia. For this test, hind paw withdrawal latencies (PWL) to a noxious thermal stimulus were determined using a plantar test apparatus (commercially available from Ugo Basile of Italy) following the technique described by K. Hargreaves et al., "A New and Sensitive Method for Measuring Thermal Nociception in Cutaneous Hyperalgesia," Pain 32(1):77-88 (1988). The maximum exposure time was set at 32 seconds to avoid tissue damage

and any directed paw withdrawal from the heat source is taken as the end point. Three latencies were determined at each time point and averaged. Burn injury-induced thermal hyperalgesia was demonstrated by a decrease in PWL from baseline (expressed as a % decrease from baseline), and a smaller % decrease from baseline indicated less hyperalgesia. Compound 1 did not significantly affect thermal hyperalgesia. The results are provided in Tables 5.3A and 5.3B.

Table 5.3A: Series 1 - Thermal Hyperalgesia Results after Dosing with Several Medicaments

| Time after Administration (hours) | Paw Withdrawal Latency (sec) (mean ± S.E.M.) | | | | |
|---|---|---|---|---|---|
| | AMG 517 | Ketorolac | Indomethacin | Control | Vehicle |
| | 30 mg/kg PO | 10 mg/kg PO | 30 mg/kg PO | -- PO | -- PO |
| Baseline | 14.4 ± 1.35 | 13.2 ± 0.72 | 14.3 ± 1.26 | 13.9 ± 0.97 | 14.0 ± 1.06 |
| 1 | 8.6 ± 0.91 | 9.2 ± 0.96 | 8.3 ± 0.91 | 16.0 ± 0.61 | 6.3 ± 0.66 |
| 3 | 11.8 ± 0.66 | 11.9 ± 1.27 | 11.2 ± 0.87 | 14.5 ± 0.80 | 9.1 ± 0.47 |
| 5 | 12.1 ± 0.60 | 11.4 ± 0.54 | 11.4 ± 0.51 | 13.9 ± 0.57 | 9.1 ± 0.47 |
| | | | | | |
| | % Change from Baseline (mean ± S.E.M.) | | | | |
| Time after Administration (hours) | AMG 517 | Ketorolac | Indomethacin | Control | Vehicle |
| | 30 mg/kg PO | 10 mg/kg PO | 30 mg/kg PO | -- PO | -- PO |
| 1 | -50.1 ± 25.49 | -22.3 ± 6.30 | -41.5 ± 10.71 | 10.0 ± 4.43 | -48.3 ± 9.62 |
| 3 | -27.1 ± 16.54 | -6.7 ± 5.55 | -26.1 ± 13.18 | 0.9 ± 6.85 | -32.0 ± 8.29 |
| 5 | -24.8 ± 15.95 | -10.2 ± 3.05 | -25.5 ± 13.63 | -2.6 ± 6.14 | -326 ± 9.25 |

Table 5.3B: Series 2 - Thermal Hyperalgesia Results after Dosing with Compound 1

| Time after Administration (hours) | Paw Withdrawal Latency (sec) (mean ± S.E.M.) | | | | | |
|---|---|---|---|---|---|---|
| | Compound 1 | | | Ketorolac | Control | Vehicle |
| | 3 mg/kg PO | 10 mg/kg PO | 30 mg/kg PO | 30 mg/kg PO | -- PO | -- PO |
| Baseline | 14.5 ± 1.22 | 14.4 ± 1.17 | 13.7 ± 0.92 | 14.7 ± 1.29 | 14.4 ± 094 | 13.4 ± 1.19 |
| 1 | 8.1 ± 0.77 | 8.9 ± 1.08 | 7.8 ± 0.73 | 8.5 ± 0.89 | 17.2 ± 1.68 | 6.4 ± 0.66 |
| 3 | 9.2 ± 0.99 | 12.0 ± 0.66 | 11.5 ± 1.04 | 12.6 ± 1.33* | 15.0 ± 0.87 | 8.1 ± 0.65 |
| 5 | 9.9 ± 0.70 | 11.3 ± 0.98 | 11.2 ± 0.81 | 14.3 ± 0.80† | 14.9 ± 0.74 | 8.8 ± 0.55 |
| | | | | | | |
| | % Change from Baseline (mean ± S.E.M.) | | | | | |
| | Compound 1 | | | Ketorolac | Control | Vehicle |
| Time after Administration (hours) | 3 mg/kg PO | 10 mg/kg PO | 30 mg/kg PO | 30 mg/kg PO | -PO | -PO |
| 1 | -45.8 ± 14.53 | -36.0 ± 11.15 | -34.3 ± 6.08 | -47.6 ± 19.35 | 15.5 ± 8.83 | -44.6 ± 12.33 |
| 3 | -34.4 ± 7.57 | -19.7 ± 10.24 | -15.7 ± 10.29 | -24.0 ± 19.85 | 0.8 ± 8.65 | -35.3 ± 12.16 |

(continued)

| | % Change from Baseline (mean ± S.E.M.) | | | | | |
|---|---|---|---|---|---|---|
| | Compound 1 | | | Ketorolac | Control | Vehicle |
| Time after Administration (hours) | 3 mg/kg PO | 10 mg/kg PO | 30 mg/kg PO | 30 mg/kg PO | -PO | -PO |
| 5 | -35.7 ± 15.81 | -22.5 ± 9.23 | -16.6 ± 8.94 | -10.2 ± 12.40 | 0.9 ± 5.55 | -30.6 ± 10.91 |
| * indicates P < 0.05<br>† indicates P < 0.001 | | | | | | |

**Hind Limb Weight Bearing Between Injured and Non-Injured Paw as an Assessment of Pain:** A commercially available incapacitance meter was used to determine the weight distribution on the two hind paws of the animal (Linton Incapacitance Tester (Linton Instrumentation, Norfolk, UK)). The weight bearing difference (WBD) between the injured and non-injured hindpaw was measured. The animal was placed in the testing chamber that allows the animal to stand normally, distributing its body weight on the hind limbs for the test. Each hind paw was positioned on a separate pressure sensitive pad, which independently measures the pressure applied by each limb. The device continuously collects data after either a force threshold has been exceeded, or the investigator depresses the start pad. Rats were allowed to acclimate when placed on the apparatus, and when they were stationary, the force exerted was then continuously monitored for 3 s. The endpoint was the amount of pressure applied to each limb over time. Three readings were taken for each rat at each time point, and the average used for data analysis. WBD was expressed as "%WL", *i.e.* the percentage of weight borne on the burn-injured left hind paw, using the following formula:

$$\%WL = \frac{WLP}{(WLP + WRP)} \times 100$$

where WLP is the weight on the left hind paw and WRP is the weight on the right (untreated) hind paw. The 50% value of %WL corresponds to an equal weight distribution across both hind paws. For the weight bearing assay, the rat will shift its weight away from the injured paw to indicate the sensation of pain; weight borne more evenly on the non-injured and injured paw indicates a reduction in pain. Compound 1 did not significantly affect weight bearing. Two series of tests were performed; the results are provided in Tables 5.4A and 5.4B.

Table 5.4A: Series 1 - Weight Bearing Results after Dosing with Several Medicaments (n=9 rats/ treatment group)

| | %WL (mean ± S.E.M.) | | | | | |
|---|---|---|---|---|---|---|
| Time after Administration (hours) | Gabapentin | AMG 517 | Ketorolac | Indomethacin | Control | Vehicle |
| | 100 mg/kg IP in NaCl | 30 mg/kg PO | 10 mg/kg PO | 30 mg/kg PO | -- PO | -- PO |
| Baseline | 51.1 ± 0.57 | 49.4 ± 0.44 | 49.8 ± 0.31 | 49.1 ± 0.53 | 49.3 ± 1.00 | 50.5 ± 0.57 |
| 1 | 33.6 ± 3.09* | 39.2 ± 4.30† | 50.0 ± 2.20†† | 42.6 ± 3.81†† | 49.4 ± 1.07 | 22.5 ± 5.30 |
| 3 | 44.8 ± 2.58 | 43.1 ± 2.82 | 42.6 ± 1.91 | 42.6 ± 2.14 | 50.4 ± 1.00 | 44.4 ± 1.29 |
| 5 | 44.0 ± 2.24 | 43.0 ± 3.37 | 40.1 ± 2.72 | 43.7 ± 2.38 | 49.9 ± 0.86 | 40.5 ± 1.42 |

Table 5.4B: Series 2 - Weight Bearing Results after Dosing with Compound 1 (n=10 rats/ treatment group)

| Time after Administration (hours) | %WL (mean ± S.E.M.) | | | | | |
|---|---|---|---|---|---|---|
| | Compound 1 | | | Indomethacin | Control | Vehicle |
| | 3 mg/kg PO | 10 mg/kg PO | 30 mg/kg PO | 30 mg/kg PO | -- PO | -- PO |
| Baseline | 50.6 ± 0.62 | 50.6 ± 0.71 | 50.3 ± 0.53 | 50.4 ± 0.54 | 50.6 ± 0.55 | 50.5 ± 0.56 |
| 1 | 24.4 ± 4.44 | 37.6 ± 2.54 | 31.4 ± 3.92 | 43.2 ± 2.08* | 51.7 ± 1.57 | 30.3 ± 5.38 |
| 3 | 42.0 ± 4.51 | 47.3 ± 1.92 | 50.3 ± 2.07 | 46.8 ± 2.17 | 51.3 ± 1.03 | 43.3 ± 2.34 |
| 5 | 49.5 ± 3.60 | 49.0± 2.15 | 55.6 ± 3.03 | 48.2 ± 2.54 | 51.3 ± 1.31 | 50.2 ± 3.29 |
| * indicates P < 0.05<br>† indicates P < 0.001<br>†† indicates P < 0.0001 | | | | | | |

The invention is not to be limited in scope by the specific embodiments disclosed in the examples that are intended as illustrations of a few aspects of the invention.

## Claims

**1.** A compound of formula (I):

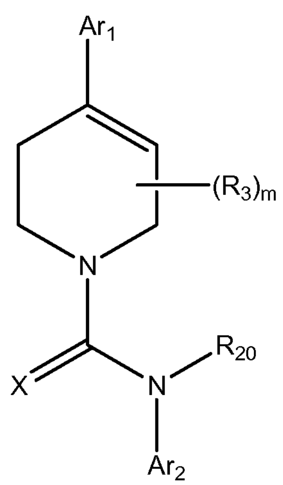

(I)

for use in the treatment of pain associated with a burn injury, wherein

X is O;
$Ar_1$ is:

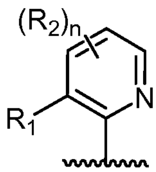

$Ar_2$ is:

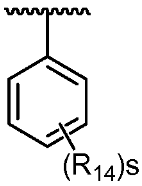

or

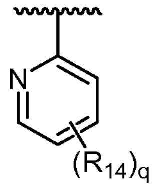

;

$R_1$ is -halo;
each $R_2$ is:

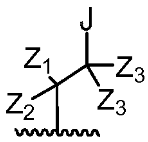

$Z_1$ is -H, $-OR_7$, or $-CH_2-OR_7$;
$Z_2$ is -H, $-(C_1-C_6)$alkyl, or $-CH_2-OR_7$;
each $Z_3$ is independently -H, or $-(C_1-C_6)$alkyl;
J is $-OR_{20}$;
each $R_3$ is independently selected from the group consisting of -H, $(C_1-C_6)$alkyl, and $CH_2OR_7$;
each $R_7$ is independently -H, $-(C_1-C_6)$alkyl, $-(C_2-C_6)$alkenyl, $-(C_1-C_6)$haloalkyl, $-(C_1-C_6)$hydroxyalkyl, or $-(C_1-C_6)$alkoxy$(C_1-C_6)$alkyl;
each $R_{14}$ is independently $-(C_1-C_6)$alkyl, $-C(halo)_3$, $-CH(halo)_2$, $-CH_2(halo)$, -halo, $-OC(halo)_3$, $-OR_7$, or $-SO_2C(halo)_3$;
each $R_{20}$ is independently -H, or $-(C_1-C_6)$alkyl;
each halo is independently -F, -Cl, -Br, or -I;
n is the integer 1, 2, or 3;
q is the integer 1, or 2;
s is the integer 1, or 2;
m is the integer 0, 1, or 2;

or a pharmaceutically acceptable salt or solvate thereof.

**2.** The compound for use of claim 1, wherein:

$Z_1$ is H or $-CH_2OR_7$; and
each $R_{14}$ is independently selected from halo, $C(halo)_3$, or $OC(halo)_3$;

or a pharmaceutically acceptable salt or solvate thereof.

**3.** The compound for use of claim 1, wherein $Ar_1$ is:

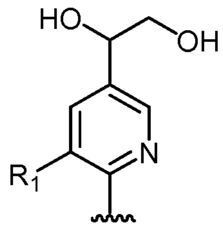

or a pharmaceutically acceptable salt or solvate thereof.

**4.** The compound for use of any one of claims 1 or 3, wherein $Ar_2$ is selected from:

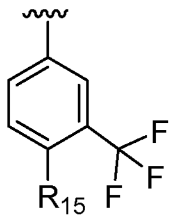 , 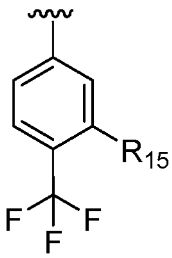 , 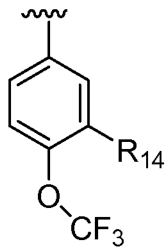 , 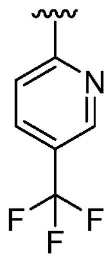 , 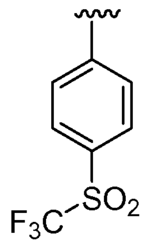 or 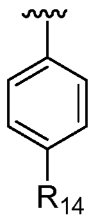

;

wherein $R_{14}$ is selected from halo, $C(halo)_3$, $-(C_1-C_6)alkyl$, $OR_7$, $OC(halo)_3$, or $SO_2C(halo)_3$, and preferably is halo, $C(halo)_3$, or $OC(halo)_3$; and
$R_{15}$ is-Cl, -F, -Br, $-CH_3$, $-CH_2CH_3$, $-OCH_3$, $-OCH(CH_3)_2$ or $-OCH_2CH_3$;

or a pharmaceutically acceptable salt or solvate thereof.

**5.** The compound for use of claim 1, wherein the compound of formula I is selected from the group consisting of:

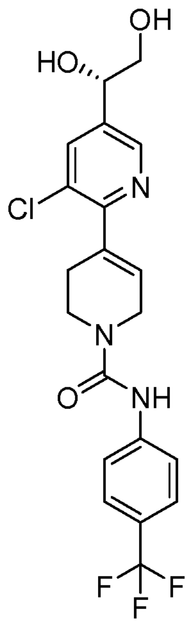 , 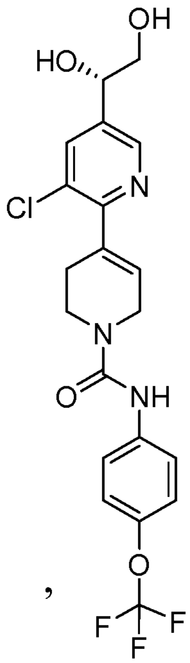 , 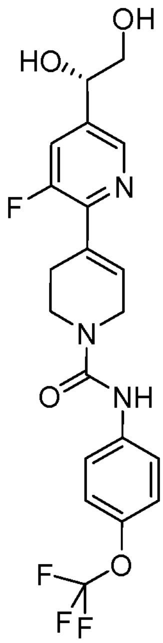 ,

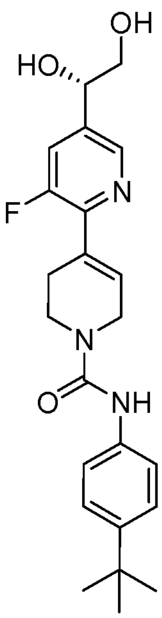 , 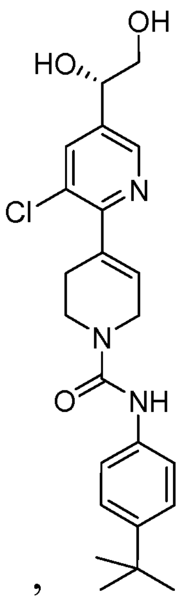 , 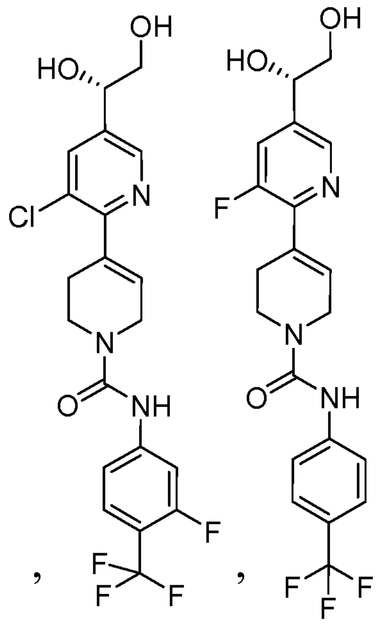 , , 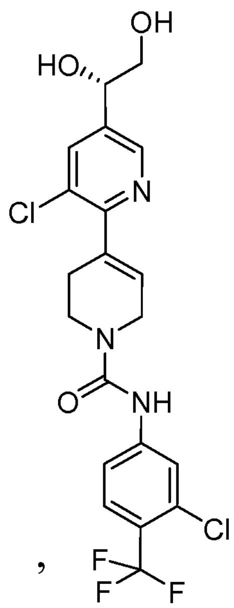 ,

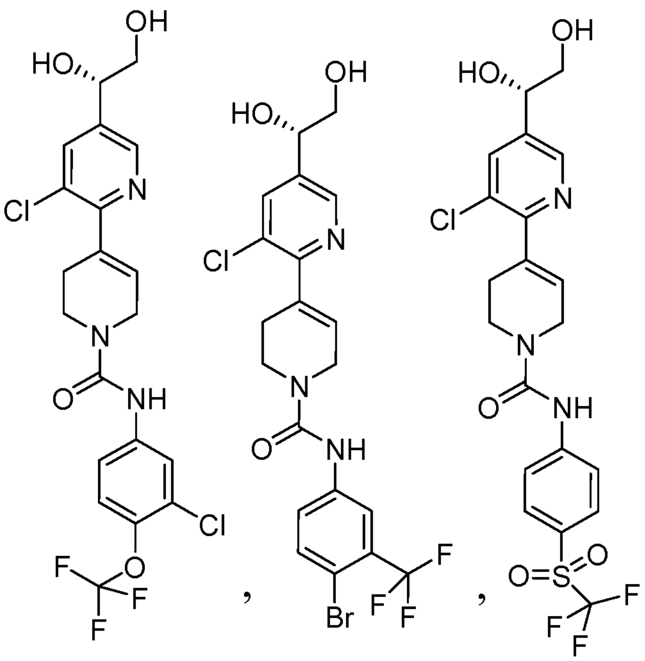 , , , 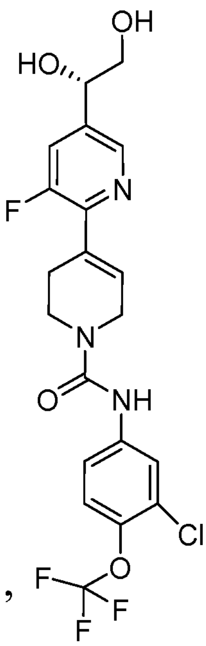 , 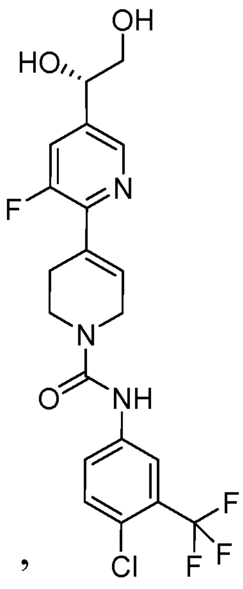 ,

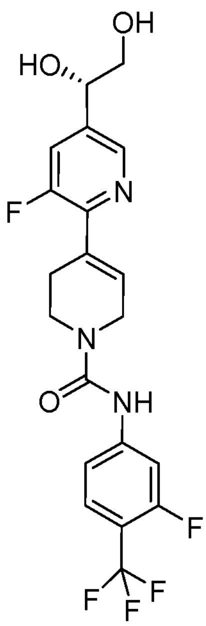 , 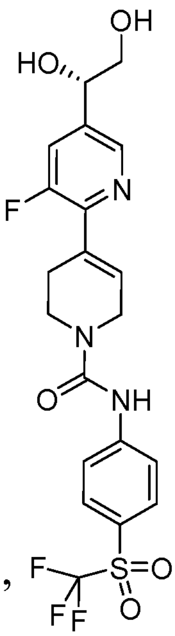 , 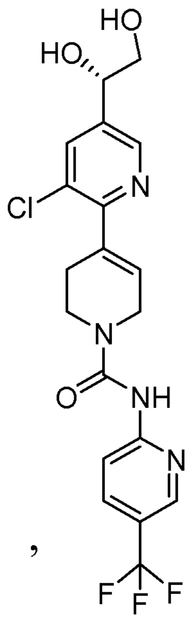 , 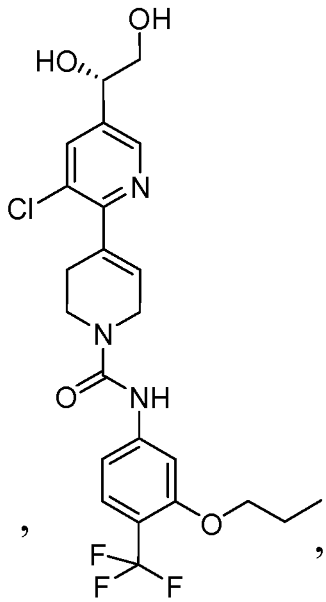 ,

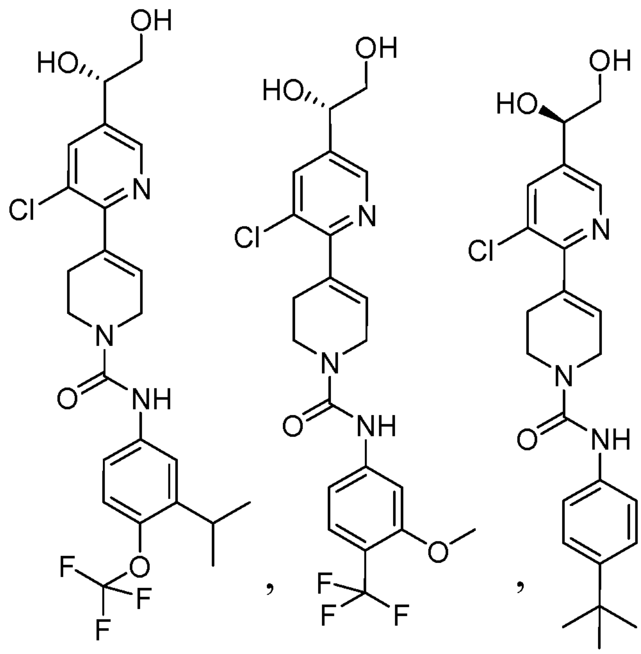 , , , 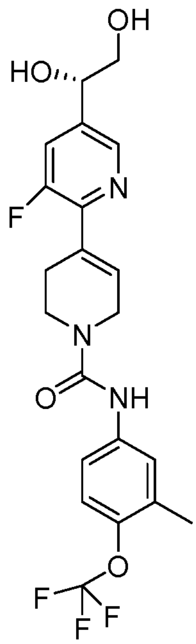 ,

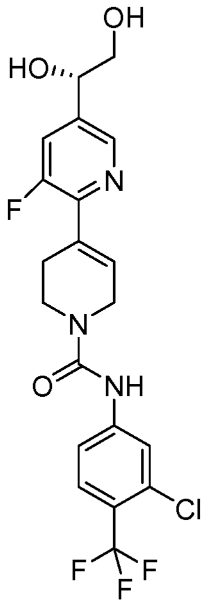 , 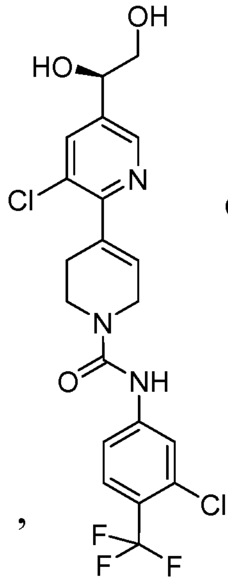 , 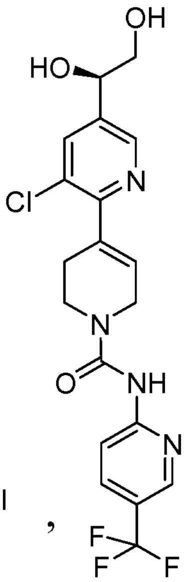 , and 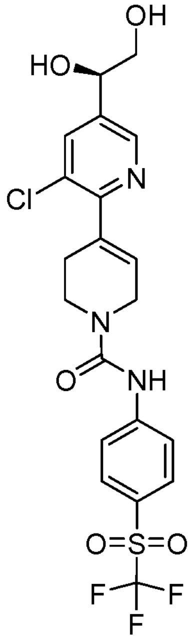 ;

and the pharmaceutically acceptable salts and solvates thereof.

**6.** A compound of formula (II):

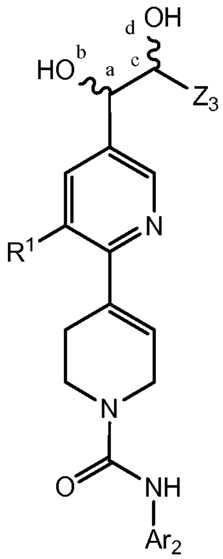

(II)

for use in the treatment of pain associated with a burn injury, wherein:

$R_1$ is -H, -halo, -($C_1$-$C_4$)alkyl, -C(halo)$_3$, -CH(halo)$_2$, or -$CH_2$(halo);
$Z_3$ is -H or -$CH_3$;
$Ar_2$ is

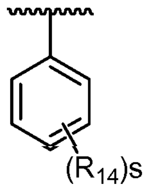

or

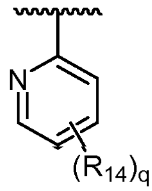

;

and

each $R_{14}$ is independently $-(C_1-C_6)$alkyl, $-C(halo)_3$, $-CH(halo)_2$, $-CH_2(halo)$, -halo, $-OC(halo)_3$, $-OR_7$, or $-SO_2C(halo)_3$;

q is the integer 1, or 2;

s is the integer 1, or 2;

each halo is independently -F, -Cl, -Br, or -I;

or a pharmaceutically acceptable salt or solvate thereof.

**7.** A compound for use according to claim 6 having the following formula:

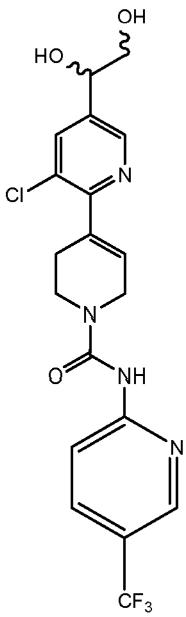

or a pharmaceutically acceptable salt or solvate thereof.

**8.** A compound for use according to claim 6 having the following formula:

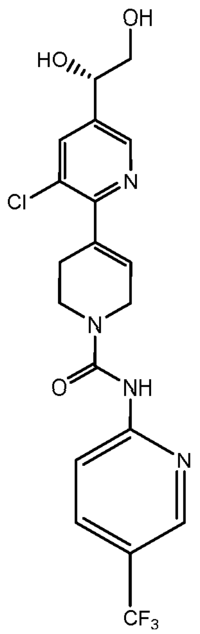

or a pharmaceutically acceptable salt or solvate thereof.

9. The compound for use as defined in any one of claims 1-8, or a pharmaceutically acceptable salt or solvate thereof, wherein the % *ee* of the compound is at least about 90 %.

10. The compound for use as defined in any one of claims 1-9, or a pharmaceutically acceptable salt or solvate thereof, wherein the % *ee* of the compound is at least about 93 %.

11. The compound for use as defined in any one of claims 1-10, or a pharmaceutically acceptable salt or solvate thereof, wherein the burn injury originates from a thermal exposure, from a radiation exposure, from contact with a chemical agent, or from friction.

12. A composition for use in the treatment of pain associated with a burn injury comprising a compound as defined in any one of claims 1 to 11, or a pharmaceutically acceptable salt thereof, and a pharmaceutically acceptable carrier or excipient.

## Patentansprüche

1. Verbindung der Formel (I):

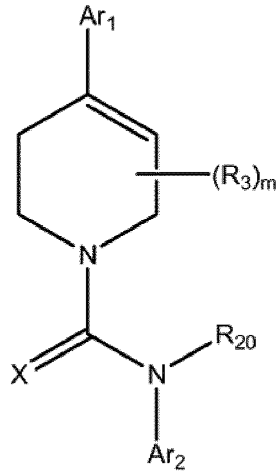

(I)

zur Verwendung bei der Behandlung von Schmerzen, die mit einer Verbrennungsverletzung assoziiert sind, wobei

X gleich O ist;
$Ar_1$ Folgendes ist:

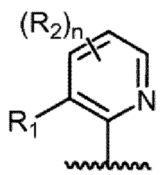

$Ar_2$ Folgendes ist:

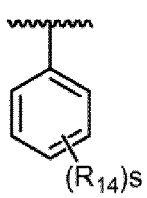 oder 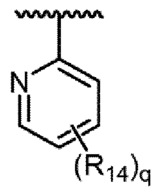 ;

$R_1$ gleich -Halo ist;
jedes $R_2$ Folgendes ist:

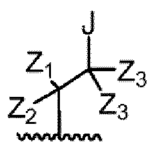

$Z_1$ gleich -H, $-OR_7$, oder $-CH_2-OR_7$ ist;
$Z_2$ gleich -H, $-(C_1-C_6)$alkyl oder $-CH_2-OR_7$ ist;
jedes $Z_3$ unabhängig gleich -H oder $-(C_1-C_6)$alkyl ist;
J gleich $OR_{20}$ ist;
jedes $R_3$ unabhängig ausgewählt ist aus der Gruppe bestehend aus -H, $(C_1-C_6)$alkyl und $CH_2OR_7$;
jedes $R_7$ unabhängig gleich -H, $-(C_1-C_6)$alkyl, $-(C_2-C_6)$alkenyl, $-(C_1-C_6)$halogenalkenyl, $-(C_1-C_6)$hydroxyalkyl

oder-$(C_1-C_6)$alkoxy$(C_1-C_6)$alkyl ist;
jedes $R_{14}$ unabhängig gleich $-(C_1-C_6)$alkyl, -C(Halogen)$_3$,-CH(Halogen)$_2$, $-CH_2$(Halogen), -Halo, -OC(Halogen)$_3$, $-OR_7$ oder-$SO_2C$(Halogen)$_3$ ist;
jedes $R_{20}$ unabhängig gleich -H oder $-(C_1-C_6)$alkyl ist;
jedes Halogen unabhängig gleich -F, -Cl, -Br oder -I ist;
n die ganze Zahl 1, 2 oder 3 ist;
q die ganze Zahl 1 oder 2 ist;
s die ganze Zahl 1 oder 2 ist;
m die ganze Zahl 0, 1 oder 2 ist;

oder ein pharmazeutisch unbedenkliches Salz oder Solvat davon.

**2.** Verbindung zur Verwendung nach Anspruch 1, wobei:

$Z_1$ gleich H oder $-CH_2OR_7$ ist; und
jedes $R_{14}$ unabhängig ausgewählt ist aus Halogen, C(Halogen)$_3$ oder OC(Halogen)$_3$;

oder ein pharmazeutisch unbedenkliches Salz oder Solvat davon.

**3.** Verbindung zur Verwendung nach Anspruch 1, wobei $Ar_1$ Folgendes ist:

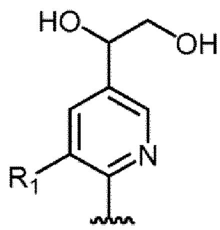

oder ein pharmazeutisch unbedenkliches Salz oder Solvat davon.

**4.** Verbindung zur Verwendung nach einem der Ansprüche 1 oder 3, wobei $Ar_2$ ausgewählt ist aus:

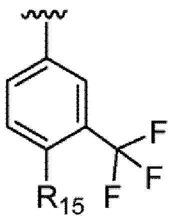 , 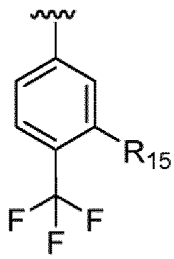 , 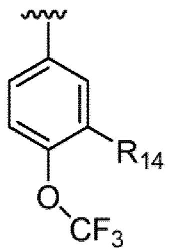 , 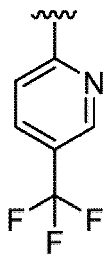 , 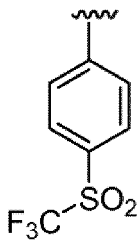 oder 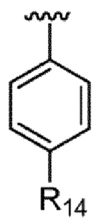

;

wobei $R_{14}$ ausgewählt ist aus Halogen, C(Halogen)$_3$, $-(C_1-C_6)$alkyl, $OR_7$, OC(Halogen)$_3$ oder $SO_2C$(Halogen)$_3$, und vorzugsweise gleich Halogen, C(Halogen)$_3$ oder OC(Halogen)$_3$ ist; und
$R_{15}$ gleich -Cl, -F, -Br, $-CH_3$, $-CH_2CH_3$, $-OCH_3$, $OCH(CH_3)_2$ oder $-OCH_2CH_3$ ist;

oder ein pharmazeutisch unbedenkliches Salz oder Solvat davon.

**5.** Verbindung zur Verwendung nach Anspruch 1, wobei die Verbindung der Formel I ausgewählt ist aus der Gruppe bestehend aus :

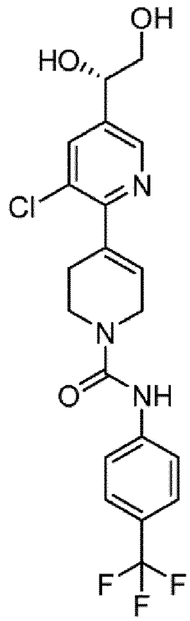 , 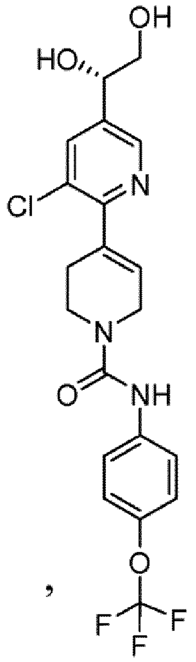 , 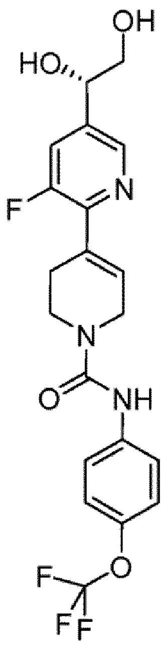 ,

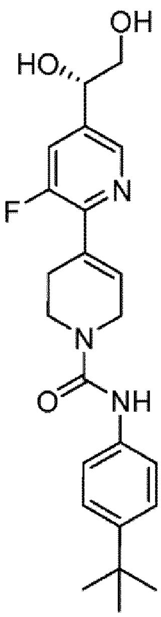 , 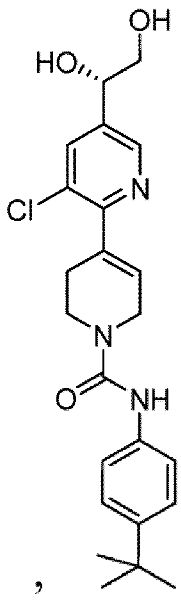 , 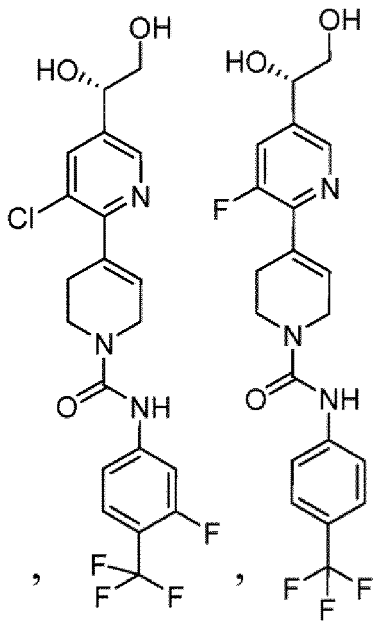 , , 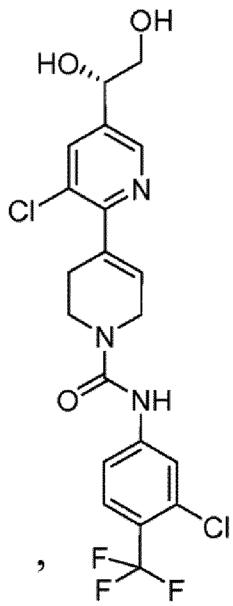 ,

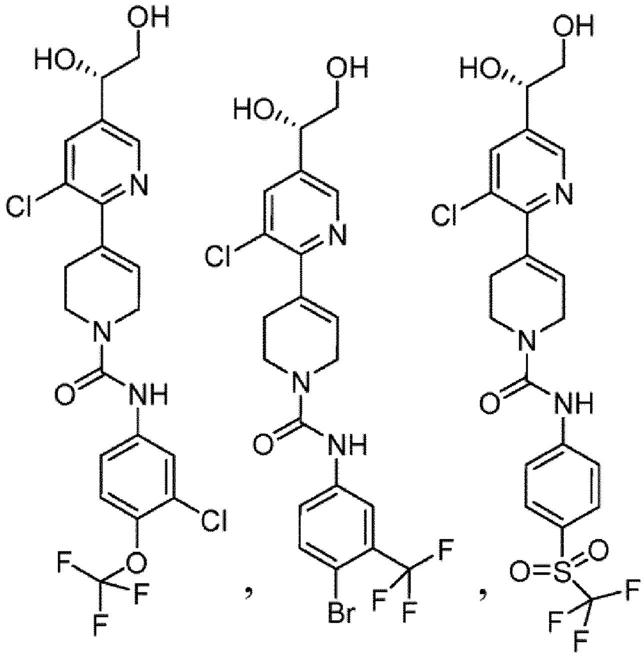 , , ,

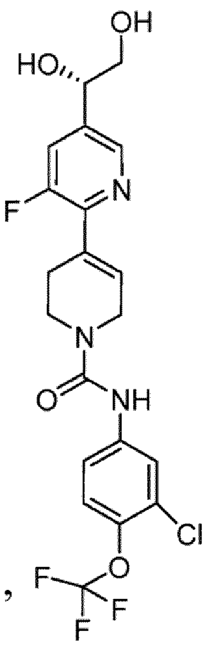 ,

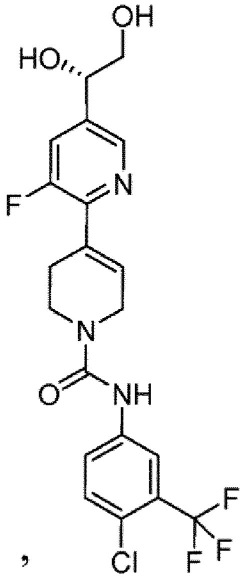 ,

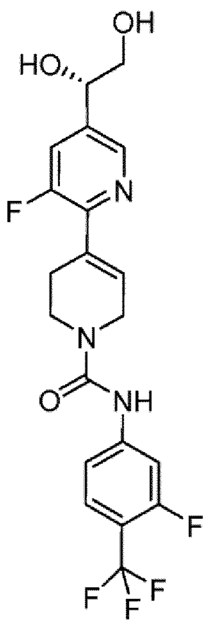 ,

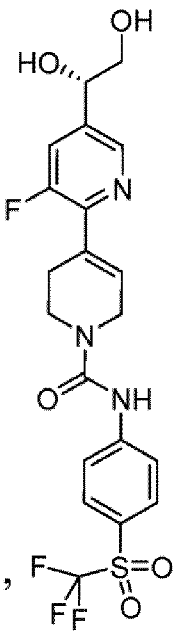 ,

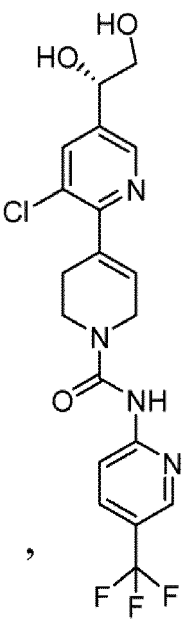 ,

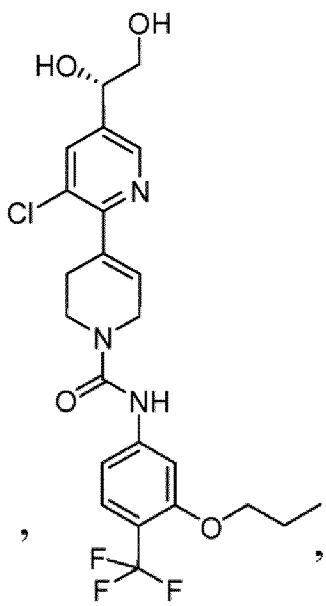 ,

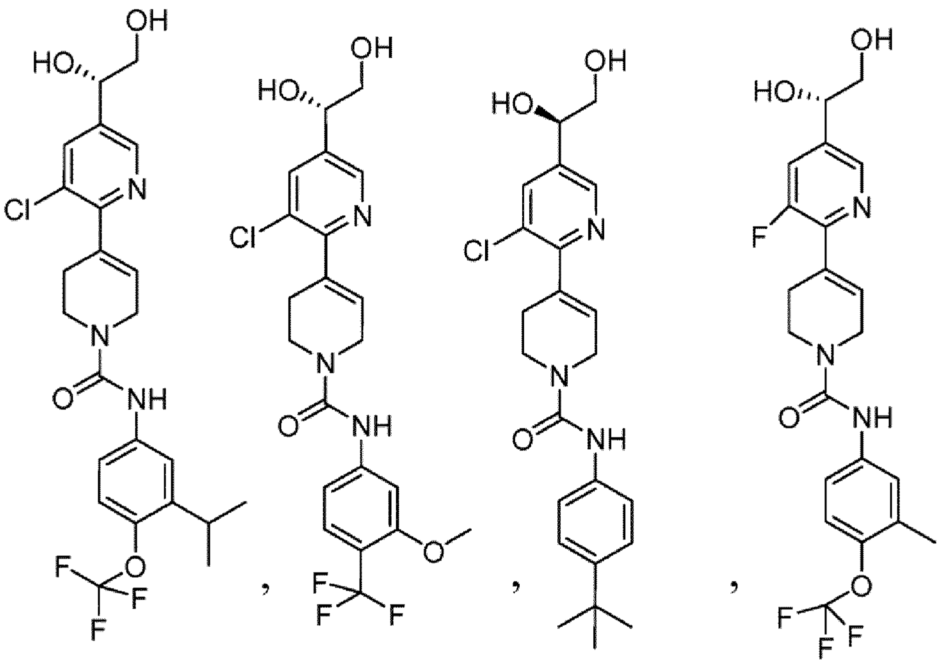

, , , ,

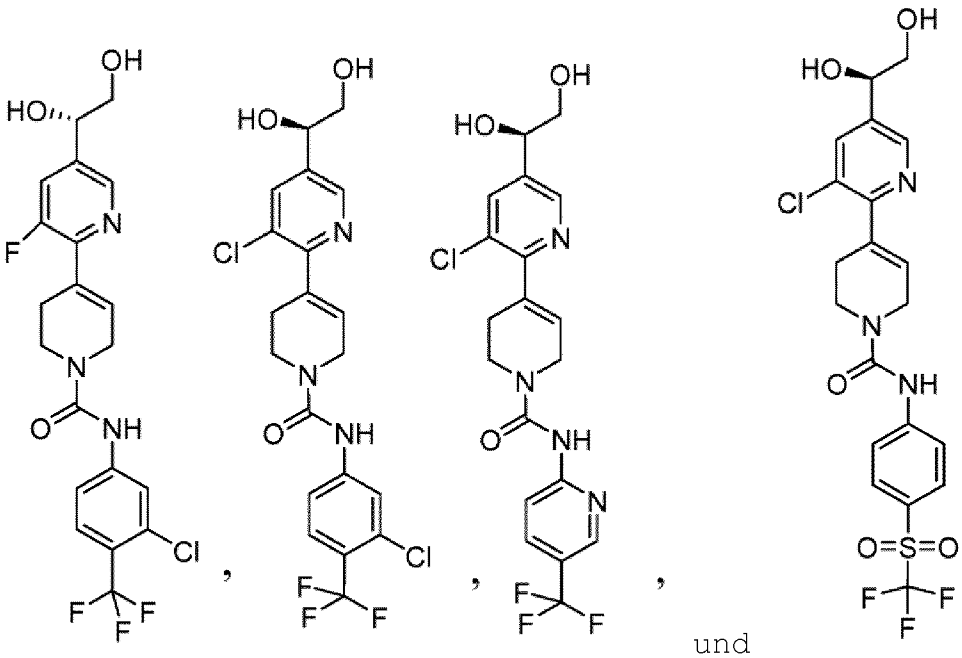

, , , und ;

und den pharmazeutisch unbedenklichen Salzen und Solvaten davon.

**6.** Verbindung der Formel (II):

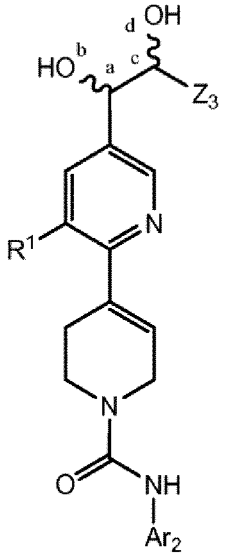

(II)

zur Verwendung bei der Behandlung von Schmerzen, die mit einer Verbrennungsverletzung assoziiert sind, wobei:

$R_1$ gleich -H, -Halo, -($C_1$-$C_4$)alkyl, -C(Halogen)$_3$,-CH(Halogen)$_2$ oder $CH_2$(Halogen) ist;
$Z_3$ gleich -H oder -$CH_3$ ist;
$Ar_2$ Folgendes ist:

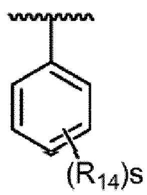 oder 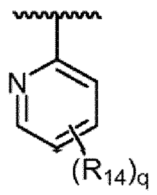 ;

und
jedes $R_{14}$ unabhängig gleich -($C_1$-$C_6$) alkyl, -C(Halogen)$_3$,-CH(Halogen)$_2$, $CH_2$(Halogen), -Halo, -OC(Halogen)$_3$, -$OR_7$ oder-$SO_2$C(Halogen)$_3$ ist;
q die ganze Zahl 1 oder 2 ist;
s die ganze Zahl 1 oder 2 ist;
jedes Halogen unabhängig -F, -Cl, -Br oder -I ist;

oder ein pharmazeutisch unbedenkliches Salz oder Solvat davon.

**7.** Verbindung zur Verwendung nach Anspruch 6, die die folgende Formel aufweist:

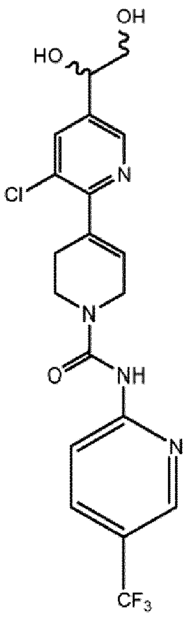

oder ein pharmazeutisch unbedenkliches Salz oder Solvat davon.

**8.** Verbindung zur Verwendung nach Anspruch 6, die die folgende Formel aufweist:

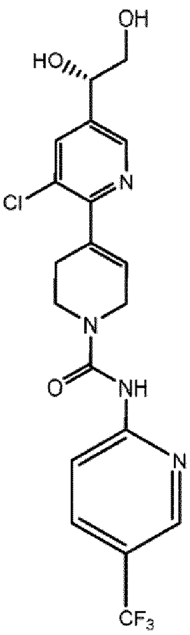

oder ein pharmazeutisch unbedenkliches Salz oder Solvat davon.

**9.** Verbindung zur Verwendung nach einem der Ansprüche 1-8 oder pharmazeutisch unbedenkliches Salz oder Solvat davon, wobei die ee-% der Verbindung mindestens etwa 90 % betragen.

**10.** Verbindung zur Verwendung nach einem der Ansprüche 1-9 oder pharmazeutisch unbedenkliches Salz oder Solvat davon, wobei die ee-% der Verbindung mindestens 93 % betragen.

**11.** Verbindung zur Verwendung nach einem der Ansprüche 1-10 oder pharmazeutisch unbedenkliches Salz oder Solvat davon, wobei die Verbrennungsverletzung von einer Wärmeeinwirkung, von einer Strahlenexposition, von einem Kontakt mit einer Chemikalie oder von einer Reibung stammt.

**12.** Zusammensetzung zur Verwendung bei der Behandlung von Schmerzen, die mit einer Verbrennungsverletzung assoziiert sind, wobei die Zusammensetzung eine Verbindung nach einem der Ansprüche 1-11 oder ein pharmazeutisch unbedenkliches Salz oder Solvat davon und einen pharmazeutisch unbedenklichen Träger oder Hilfsstoff umfasst.

**Revendications**

**1.** Composé répondant à la formule (I) :

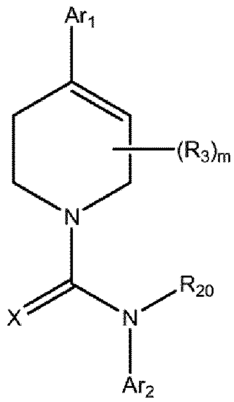

(I)

pour une utilisation dans le traitement de la douleur associée à une blessure par brûlure, dans lequel

X est O ;
$Ar_1$ est :

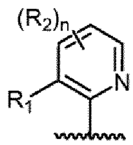

$Ar_2$ est :

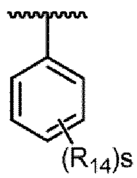

ou

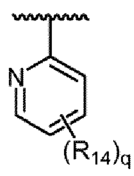

;

$R_1$ est -halogéno ;
chaque $R_2$ est :

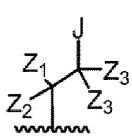

$Z_1$ est -H, $-OR_7$ ou $-CH_2-OR_7$ ;
$Z_2$ est -H, -alkyle en $C_1$-$C_6$ ou $-CH_2-OR_7$ ;

chaque $Z_3$ est indépendamment -H ou -alkyle en $C_1$-$C_6$ ;
J est $-OR_{20}$ ;
chaque $R_3$ est sélectionné indépendamment à partir du groupe constitué par -H, alkyle en $C_1$-$C_6$ et $CH_2$-$OR_7$ ;
chaque $R_7$ est indépendamment -H, -alkyle en $C_1$-$C_6$, -alcényle en $C_2$-$C_6$, -halogénoalkyle en $C_1$-$C_6$, -hydroxyalkyle en $C_1$-$C_6$ ou-alcoxy en $C_1$-$C_6$-alkyle en $C_1$-$C_6$ ;
chaque $R_{14}$ est indépendamment -alkyle en $C_1$-$C_6$, -C(halogéno)$_3$,-CH(halogéno)$_2$, -$CH_2$(halogéno), -halogéno, -OC(halogéno)$_3$, $-OR_7$ ou -$SO_2$C(halogéno)$_3$ ;
chaque $R_{20}$ est indépendamment -H ou -alkyle en $C_1$-$C_6$ ;
chaque halogéno est indépendamment -F, -Cl, -Br ou -I ;
n est l'entier 1, 2 ou 3 ;
q est l'entier 1 ou 2 ;
s est l'entier 1 ou 2 ;
m est l'entier 0, 1 ou 2 ;
ou sel ou solvate pharmaceutiquement acceptable de celui-ci.

**2.** Composé pour une utilisation selon la revendication 1, dans lequel :

$Z_1$ est H ou $-CH_2OR_7$ ;
chaque $R_{14}$ est sélectionné indépendamment à partir de halogéno, C(halogéno)$_3$ ou OC(halogéno)$_3$ ;
ou sel ou solvate pharmaceutiquement acceptable de celui-ci.

**3.** Composé pour une utilisation selon la revendication 1, dans lequel $Ar_1$ est :

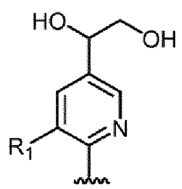

ou sel ou solvate pharmaceutiquement acceptable de celui-ci.

**4.** Composé pour une utilisation selon l'une quelconque des revendications 1 ou 3, dans lequel $Ar_2$ est sélectionné à partir de :

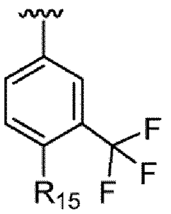 , 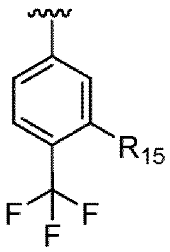 , 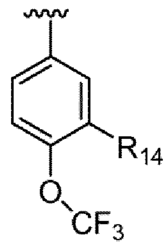 , 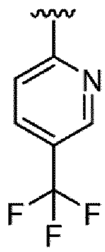 , 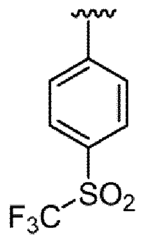 ou 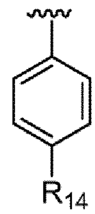 ;

dans lequel $R_{14}$ est sélectionné à partir de halogéno, C(halogéno)$_3$, -alkyle en $C_1$-$C_6$, $OR_7$, OC(halogéno)$_3$ ou $SO_2$C(halogéno)$_3$ et de préférence est halogéno, C(halogéno)$_3$, ou OC(halogéno)$_3$ ; et
$R_{15}$ est -Cl, -F, -Br, $-CH_3$, $-CH_2CH_3$, $-OCH_3$, $-OCH(CH_3)_2$ ou-$OCH_2CH_3$ ;
ou sel ou solvate pharmaceutiquement acceptable de celui-ci.

**5.** Composé pour une utilisation selon la revendication 1, dans lequel le composé répondant à la formule I est sélectionné à partir du groupe constitué par :

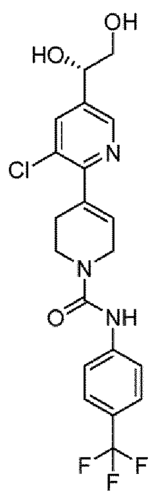 , 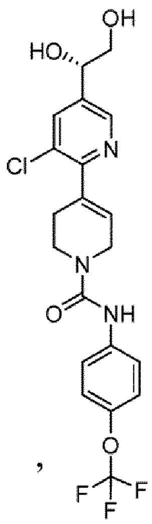 , 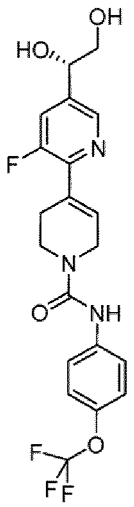 ,

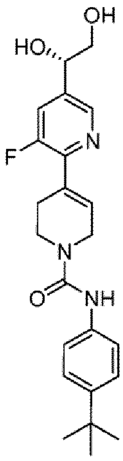 , 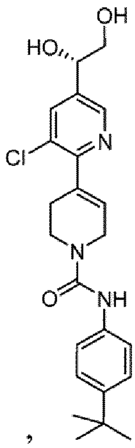 , 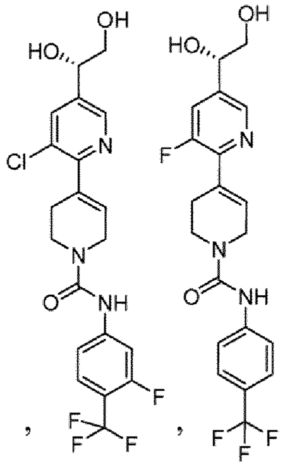 , 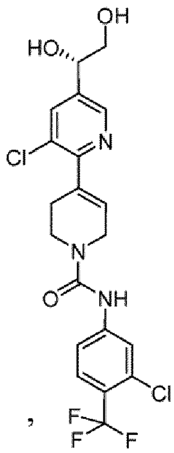 ,

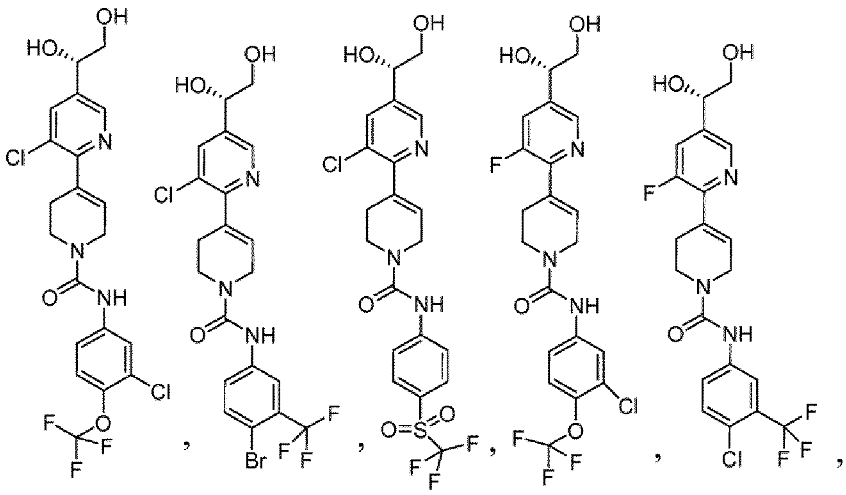 ,

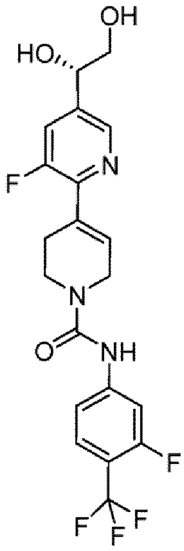, 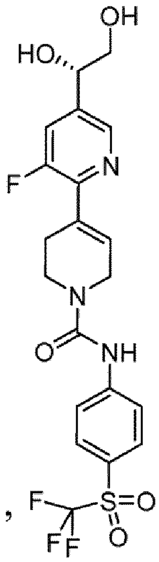, 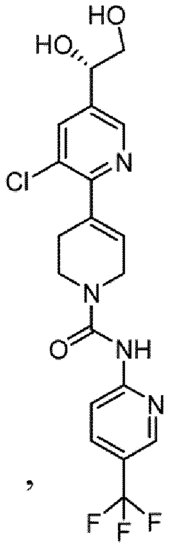, 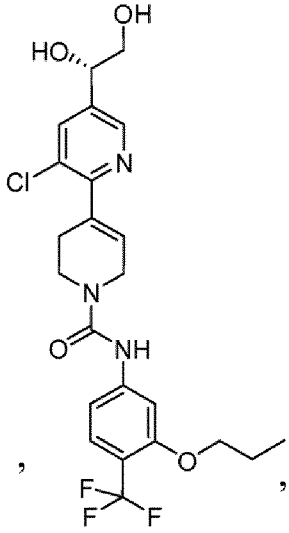,

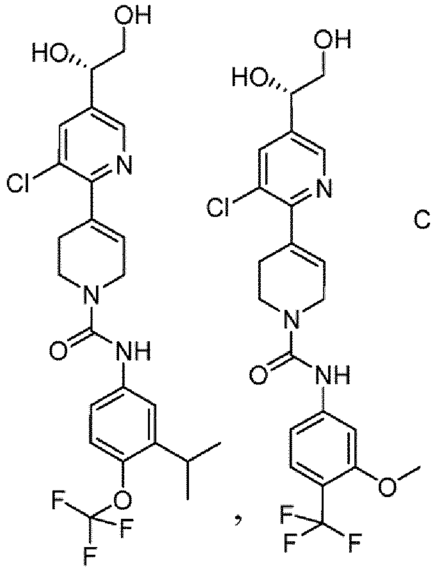, , , 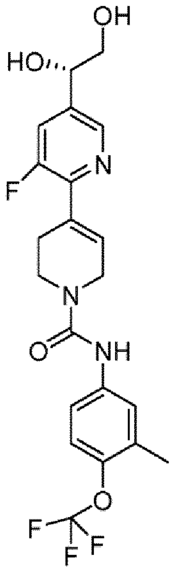,

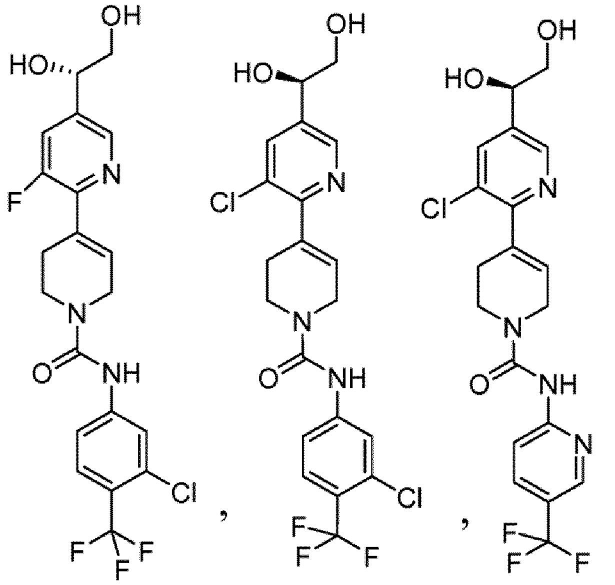 , , , et 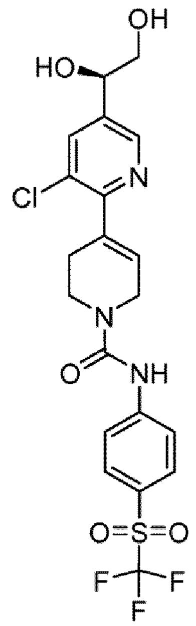 ;

et sels ou solvates pharmaceutiquement acceptables de celui-ci.

**6.** Composé répondant à la formule (II) :

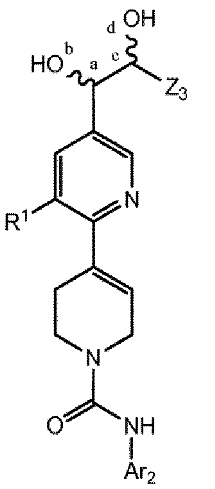

(II)

pour une utilisation dans le traitement de la douleur associée à une blessure par brûlure, dans lequel :

$R_1$ est -H, -halogéno, alkyle en $C_1$-$C_4$, -C(halogéno)$_3$,-CH(halogéno)$_2$ ou -$CH_2$(halogéno) ;
$Z_3$ est -H ou -$CH_3$ ;
$Ar_2$ est :

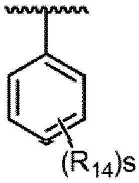 ou 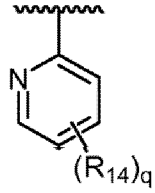 ;

et

chaque $R_{14}$ est indépendamment -alkyle en $C_1$-$C_6$, -C(halogéno)$_3$,-CH(halogéno)$_2$, -$CH_2$(halogéno), -halogéno, -OC(halogéno)$_3$, -$OR_7$ ou -$SO_2$C(halogéno)$_3$ ;

q est l'entier 1 ou 2 ;

s est l'entier 1 ou 2 ;

chaque halogéno est indépendamment -F, -Cl, -Br ou -I ;

ou sel ou solvate pharmaceutiquement acceptable de celui-ci.

**7.** Composé pour une utilisation selon la revendication 6 répondant à la formule suivante :

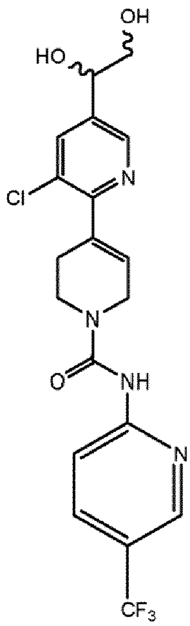

ou sel ou solvate pharmaceutiquement acceptable de celui-ci.

**8.** Composé pour une utilisation selon la revendication 6 répondant à la formule suivante :

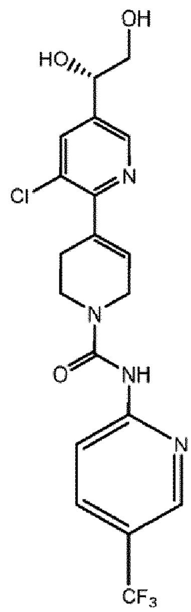

ou sel ou solvate pharmaceutiquement acceptable de celui-ci.

9. Composé pour une utilisation selon l'une quelconque des revendications 1 à 8, ou sel ou solvate pharmaceutiquement acceptable de celui-ci, dans lequel le % ee du composé est d'au moins environ 90 %.

10. Composé pour une utilisation selon l'une quelconque des revendications 1 à 9, ou sel ou solvate pharmaceutiquement acceptable de celui-ci, dans lequel le % ee du composé est d'au moins environ 93 %.

11. Composé pour une utilisation selon l'une quelconque des revendications 1 à 10, ou sel ou solvate pharmaceutiquement acceptable de celui-ci, dans lequel la blessure par brûlure a pour origine une exposition thermique, une exposition à des radiations, un contact avec un agent chimique ou un frottement.

12. Composition pour une utilisation dans le traitement de la douleur associée à une blessure par brûlure comprenant un composé selon l'une quelconque des revendications 1 à 11 ou un sel pharmaceutiquement acceptable de celui-ci et un véhicule ou un excipient pharmaceutiquement acceptable.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6984647 B
- US 7678812 B
- US 20100261911 A
- US 20090209633 A
- US 8394828 B
- US 7767705 B
- US 20100249203 A
- US 7355045 B2
- US 8476277 B
- US 7776861 B
- US 5698155 A
- US 3845770 A
- US 3916899 A
- US 3536809 A
- US 3598123 A
- US 4008719 A
- US 5674533 A
- US 5059595 A
- US 5591767 A
- US 5120548 A
- US 5073543 A
- US 5639476 A
- US 5354556 A
- US 5733566 A
- US 6136839 A


### Non-patent literature cited in the description


- **MEENA et al.** Effect of topical phenytoin on burn wound healing in rats. *Indian J. Exper. Biology,* 2011, vol. 49, 56-59
- **STODDARD et al.** Acute stress symptoms in young children with burns. *J. Amer. Acad. Child Adolesc. Psychiatry,* 2006, vol. 45, 87-93
- **WANG et al.** Nociceptive behavior following hindpaw burn injury in young rats: response to systemic morphine. *Pain Med.,* 2011, vol. 12, 87-98
- **WHITE et al.** Severe burn injury induces a characteristic activation of extracellular signal-regulated kinase 1/2 in spinal dorsal horn neurons. *Eur. J. Pain,* 2011, vol. 15, 683-690
- **WONG et al.** Surgical approaches to create murine models of human wound healing. *J Biomedicine Biotech.,* 2011, vol. 2011, 1-8
- **OKADA et al.** TRPV1 involvement in inflammatory tissue fibrosis in mice. *Amer. J. Pathology,* 2011, vol. 178, 2654-2664
- **PEREIRA et al.** Development of animal model for studying deep second-degree thermal burns. *J. Biomedicine Biotech.,* 2012, vol. 2012, 1-7
- **DI MARZO et al.** Endovanilloid signaling in pain. *Current Opinion in Neurobiology,* 2002, vol. 12, 372-379
- **LAYCOCK et al.** *Eur. J. Pharmacol,* 2013, vol. 716 (1), 169-178
- **CAIRA et al.** Preparation and Crystal Characterization of a Polymorph, a Monohydrate, and an Ethyl Acetate Solvate of the Antifungal Fluconazole. *J. Pharmaceut. Sci.,* 2004, vol. 93 (3), 601-611
- **Y VAN TONDER et al.** Preparation and Physicochemical Characterization of 5 Niclosamide Solvates and 1 Hemisolvate. *AAPS Pharm. Sci. Tech.,* 2004, vol. 5 (1
- **BINGHAM et al.** Over one hundred solvates of sulfathiazole. *Chem. Comm.,* 2001, 603-604
- The Preparation and Characterization of Tritiated Neurochemicals. **FILER.** Isotopes in the Physical and Biomedical Sciences, Vol. 1, Labeled Compounds (Part A). 1987, vol. 1, 155-192
- Pharmacodynamics: Mechanisms of Drug Action and the Relationship Between Drug Concentration and Effect. **ROSS ; KENAKIN et al.** Goodman & Gilman's The Pharmacological Basis of Therapeutics. 2001, 31-32
- **LANGER.** New Methods of Drug Delivery. *Science,* 1990, vol. 249, 1527-1533
- **LOPEZ-BERESTEIN.** Treatment of Systemic Fungal Infections with Liposomal-Amphotericin B. *Liposomes in the Therapy of Infectious Disease and Cancer,* 1989, 317-327
- **TREAT et al.** Liposome encapsulated doxorubicin - preliminary results of phase I and phase II trials. *Liposomes in the Therapy of Infectious Disease and Cancer,* 1989, 353-365
- Medical Applications of Controlled Release. **GOODSON.** Applications and Evaluation. 1984, vol. 2, 115-138
- **LANGER.** *Science,* 1990, vol. 249, 1527-1533
- **SEFTON.** Implantable Pumps. *CRC Crit. Rev. Biomed. Eng.,* 1987, vol. 14 (3), 201-240

- **BUCHWALD et al.** Long-term, Continuous Intravenous Heparin Administration by an Implantable Infusion Pump in Ambulatory Patients with Recurrent Venous Thrombosis. *Surgery,* 1980, vol. 88, 507-516
- **SAUDEK et al.** A Preliminary Trial of the Programmable Implantable Medication System for Insulin Delivery. *New Engl. J. Med.,* 1989, vol. 321, 574-579
- Drug Product Design and Performance. **GOODSON ; SMOLEN et al.** Controlled Drug Bioavailability. John Wiley & Sons, 1984, vol. 1
- **LANGER et al.** Chemical and Physical Structure of Polymers as Carriers for Controlled Release of Bioactive Agents: A Review. *J. Macromol. Sci. Rev. Macromol. Chem.,* 1983, vol. C23 (1), 61-126
- **LEVY et al.** Inhibition of Calcification of Bioprosthetic Heart Valves by Local Controlled-Release Diphosphonate. *Science,* 1985, vol. 228, 190-192
- **DURING et al.** Controlled Release of Dopamine from a Polymeric Brain Implant: In Vivo Characterization. *Ann. Neurol.,* 1989, vol. 25, 351-356
- **HOWARD et al.** Intracerebral drug delivery in rats with lesion-induced memory deficits. *J. Neurosurg.,* 1989, vol. 71, 105
- Handbook of Pharmaceutical Excipients. Amer. Pharmaceutical Ass'n, 1986
- Preformulation. **RADEBOUGH et al.** Remington's Pharmaceutical Sciences. Mack Publishing, 1995, vol. 2, 1447-1676
- Pharmaceutical Dosage Forms: Tablets. Marcel Dekker, Inc, 1989
- Tablets, Capsules, and Pills. **KING.** Remington's Pharmaceutical Sciences. Mack Publishing, 1980, 1553-1593
- Pharmaceutical Dosage Forms: Disperse Systems. Marcel Dekker, Inc, 1996
- Remington: The Science and Practice of Pharmacy. Pharmaceutical Press, 2012
- Analgesic-Antipyretic and Anti-inflammatory Agents and Drugs Employed in the Treatment of Gout. **INSEL et al.** Goodman & Gilman's The Pharmacological Basis of Therapeutics. McGraw-Hill, 1996, 617-657
- Analgesic, Antipyretic and Anti-Inflammatory Drugs. **HANSON.** Remington: The Science and Practice of Pharmacy. Mack Publishing, 1995, vol. 2, 1196-1221
- **EM. DOHERTY et al.** Novel vanilloid receptor-1 antagonists:2. Structure-activity relationships of 4-oxopyrimidines leading to the selection of a clinical candidate. *J Med Chem,* 2007, vol. 50, 3515-3527
- **C. STEIN.** Unilateral Inflammation of the Hindpaw in Rats as a Model of Prolonged Noxious Stimulation: Alterations in Behavior and Nociceptive Thresholds. *Pharmacol. Biochem. and Behavior,* 1988, vol. 31, 451-455
- **K. HARGREAVES et al.** A New and Sensitive Method for Measuring Thermal Nociception in Cutaneous Hyperalgesia. *Pain,* 1988, vol. 32 (1), 77-88